(19) Europäisches Patentamt
European Patent Office
Office européen des brevets

(11) EP 1 040 829 A2

(12) EUROPEAN PATENT APPLICATION

(43) Date of publication:
**04.10.2000 Bulletin 2000/40**

(21) Application number: **99310202.9**

(22) Date of filing: **16.12.1999**

(51) Int Cl.[7]: **A61K 31/416**, A61P 1/08, A61K 31/00 // (A61K31/416, 31:00)

(84) Designated Contracting States:
**AT BE CH CY DE DK ES FI FR GB GR IE IT LI LU MC NL PT SE**
Designated Extension States:
**AL LT LV MK RO SI**

(30) Priority: **30.12.1998 US 114217 P**

(71) Applicant: **PFIZER INC.**
**New York, N.Y. 10017 (US)**

(72) Inventors:
- **Watson, John Wesley**
**Ledyard, Connecticut 06339 (US)**
- **Woods, Anthony John**
**London NW1 2BN (GB)**
- **Andrews, Paul,**
**St. Georges Hospital Med. School**
**Tooting, London SW17 0RE (GB)**

(74) Representative:
**Simpson, Alison Elizabeth Fraser et al**
**Urquhart-Dykes & Lord,**
**30 Welbeck Street**
**London W1M 7PG (GB)**

# (54) Prokinetic agents for treating gastric hypomotility and related disorders

(57) The invention describes the use of PDE-4 (phosphodiesterase-4) inhibitors in the treatment or prevention of gastric stasis resulting from hypomotility of the stomach (with delayed emptying of the liquid and/or solid contents of the stomach).

More particularly said inhibitors are indazole derivatives such as (1H-indazol-6-yl)-cyclohexane or cyclohexene carboxylic acid derivatives, carbonitriles, amides or esters thereof. Gastric or gastrointestinal disorders, which may also be caused by adverse effects of therapeutic agents, of surgical operations or concomitant or secondary aspects of another disease, which include pain, nausea, vomiting, heartburn, postprandial discomfort, indigestion and gastro-esophageal reflux, can be prevented or treated with pharmaceutical compositions containing said PDE-4 indazole inhibitors.



Printed by Jouve, 75001 PARIS (FR)

## Description

### BACKGROUND OF THE INVENTION

The method of treatment of the present invention involves a therapeutic agent having a prokinetic effect on, *i.e.*, that promotes activity with regard to gastric motility. This type of drug is useful in treating gastric hypomotility with delayed gastric emptying of liquid and/or solid contents of the antrum (stomach), which is a component of a number of gastric or gastrointestinal disorders. The symptoms of such gastric or gastrointestinal disorders can be quite serious and include pain, nausea, vomiting, heartburn, postprandial discomfort, indigestion, and gastroesophageal reflux.

In particular, the present invention relates to therapeutic agents which by various mechanisms are able to elevate cAMP in populations of neurons in the myenteric plexus, leading to release of excitatory transmitters, *e.g.*, acetylcholine, and subsequent stimulation with resulting contraction of the smooth muscle of the antrum. The therapeutic compounds useful as active ingredients in the pharmaceutical compositions and methods of treatment of the present invention are closely related, in terms of their chemical structure and biological activity, to inhibitors of the phosphodiesterase-IV (PDE4) isoenzyme. However, to date the art has incorrectly taught that PDE4 inhibitors antagonize gastrointestinal contractile responses, suggesting their use as antikinetic agents for treating hypermotility disorders; rather than as prokinetic agents for treating gastric hypomotility, as surprisingly discovered in accordance with the present invention.

The gastrointestinal system must preserve a proper balance between absorption and secretion of water and electrolytes in order to keep nutrients, wastes, electrolytes and water in a life-sustaining flux. Equally important to successful performance of this ongoing process is the maintenance along the gastrointestinal tract of the appropriate anterograde motility. Gastrointestinal motility is also known to be a key component of vomiting. This aspect of its role is important in light of the fact that some antiemetic agents have enhanced gastric emptying as a significant aspect of their actions.

The causes of gastric stasis (hypomotility) are for the most part unknown. Frequently, it is a consequence of diabetic neuropathy, a concomitant of anorexia nervosa and achlorhydria, and a result of gastric surgery. In particular, gastric hypomotility can be a concomitant of post-surgical recovery, especially in the period of emergence from general anesthesia. Gastric hypomotility can be induced by morphine and morphine-like opioids; it can be a secondary aspect of some primary disease or disorder, especially one which is organic, including particularly a gastroenteric or gastroesophageal organ or tissue, or an organ or tissue of the central nervous system; or it can be an adverse side effect of a therapeutic agent administered in the course of treating some other and possibly unrelated disease or disorder.

Therapeutic agents which may have an adverse impact on gastric motility include analgesics acting by inhibition of prostaglandin synthesis, which includes most NSAIDs; antacids which contain calcium carbonate or aluminum hydroxide; anticholinergic agents; antidiarrheal agents; antihistamines which are $H_1$ blockers or have an anticholinergic effect; antiparkinsonian drugs which have an anticholinergic effect; barium sulfate; corticosteroids; clonidine; diuretics which cause hypokalemia; ganglionic blocking agents; heavy metals including especially lead; iron; laxatives, especially when used chronically; lithium; monoamine oxidase inhibitors; muscle relaxants; octreotide; opioids; phenothiazines having an anticholinergic effect; polystyrene resins; propranolol; tricyclic antidepressants having an anticholinergic effect; and verapamil.

All such events, agents, conditions and diseases are either etiogenic or pathogenic in relation to gastric hypomotility. That is, each and any such event, agent, condition, or disease either directly causes the gastric hypomotility, or else it gives origin to a disease or to morbid symptoms which include or result in gastric hypomotility. Accordingly, there is presently a need for therapeutic agents which are effective in treating gastric hypomotility or "gastric stasis", a term which as used herein is intended to mean any condition in which normal passage of the stomach's content is impaired due to impaired gastric motility, but not to any mechanical obstruction.

Vomiting, commonly referred to by its medical name "emesis", as well as nausea, whatever their ostensible cause, are also related mechanistically to changes in gastric motility. For example, when symptoms of nausea appear, gastric tone is reduced, gastric peristalsis is reduced or absent, and the tone of the duodenum and upper jejunum is increased, which eventually results in gastric reflux. When the upper portion of the stomach then relaxes while the pylorus constricts, expulsion of the gastric contents is produced by the coordinated contraction of the diaphragm and abdominal muscles. The causes of nausea and vomiting vary. Nausea and vomitinig may follow the administration of a variety of drugs, especially cancer chemotherapeutic agents, may accompany infectious as well as noninfectious gastric or gastrointestinal disorders, may occur upon emergence from general anethesia, may be as aspect of early pregnancy, or may result from motion sickness. Emesis is a complex process which is coordinated by the vomiting center, including the chemoreceptor trigger zone, and which is mediated by various efferent pathways. Dopamine receptors in the stomach appear to mediate the inhibition of gastric motility that occurs during nausea and vomiting, as well as delayed gastric emptying responsive to food-originated gastric distension. Consequently, dopamine antagonists are used as prokinetic agents for treating these aspects of emesis. The neuropharmacology of the afferent and

efferent pathways of the vomiting center is currently sufficiently well understood to have provided a basis for rational antiemetic therapy. For example, it is known that serotonin acting on 5-$HT_3$ receptors is an important emetic signal and transmitter , and that dopamine acting at D2 receptors is implicated in emetic signaling through the trigger zone, as is acetylcholine acting through the muscarinic family of receptors. Thus, D2 and 5-$HT_3$ receptor antagonists have antiemetic properties.

In the same way that complex mechanisms have been found to govern emesis, neural regulation of gastric motility has been found to involve a complex combination of stimulation by cholinergic neurons and inhibition by adrenergic neurons, both of which are modulated by the enteric nervous system which in turn is influenced by serotonin and dopamine. For example, it is known that D2 and 5-$HT_3$ receptor antagonists, as well as 5-$HT_4$ receptor agonists, stimulate gastric motility and are thus prokinetic agents, although there may be a dependency on cholinergic transmission. Other known prokinetic agents include the gastrointestinal peptide motilin, and derivatives of erythromycin which stimulate motilin receptors.

Many antiemetic drugs have been found to provide some relief for the symptoms of gastric hypomotility, but in the vast majority of cases they do not accelerate gastric emptying, and they frequently have undesirable side effects as well. Thus, there continues to be an ongoing search for effective prokinetic drugs useful in treating gastric hypomotility, especially gastric hypomotility with delayed emptying. Typical prokinetic drugs which have been discovered to date are illustrated and described below.

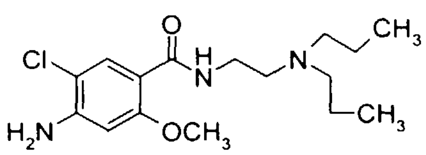

Metoclopramide

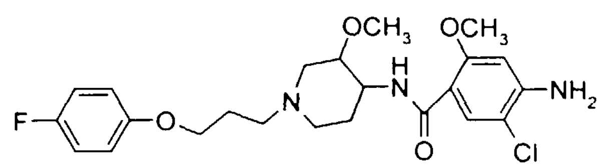

Cisapride

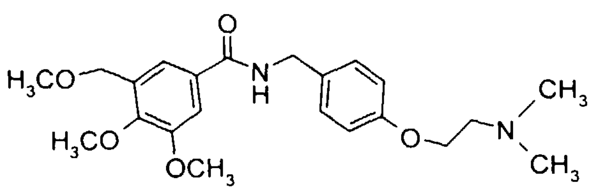

Tribenzomethamide

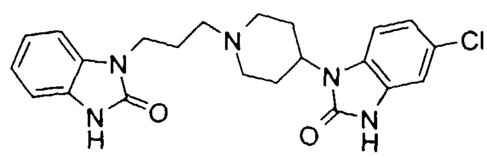

Domperidone

Metoclopramide in the gastrointestinal tract enhances the motility of smooth muscle from the esophagus through the proximal small bowel and accelerates gastric emptying and the transit of intestinal contents from the duodenum to the ileocecal valve. Metoclopramide decreases receptive relaxation in the upper stomach and increases antral contractions, which together accelerates gastric emptying and reduces reflux from the duodenum and stomach into the esophagus. Metoclopramide is a dopaminergic antagonist, but not all dopaminergic antagonists speed gastric emptying.

Trimethobenzamide is also a dopamine receptor antagonist but with modest antiemetic effects. It is not as effective as metoclopramide.

Cisapride affects gastric and small intestine motility to a very similar extent as metoclopramide, but it also increases colonic motility and can cause diarrhea. It appears to be devoid of dopamine receptor antagonist activity and may involve enhancement of the release of myenteric acetylcholine.

Domperidone is a dopamine receptor antagonist with both prokinetic and antiemetic acitivity which inhibits receptive relaxation, enhances coordinated antral-duodenal motility, and accelerates transit in the small intestine. However, domperidone has a negligible effect on colonic motility.

The compounds used as therapeutic agents in the methods of treatment and pharmaceutical compositions of the present invention are related, in terms of their chemical structure and biological activity, to inhibitors of PDE4. Phosphodiesterase-4 is a cAMP-specific phosphodiesterase which plays an important role in the regulation of inflammatory and immune cell activation. The isolation and characterization of the multiple isoforms of the phosphodiesterase enzyme has sparked a great deal of interest in the discovery of selective inhibitors of these isozymes; and to date a

significant variety of different structural types of compounds active as PDE4 inhibitors has been reported. These different structural classes of PDE4 inhibitors are discussed in greater detail further below.

The cyclic nucleotides cAMP and cGMP are currently viewed as important intracellular messengers for relaxation of gastrointestinal smooth muscle. In turn, the intracellular levels of cAMP and cGMP are controlled by a balance between their synthesis and their catabolism; and the phosphodiesterase enzymes, including PDE4, are the only enzymes in mammalian cells responsible for the breakdown of such cyclic nucleotides. Thus, inhibition of the phosphodiesterases produces an increase in cAMP and cGMP within the cells involved, which in turn is associated with relaxation in several regions of the gut, including the lower esophageal sphincter, proximal colon, and taenia coli. While PDE isozymes have been characterized in cardiac muscle, and airway and arterial smooth muscles, only recently have they been characterized in gastrointestinal smooth muscle. Inhibitors of phosphodiesterases potentiate forskolin-induced relaxation of rabbit intestinal smooth muscle and enhance the increase in cAMP produced by forskolin in canine colonic smooth muscle.

However, it is not known in the art what influence PDE inhibitors would have on the response to antigen in gastrointestinal smooth muscle. This activity is significant because the release of inflammatory mediators such as histamine or the peptidoleukotrienes can significantly and adversely alter gastric motility. Addition of antigen to isolated strips of gastrointestinal smooth muscle obtained from sensitized rat or guinea-pig has been reported to contract both small intestinal and colonic tissue. It has also been reported that addition of antigen to sensitized guinea-pig colon produces a biphasic contraction consisting of an initial rapid response that is blocked by the $H_1$ -histamine receptor antagonist mepyramine, and a slower developing late contraction that is reduced by a peptidoleukotriene ($LTD_4$) receptor antagonist, ritolukast. Mepyramine (pyrilamine) and ritolukast may be represented by the following general structural formulas:

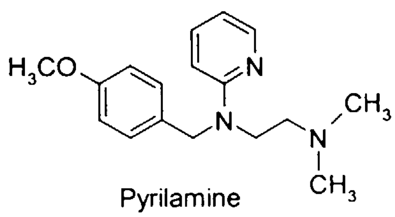

Pyrilamine

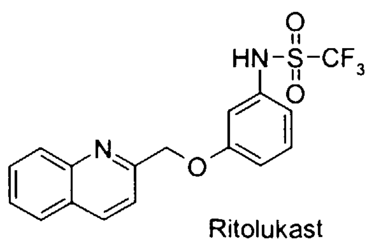

Ritolukast

Since phosphodiesterase-4 has been demonstrated to be the major cAMP metabolizing enzyme in many inflammatory cell types, a significant amount of basic research has been conducted in this area, and at least four isotypes of the PDE4 isozyme have been identified and characterized. Attention has also been focused on a high-affinity allosteric binding site which is abundant in the brain PDE4 isozyme, the so-called "rolipram" binding site, whose differential modulation relative to the cAMP catalytic site is anticipated to yield drugs with greater therapeutic utility. Rolipram may be represented by the following general structural formula:

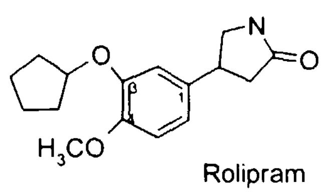

Rolipram

PDE4 inhibitors well known in the art for some time may conveniently be grouped into three structural classes: catechol ethers, quinazolinediones, and xanthines. Representative members of each of these classes are, respectively, rolipram and Ro 20-1724; nitraquazone; and denbufylline, which may be represented by the following general structural formulas:

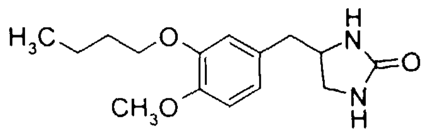

Ro 20-1724

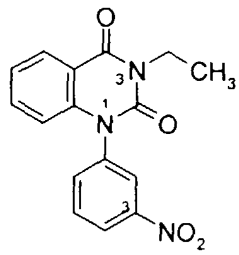

Nitraquazone

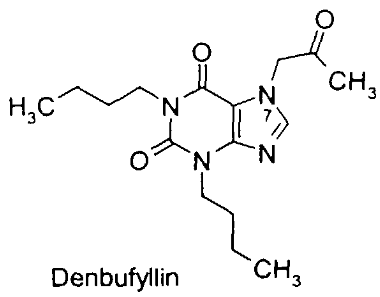

Denbufyllin

DESCRIPTION OF THE STATE OF THE ART

A number of published reports have appeared in the technical literature describing the results of investigations into the role of phosphodiesterase inhibitors in modulating contractile responses of various muscle tissues under varying conditions. Representative reports are briefly summarized herein. For example, Gustafsson *et al.*, Theophylline interferes with the modulatory role of endogenous adenosine on cholinergic neurotransmission in guinea pig ileum", *Acta Physiol Scand,* 1981, 111, 269-280, describes studies of the ability of theophylline and other phosphodiesterase inhibitors to alter the contractile responses of longitudinal muscle to cholinergic nerve stimulation. It concludes that theophylline has a biphasic effect on cholinergic transmission, providing a potentiation of the response to transmural stimulation at the lower doses investigated, while inhibiting contraction responses to the same transmural stimulation at the higher concentrations investigated. It further concluded that the other phosphodiesterase inhibitors studied, dipyridamole and dilazep, caused a depression of the contractile responses to said transmural nerve stimulation over the entire concentration range studied. Theophylline, dipyridamole and dilazep may be represented by the following general structural formulas:

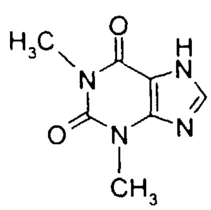

Theophylline

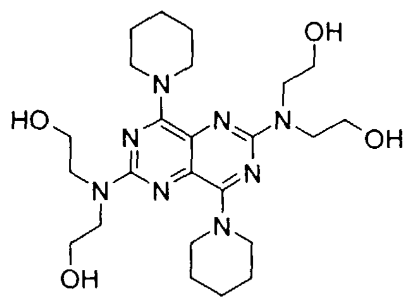

Dipyridamole

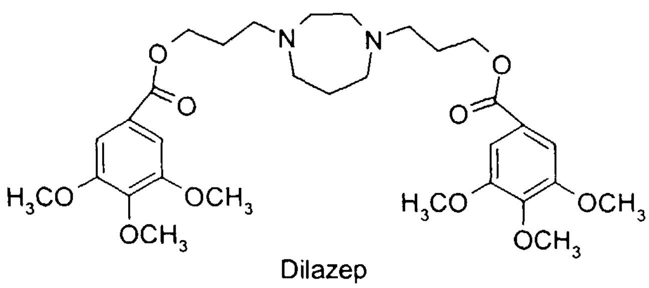

Dilazep

Grous *et al.,* "Change in Intracellular Cyclic Nucleotide Content Accompanies Relaxation of the Isolated Canine Internal Anal Sphincter", *J Gastrointest Motil* 1991, 3(1), 46-52, is concerned with the role that the cyclic nucleotides play in controlling gut smooth muscle tone, and concludes that both cyclic AMP and cyclic GMP are important intracellular mediators of relaxation in several smooth muscles; that relaxation produced by activation of intrinsic inhibitory neurons is associated with an elevation of intracellular cGMP, both of which are blocked by forskolin; and that cGMP may be the intracellular mediator of neuronally induced relaxation of gut sphincteric regions. Forskolin (colforsin) may be represented by the following general structural formula:

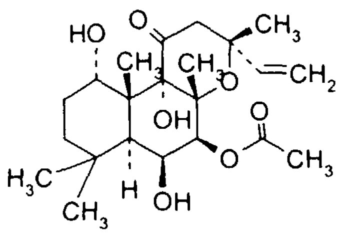

Forskolin (Colforsin)

Barnette *et al.,* Initial Biochemical and Functional Characterization of Cyclic Nucleotide Phosphodiesterase Isozymes in Canine Colonic Smooth Muscle", *J. Pharmacol. Exp. Thera.,* 1993, 264(2), 801-812, describes a study intended to determine the role of the various PDE isozymes in regulating cyclic nucleotide content of gastrointestinal smooth muscle. From the results of this study it was determined that colonic smooth muscle contains at least two forms of PDE with a high affinity for cGMP, one of which is inhibited by zaprinast and thus a PDE5 inhibitor, and at least two isozymes that prefer cAMP as a substrate, one of which is inhibited by SB 94120 and thus a PDE3 inhibitor, and the other by rolipram. The ability of these selective inhibitors to antagonize a carbachol-induced contraction of isolated circular colonic muscle strips in the presence of forskolin was also determined and found to be concentration dependent, with decreases in contractile activity observed in the following potency order: rolipram > Ro 20-1724 > isobutyl methylxanthine > SB 94120 > siguazodan > zaprinast. From these results it was concluded that PDE4 is more important than the other isozymes in regulating agonist-induced increases in cAMP content in colonic smooth muscle. Siguazodan and zaprinast may be represented by the following general structural formulas:

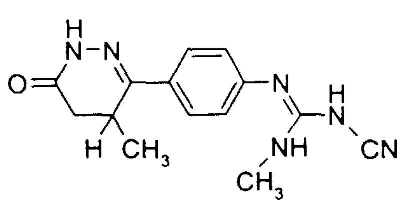

Siguazodan

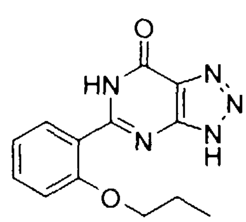

Zaprinast

Grous and Barnette, "Prevention by phosphodiesterase inhibitors of antigen-induced contraction of guinea-pig colonic smooth muscle", *Br. J. Pharmacol.,* 1994, 111, 259-263, reports on the investigation of the ability of various PDE inhibitors to reduce the initial and/or late response to ovalbumin in isolated strips of gastrointestinal (guinea-pig colonic) smooth muscle from sensitized animals. From the results of the investigation it is concluded that PDE inhibitors inhibit antigen-induced contractions in guinea-pig colon, perhaps by decreasing the release of inflammatory mediators. It is also proposed that selective PDE inhibition may decrease the responsiveness of the gastrointestinal tract seen during inflammation, and thus may have a therapeutic benefit in treating altered gastric motility associated with inflammatory diseases of the gastrointestinal tract.

Christofi *et al.*, "Adenosine $A_2$ receptor-mediated excitation of a subset of AH/Type 2 neurons and elevation of cAMP levels in myenteric ganglia of guinea-pig ileum", *Neurogastroenterol. Mot.,* 1994, 6, 67-78, reports on a study to test the hypothesis that excitatory $A_2$ and inhibitory $A_1$ receptors coexist on myenteric AH/Type 2 neurons, and are positively coupled to adenylate cyclase to stimulate cAMP formation. The results of the study provided positive confirmation of both aspects of the hypothesis.

Larsson *et al.,* "Distribution and effects of pituitary adenylate cyclase activating peptide in cat and human lower oesophageal sphincter", *Br. J. Pharmacol.,* 1995, 116, 2873-2880, describes the results of an investigation into the localization, tissue concentrations, and effects of pituitary adenylate cyclase activating peptide (PACAP), as compared with those of vasoactive intestinal peptide (VIP) and helospectin, in the smooth muscle of cat and human lower oesophageal sphincter (LOS). The results of this investigation established that both PACAP and VIP induced concentration-dependent relaxations in circular smooth muscle (LOS) from both cat and human; that in cat LOS both cAMP and cGMP levels were increased after exposure to PACAP and helospectin, while exposure to VIP was followed by an increase in cAMP levels only; and that in human LOS there was an increase in cAMP levels without any change in cGMP levels.

Tsugeno *et al.,* "Effects of phosphodiesterase inhibitors on spontaneous electrical activity (slow waves) in the guinea-pig gastric muscle", *J. Physiol.,* 1995, 485(2), 493-502, is concerned with the effect of PDE inhibitors on slow waves, which are the rhythmic slow depolarizations of the plasma membrane accompanied by contractions, which are characteristic of guinea-pig gastric muscle. The results of the study described therein demonstrate that the PDE inhibitors caffeine, theophylline, isobutyl methylxanthine, and rolipram all markedly reduce the frequency of slow waves in this muscle and the tension development triggered by slow waves, without altering membrane potential significantly, *i.e.,* without clear membrane hyperpolarization or significant changes in the rate of rise and the duration of slow waves. The results of the study also established that xanthine derivatives inhibited mechanical activity more strongly than electrical activity, which was surmised to be partially due to an inhibitory action of cAMP on the contractile machinery through phosphorylation of myosin light chain kinase. Caffeine and 3-isobutyl-1-methylxanthine (IBMX) may be represented by the following general structural formulas:

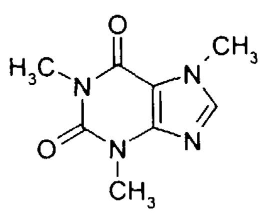

Caffeine

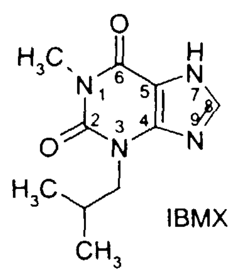

IBMX

Lefebre *et al.,* "Study of NO and VIP as non-adrenergic non-cholinergic neurotransmitters in the pig gastric fundus", *Br. J. Pharmacol.,* 1995, 116, 2017-2026, reports on an investigation of the contribution of nitric oxide (NO) and vasoactive intestinal polypeptide (VIP) to non-adrenergic non-cholinergic (NANC) relaxations in the pig gastric fundus. The results show that electrical field stimulation at intervals induces short-lasting, frequency-dependent relaxations while continuous stimulation induces sustained frequency-dependent relaxations; that forskolin, zaprinast and IBMX induce concentration-dependent relaxations; that zaprinast increases the duration of the NANC relaxations induced by electrical stimulation at intervals in circular, but not longitudinal muscle strips; that sustained electrical stimulation increases both cAMP and cGMP content while electrical stimulation at intervals increases only cGMP content; that NO induces nearly a 40-fold increase in cGMP content; and that analysis for NO synthase reveals immunoreactive neuronal cell bodies in the myenteric plexuses and nerve fibers in both circular and longitudinal muscle. On the basis of these results it was concluded that a cAMP- and cGMP-dependent pathway for relaxation is present in both the circular and longitudinal muscle layer of the pig gastric fundus, that NO appears to contribute to short-lasting as well as sustained NANC relaxations, and that VIP may be involved during sustained stimulation at lower frequencies of

stimulation.

All of the above-mentioned reports from the technical literature concerning *in vitro* studies of the effect of inhibitors having varying degrees of selectivity for the PDE4 isozyme have shown that such inhibitors produce an inhibition of activity in smooth muscle taken, *e.g.*, from the stomach, colon and internal anal sphincter of various test animals. These studies have comprised inhibition of ongoing spontaneous contractions, reduction in tone, i.e., the overall level of tension, and a suppression of the accompanying electrical correlates, *e.g.,* hyperpolarization and reduced probability of spiking. The effects thus obtained and reported are consistent with the actions of neurotransmitters such as VIP which produce relaxation of the gut muscle by means of an elevation in the cAMP intracellular content. From all of these *in vitro* studies and observations, the person of ordinary skill in this art would be persuaded to expect that inhibitors of PDE4 would also produce a relaxation of gut smooth muscle *in vivo,* the functional consequence of which would be a delay in gastric emptying, an increase in gut transit time, and potentially gastric hypomotility or related gastric or gastrointestinal disorders. Thus, the clear teaching of the above-discussed technical literature is that inhibitors of PDE4, if used at all for treating gastric or gastrointestinal disorders, should be used to treat diarrhea.

It was surprising and wholly unexpected, therefore, that after administration of PDE4 inhibitors, there gradually developes an excitatory response consisting of an increase in contraction amplitude rather than tone. This response is particularly marked in the antral region of the stomach of the animal being studied, and has a duration of greater than 30 minutes. In accordance with the present invention PDE4 inhibitors of a wide variety of structural types are found to be suitable for treating gastric hypomotility and related disorders. A detailed description of the different classes of PDE4 inhibitors which may be utilized in the methods of treatment of the present invention may be found further below in the instant specification.

A representative example of art related to structural types of PDE4 inhibitors is Lombardo, "Phosphodiesterase-IV Inhibitors: Novel Therapeutics for the Treatment of Inflammatory Diseases", *Current Pharmaceutical Design,* 1995, 255-268, contains a review and analysis of the structure types of isozyme-specific PDE4 inhibitors currently being pursued by major investigators concerned with their use in the treatment of inflammatory diseases, with a view toward gaining an insight into the direction of PDE4 research in the last half of the current decade. The structures are analyzed therein on a company by company basis.

## SUMMARY OF THE INVENTION

The present invention relates to a method of treating or preventing stasis in all or any part or parts of the stomach of a patient, especially a mammalian patient, and more especially a human patient, in need of such treatment, wherein said stasis results from hypomotility in said stomach or part thereof. The method of treating or preventing stasis to which the present invention relates comprises administering to said patient a therapeutically effective amount of an inhibitor of phosphodiesterase-4 (PDE4), including isozyme subtypes thereof, sufficient to restore normal motility to said patient. The present invention further relates to the above-described method of treatment wherein said stasis comprises gastric hypomotility with delayed emptying of the liquid and/or solid contents of the stomach of said patient. In preferred embodiments of the methods of treatment of the present invention, the mammalian patient being treated is a human.

The present invention still further relates to a method of treating or preventing a gastric or gastrointestinal disorder in a mammalian patient in need of such treatment, wherein said gastric or gastrointestinal disorder is characterized by one or more symptoms selected from pain, nausea, vomiting, heartburn, postprandial discomfort, indigestion and gastroesophageal reflux.

There is further provided a method of treating or preventing a gastric or gastrointestinal disorder in a mammalian patient, especially a human patient, in need of such treatment, wherein said gastric or gastrointestinal disorder is (*i*) a sign or concomitant of diabetic neuropathy, anorexia nervosa, achlorhydria, gastrointestinal surgery, post-surgical recovery in the period of emergence from general anesthesia; or the administration of morphine and morphine-like opioids; (*ii*) a secondary aspect of a primary disease or disorder which is organic, wherein said disease or disorder involves particularly a gastroenteric or gastroesophageal organ or tissue, or an organ or tissue of the central nervous system; or (*iii*) an adverse side effect of a different therapeutic agent administered to said patient in the course of treating another unrelated disease or disorder in said patient.

The present invention also relates to pharmaceutical compositions comprising a pharmaceutically acceptable carrier together with a therapeutically effective amount of an inhibitor of phosphodiesterase-4 including isozyme subtypes thereof, sufficient to restore normal motility to a patient in need thereof. The pharmaceutical compositions of the present invention further include the above-mentioned inhibitor of PDE4 and a pharmaceutically acceptable carrier therefor, used prophylactically together with one or more therapeutic agents which cause or are known to cause gastric hypomotility or related gastric or gastrointestinal disorders when administered to said patient in therapeutically effective amounts, comprising one or more members independently selected from the group consisting essentially of analgesics acting by inhibition of prostaglandin synthesis, which includes most NSAIDs; antacids which contain calcium carbonate

or aluminum hydroxide; anticholinergic agents; antidiarrheal agents; antihistamines which are $H_1$ blockers or have an anticholinergic effect; antiparkinsonian drugs which have an anticholinergic effect; barium sulfate; corticosteroids; clonidine; diuretics which cause hypokalemia; ganglionic blocking agents; heavy metals, including especially lead; iron; laxatives, especially when used chronically; lithium; monoamine oxidase inhibitors; muscle relaxants; octreotide; opioids; phenothiazines having an anticholinergic effect; polystyrene resins; propranolol; tricyclic antidepressants having an anticholinergic effect; and verapamil.

The present invention still further provides the above-mentioned methods of treatment and pharmaceutical compositions useful in said methods of treatment, wherein said inhibitor of phosphodiesterase-4 comprises one or more members independently selected from the group consisting essentially of a compound of Formula (IA) or (IB):

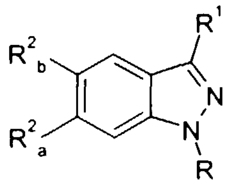

(IA)

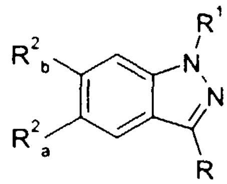

(IB)

and to pharmaceutically acceptable salts thereof, wherein:

- R is a member independently selected from the group consisting essentially of hydrogen; $(C_1\text{-}C_9)$alkyl; $-(CH_2)_n(C_3\text{-}C_{10})$ cycloalkyl wherein n is an integer selected from 0, 1, and 2; $(C_1\text{-}C_6)$ alkoxy$(C_1\text{-}C_6)$ alkyl; $(C_2\text{-}C_6)$ alkenyl; $-(CH_2)_n(C_3\text{-}C_9)$ heterocyclyl wherein n is an integer selected from 0, 1, and 2; and $-(Z^1)_b(Z^2)_c(C_6\text{-}C_{10})$ aryl wherein b and c are integers independently selected from 0 and 1, $Z^1$ is $(C_1\text{-}C_6)$ alkylene or $(C_2\text{-}C_6)$ alkenylene, and $Z^2$ is O, S, $SO_2$, or $NR^{119}$; wherein said heterocyclyl is a member independently selected from the group consisting essentially of acridinyl; benzimidazolyl; benzodioxolane; 1,3-benzodioxol-5-yl; benzo[b]furanyl; benzo[b]thiophenyl; benzoxazolyl; benzthiazolyl; carbazolyl; cinnolinyl; 2,3-dihydrobenzofuranyl; 1,3-dioxane; 1,3-dioxolane; 1,3-dithiane; 1,3-dithiolane; furanyl; imidazolidinyl; imidazolinyl; imidazolyl; 1H-indazolyl; indolinyl; indolyl; 3H-indolyl; isoindolyl; isoquinolinyl; isothiazolyl; isoxazolyl; morpholinyl; 1,8-naphthyridinyl; oxadiazolyl; 1,3-oxathiolane; oxazolidinyl; oxazolyl; oxiranyl; parathiazinyl; phenazinyl; phenothiazinyl; phenoxazinyl; phthalazinyl; piperazinyl; piperidinyl; pteridinyl; pyranyl; pyrazinyl; pyrazolidinyl; pyrazolinyl; pyrazolo[1,5-c]triazinyl; pyrazolyl; pyridazinyl; pyridyl; pyrimidinyl; pyrimidyl; pyrrolyl; pyrrolidinyl; purinyl; quinazolinyl; quinolinyl; 4H-quinolizinyl; quinoxalinyl; tetrazolidinyl; tetrazolyl; thiadiazolyl; thiazolidinyl; thiazolyl; thienyl; thiomorpholinyl; triazinyl; and triazolyl; wherein said aryl is a carbocyclic moiety which is a member independently selected from the group consisting essentially of benzyl; *cis*- and trans-decahydronaphthalenyl; 2,3-1H-dihydroindenyl (indanyl); indenyl; 1-naphthalenyl; 2-naphthalenyl; phenyl; and 1,2,3,4-tetrahydronaphthalenyl; wherein said alkyl, alkenyl, alkoxyalkyl, heterocyclyl, and aryl moieties defining said R groups are substituted by 0 to 3 substituents where each said substituent comprises a member independently selected from the group consisting essentially of bromo, chloro, or fluoro; hydroxy; $(C_1\text{-}C_5)$ alkyl; $(C_2\text{-}C_5)$ alkenyl; $(C_1\text{-}C_5)$alkoxy; $(C_3\text{-}C_6)$ cycloalkoxy; mono-, di-, and tri-fluoromethyl; nitro; $-C(=O)OR^{119}$, $-C(=O)NR^{119}R^{120}$, $-NR^{119}R^{120}$; and $-S(=O)_2NR^{119}R^{120}$;

- $R^1$ is a member independently selected from the group consisting essentially of hydrogen; $(C_1\text{-}C_9)$alkyl; $(C_2\text{-}C_3)$ alkenyl; phenyl; $(C_3\text{-}C_7)$ cycloalkyl; and $(C_3\text{-}C_7)$ cycloalkyl$(C_1\text{-}C_2)$ alkyl; wherein said alkyl, alkenyl and phenyl moieties defining said $R^1$ groups are substituted by 0 to 3 substituents where each said substituent comprises a member independently selected from the group consisting essentially of methyl; ethyl; mono-, di-, and tri-fluoromethyl; and bromo, chloro, or fluoro; and

- $R^2{}_a$ and $R^2{}_b$ are independently selected from the group consisting essentially of hydrogen and substituents hereinafter recited under ( - I - ) through ( - V - ), provided that one, but not both of $R^2{}_a$ and $R^2{}_b$ must be independently selected as hydrogen, wherein said substituents comprise moieties of groups ( - I - ) through ( - V- ) recited below, including those of partial Formulas therein set out, all as defined in detail herein:

( - I - )

-- a moiety of partial Formulas (1.0.0), (1.0.1), (1.0.2), and (1.0.3):

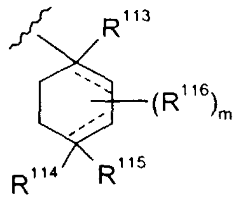
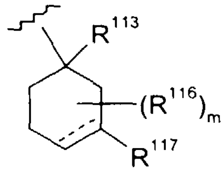
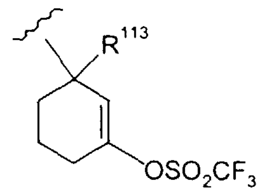
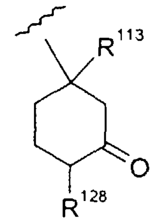

(1.0.0) (1.0.1) (1.0.2) (1.0.3)

--- wherein $R^{113}$, $R^{114}$, $R^{115}$, $R^{116}$, $R^{117}$, and $R^{128}$ are as defined further below;

( - II - )

-- a member selected from the group consisting essentially of $R^{229}$; $-C(=O)NR^{222}(CHR^{222})_mC(=O)NR^{222}O(CH_2)_q(C_6-C_{10})$ aryl); $-C(=NR^{242})NH(CH_2)_p(C_6-C_{10})$ aryl; $-C(=O)NR^{218}(CHR^{222})_mC(=O)NR^{222}(CH_2)_pOR^{222}$; $-C(=O)NR^{222}(CHR^{222})_mS(C_1-C_4)$ alkyl; $-C[=NOC(=O)R^{235}]R^{236}$; $-CR^{227}R^{228}CHR^{238}NR^{219}SO_2(CH_2)_pA$; $-CR^{227}R^{228}CHR^{238}NR^{219}P(=O)(OR^{222})C(=O)(C_1-C_4)$ alkyl; $-CR^{227}R^{238}CHR^{238}NR^{219}P(=O)[(C_1-C_4)alkoxy]_2$, $-Z^3-R^{217}$; and ,$(CR^{227}R^{228})_mNR^{219}(C(O))_qR^{220}$ wherein p is an integer selected from 0, 1, and 2; m is an integer selected from 1, 2, 3, 4, 5, and 6; and q is an integer selected from 1 and 2; and wherein $R^{217}$, $R^{218}$, $R^{219}$, $R^{220}$, $R^{222}$, $R^{227}$, $R^{228}$, $R^{235}$, $R^{236}$, $R^{238}$, and $R^{242}$ are as defined further below; and

-- a moiety of partial Formulas (2.0.0) through (2.0.8), inclusive: -

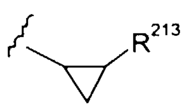
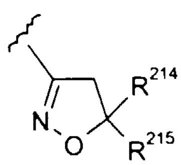
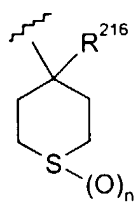
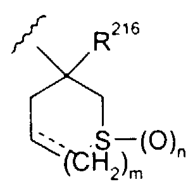
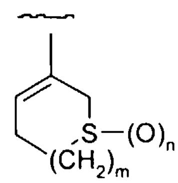

(2.0.0) (2.0.1) (2.0.2) (2.0.3) (2.0.4)

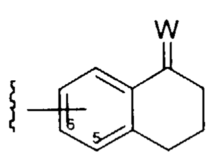
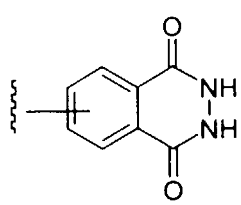
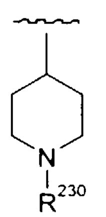
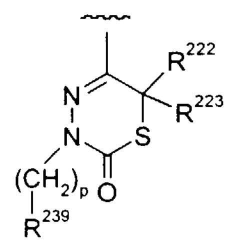

(2.0.5) (2.0.6) (2.0.7) (2.0.8)

--- wherein $R^{223}$, $R^{230}$, and $R^{239}$ are as defined further below;

( - III - )

-- a member independently selected from the group consisting essentially of 2-oxo-4-pyrrolyl; pyrazolyl; 2-oxo-3,4-dihydro-5-pyrimidyl; 2-oxo-3,4-dihydro-4-pyrimidyl; 2-oxo-tetrahydro-4-pyrimidyl; 2-oxo-tetrahyro-5-pyrimidyl; 2-oxo-4-pyrimidyl; and 2-oxo-5-pyrimidyl; wherein each of said $R^2_a$ and $R^2_b$ groups is substituted by 0, 1, 2, 3, or 4 $R^{336}$ groups; and a moiety of partial Formulas (3.0.0) through (3.0.18), inclusive:

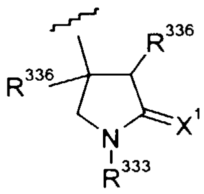 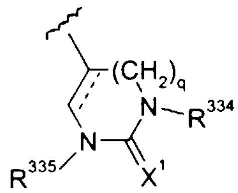 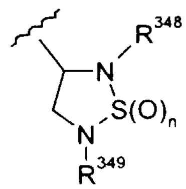 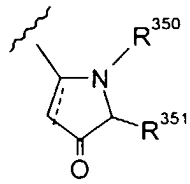 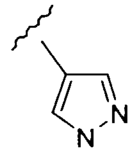

(3.0.0) (3.0.1) (3.0.2) (3.0.3) (3.0.4)

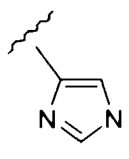 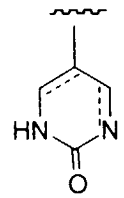 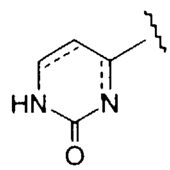 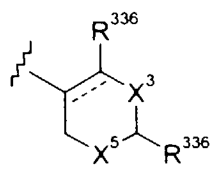 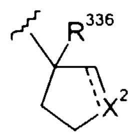

(3.0.5) (3.0.6) (3.0.7) (3.0.8) (3.0.9)

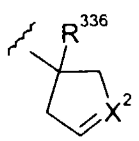 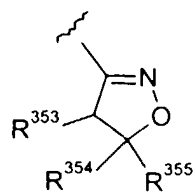 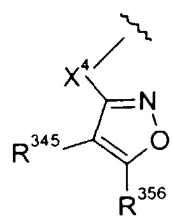 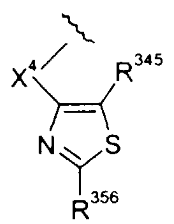 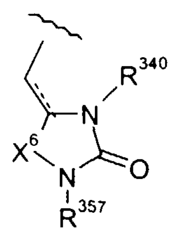

(3.0.10) (3.0.11) (3.0.12) (3.0.13) (3.0.14)

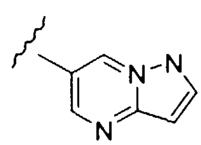 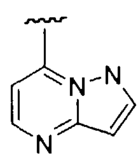 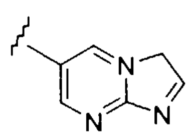 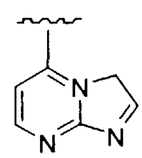

(3.0.15) (3.0.16) (3.0.17) (3.0.18)

--- wherein $R^{333}$, $R^{334}$, $R^{335}$, $R^{336}$, $R^{340}$, $R^{345}$, $R^{348}$, $R^{349}$, $R^{350}$, $R^{351}$, $R^{353}$, $R^{354}$, $R^{355}$, $R^{356}$, and $R^{357}$ are as defined further below;

(- IV -)

-- a moiety of partial Formula (4.0.0):

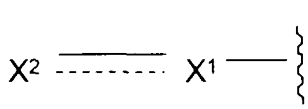

(4.0.0))

--- wherein $X^1$ and $X^2$ are as defined further below; and

( - V - )

-- a moiety of partial Formulas (5.0.0) through (5.0.13), inclusive:

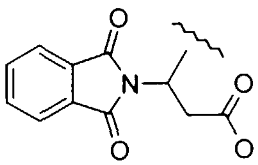
(5.0.0)

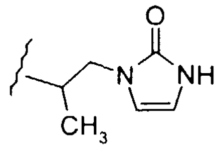
(5.0.1)

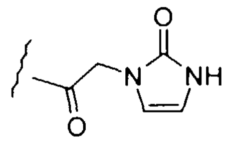
(5.0.2)

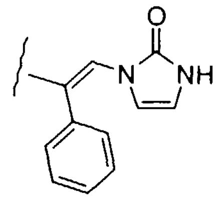
(5.0.3)

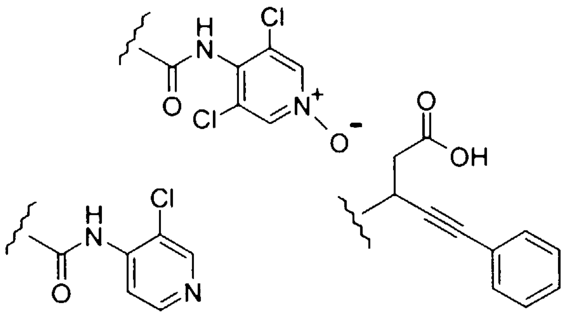
(5.0.4) (5.0.5) (5.0.6)

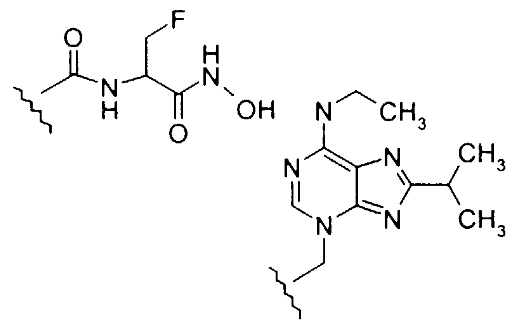
(5.0.7) (5.0.8)

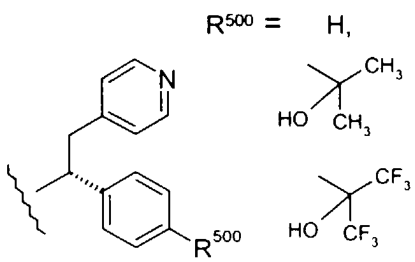
(5.0.9)

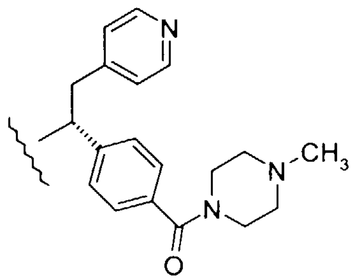
(5.0.10)

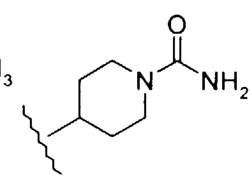
(5.0.11)

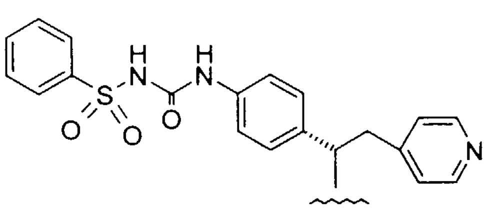
(5.0.12)

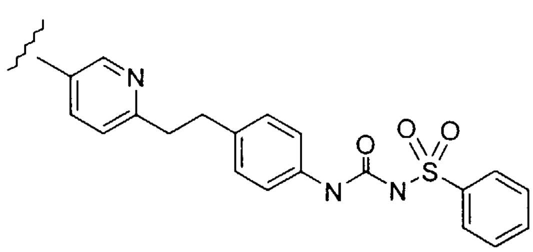
(5.0.13)

DETAILED DESCRIPTION OF THE INVENTION

The inhibitors of phosphodiesterase-4 and isozyme subtypes thereof, which are administered in amounts

sufficient to restore normal gastric motility to a subject being treated in accordance with the present invention, are drawn from a significant number of classes of compounds having different, although not divergent, general chemical structures. These are elaborated upon in the paragraphs which follow in order to provide a better understanding of the intended scope of the present invention.

In particular, the inhibitors of phosphodiesterase-4 and isozyme subtypes thereof, which are administered in amounts sufficient to restore normal gastric motility to a subject being treated in accordance with the present invention, comprise indazole-containing compounds having PDE4 inhibitory activity which are produced by an indazole-for-catechol bioisostere replacement. More particularly, the compositions of matter used as therapeutic agents in the methods and pharmaceutical compositions of the present invention comprise a compound of Formula (IA) or (IB):

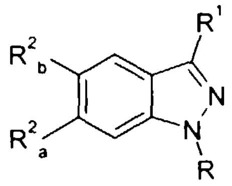

(IA)

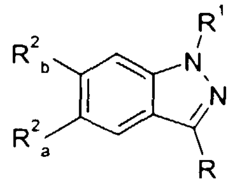

(IB)

and to pharmaceutically acceptable salts thereof, wherein:

- R is a member independently selected from the group consisting essentially of hydrogen, $(C_1\text{-}C_9)$alkyl; $-(CH_2)_n(C_3\text{-}C_{10})$cycloalkyl wherein n is an integer selected from 0, 1, and 2; $(C_1\text{-}C_6)$ alkoxy$(C_1\text{-}C_6)$ alkyl; $(C_2\text{-}C_6)$ alkenyl; $-(CH_2)_n(C_3\text{-}C_9)$ heterocyclyl wherein n is an integer selected from 0, 1, and 2; and $-(Z^1)_b(Z^2)_c(C_6\text{-}C_{10})$ aryl wherein b and c are integers independently selected from 0 and 1, $Z^1$ is $(C_1\text{-}C_6)$ alkylene or $(C_2\text{-}C_6)$ alkenylene, and $Z^2$ is O, S, $SO_2$, or $NR^{119}$; and further wherein said heterocyclyl is a member independently selected from the group consisting essentially of acridinyl; benzimidazolyl; benzodioxolane; 1,3-benzodioxol-5-yl; benzo[*b*]furanyl; benzo[*b*]thiophenyl; benzoxazolyl; benzthiazolyl; carbazolyl; cinnolinyl; 2,3-dihydrobenzofuranyl; 1,3-dioxane; 1,3-dioxolane; 1,3-dithiane; 1,3-dithiolane; furanyl; imidazolidinyl; imidazolinyl; imidazolyl; 1H-indazolyl; indolinyl; indolyl; 3H-indolyl; isoindolyl; isoquinolinyl; isothiazolyl; isoxazolyl; morpholinyl; 1,8-naphthyridinyl; oxadiazolyl; 1,3-oxathiolane; oxazolidinyl; oxazolyl; oxiranyl; parathiazinyl; phenazinyl; phenothiazinyl; phenoxazinyl; phthalazinyl; piperazinyl; piperidinyl; pteridinyl; pyranyl; pyrazinyl; pyrazolidinyl; pyrazolinyl; pyrazolo[1,5-c]triazinyl; pyrazolyl; pyridazinyl; pyridyl; pyrimidinyl; pyrimidyl; pyrrolyl; pyrrolidinyl; purinyl; quinazolinyl; quinolinyl; 4H-quinolizinyl; quinoxalinyl; tetrazolidinyl; tetrazolyl; thiadiazolyl; thiazolidinyl; thiazolyl; thienyl; thiomorpholinyl; triazinyl; and triazolyl; wherein said aryl is a carbocyclic moiety which is a member independently selected from the group consisting essentially of benzyl; *cis*- and trans-decahydronaphthalenyl; 2,3-1H-dihydroindenyl (indanyl); indenyl; 1-naphthalenyl; 2-naphthalenyl; phenyl; and 1,2,3,4-tetrahydronaphthalenyl; wherein said alkyl, alkenyl, alkoxyalkyl, heterocyclyl, and aryl moieties defining said R groups are substituted by 0 to 3 substituents where each said substituent comprises a member independently selected from the group consisting essentially of bromo, chloro, or fluoro; hydroxy; $(C_1\text{-}C_5)$ alkyl; $(C_2\text{-}C_5)$ alkenyl; $(C_1\text{-}C_5)$alkoxy; $(C_3\text{-}C_6)$ cycloalkoxy; mono-, di-, and tri-fluoromethyl; nitro; $-C(=O)OR^{119}$, $-C(=O)NR^{119}R^{120}$, $-NR^{119}R^{120}$ and $-S(=O)_2NR^{119}R^{120}$;
- $R^1$ is a member independently selected from the group consisting essentially of hydrogen; $(C_1\text{-}C_9)$ alkyl; $(C_2\text{-}C_3)$ alkenyl; phenyl; $(C_3\text{-}C_7)$ cycloalkyl; and $(C_3\text{-}C_7)$ cycloalkyl$(C_1\text{-}C_2)$ alkyl; wherein said alkyl, alkenyl and phenyl moieties defining said $R^1$ groups are substituted by 0 to 3 substituents where each said substituent comprises a member independently selected from the group consisting essentially of methyl; ethyl; mono-, di-, and tri-fluoromethyl; and bromo, chloro, or fluoro; and
- $R^2_a$ and $R^2_b$ are independently selected from the group consisting essentially of hydrogen and hereinafter recited substituents, provided that one, but not both of $R^2_a$ and $R^2_b$ must be independently selected as hydrogen, wherein said substituents comprise moieties of the groups ( - I - ) through ( - V - ):

    -- ( - I - )

    -- a moiety of partial Formulas (1.0.0), (1.0.1), (1.0.2), and (1.0.3):

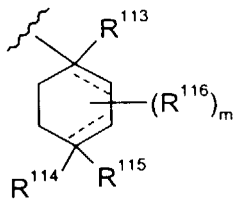 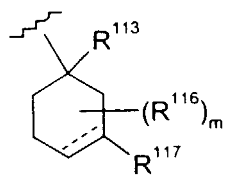 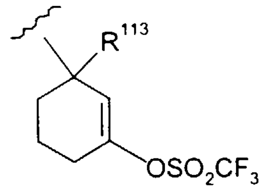 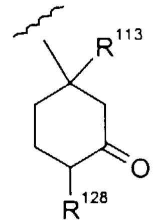

(1.0.0) (1.0.1) (1.0.2) (1.0.3)

--- wherein the dashed lines in partial Formulas (1.0.0) and (1.0.1) independently and optionally represent a single or double bond, provided that in formula (1.0.0) both dashed lines cannot both represent double bonds at the same time;

--- m is an integer selected from 0, 1, 2, 3, and 4, and when 2, may apply to a single carbon atom on the ring;

--- $R^{113}$ is a member selected from the group consisting essentially of H; bromo, chloro, or fluoro; cyano; $(C_2-C_4)$ alkynyl substituted by 0 or 1 substituent where said substituent is a member selected from the group consisting essentially of phenyl, pyridyl and pyrimidinyl; $(C_1-C_4)$alkyl substituted by 0 to 6 bromo, chloro, or fluoro; $-CH_2NHC(=O)C(=O)NH_2$; cyclopropyl substituted by 0 or 1 substituent where said substituent is a member selected from the group consisting essentially of $R^{121}$; $R^{127}$; $CH_2OR^{119}$; $NR^{119}R^{120}$; $CH_2NR^{119}R^{120}$; $C(=O)OR^{119}$; $C(=O)NR^{119}R^{120}$; $C{\equiv}CR_{11}$; C(Z)H; and $-CH{=}CR^{121}R^{121}$; provided that $R^{113}$ is H in Formula (1.0.0) when the dashed line for the ring carbon of $R^{113}$ attachment represents a double bond;

--- $R^{114}$ is a member selected from the group consisting essentially of H; $R^{116}$; $C(Y)R^{124}$; $C(=O)OR^{124}$; $C(Y)NR^{127}R^{124}$; CN; $C(NR^{127})NR^{127}R^{124}$; $C(NOR^{119})R^{124}$; $C(=O)NR^{119}NR^{119}C(=O)R^{119}$; $C(=O)NR^{119}NR^{127}R^{124}$; $C(NOR^{124})R^{119}$; $C(NR^{119})NR^{127}R^{124}$; $C(NR^{124})NR^{119}R^{120}$; $C(NCN)NR^{127}R^{124}$, $C(NCN)S(C_1-C_4)$ alkyl; $CR^{119}R^{120}OR^{124}$, $CR^{119}R^{120}SR^{124}$, $CR^{119}R^{120}S(O)_nR^{125}$ where n is an integer selected from 0, 1, and 2; $CR^{119}R^{120}NR^{124}R^{127}$; $CR^{119}R^{120}NR^{127}S(=O)_2R_{15}$; $CR^{119}R^{120}NR^{127}C(Y)R^{124}$; $CR^{119}R^{120}NR^{127}C(=O)OR^{125}$; $CR^{119}R^{120}NR^{127}C(Y)NR^{127}R^{124}$; $CR^{119}R^{120}NR^{127}C(NCN)NR^{127}R^{124}$; $CR^{119}R^{120}NR^{127}C(CR^{119}NO_2)S(C_1-C_4)$ alkyl; $CR^{119}R^{120}C(=O)OR^{125}$; $CR^{119}R^{120}C(Y)NR^{127}R^{124}$; $CR^{119}R^{120}C(NR^{127})NR^{127}R^{124}$; $CR^{119}R^{120}CN$; $CR^{119}R^{120}C(NOR^{120})R^{124}$; $CR^{119}R^{120}C(NOR^{124})R^{120}$; $CR^{119}R^{120}NR^{127}C(NR^{127})S(C_1-C_4)$ alkyl; $CR^{119}R^{120}NR^{127}C(NR^{127})NR^{127}R^{124}$; $CR^{119}R^{120}NR^{127}C(=O)C(=O)NR^{127}R^{124}$; $CR^{119}R^{120}NR^{127}C(=O)C(=O)OR^{124}$; tetrazolyl; thiazolyl; imidazolyl; imidazolidinyl; pyrazolyl; thiazolidinyl; oxazolyl; oxazolidinyl; triazolyl; isoxazolyl; oxadiazolyl; thiadiazolyl; $CR^{119}R^{120}$(tetrazolyl); $CR^{119}R^{120}$(thiazolyl); $CR^{119}R^{120}$(imidazolyl); $CR^{119}R^{120}$(imidazolidinyl); $CR^{119}R^{120}$(pyrazolyl); $CR^{119}R^{120}$(thiazolidinyl); $CR^{119}R^{120}$(oxazolyl); $CR^{119}R^{120}$(oxazolidinyl); $CR^{119}R^{120}$(triazolyl); $CR^{119}R^{120}$(isoxazolyl); $CR^{119}R^{120}$(oxadiazolyl); $CR^{119}R^{120}$(thiadiazolyl); $CR^{119}R^{120}$(morpholinyl); $CR^{119}R^{120}$(piperidinyl); $CR^{119}R^{120}$(piperazinyl); and $CR^{119}R^{120}$(pyrrolyl); said heterocyclic groups being substituted by 0 to 3 substituents $R^{124}$;

--- $R^{115}$ is a member selected from the group consisting essentially of $R^{119}$; $OR^{119}$; $-CH_2OR^{119}$; cyano; $C(=O)R^{119}$; $C(=O)OR^{119}$; $C(=O)NR^{119}R^{120}$; and $NR^{119}R^{120}$; provided that $R^{115}$ is absent when the dashed line in partial Formula (1.0.0) represents a double bond; or

--- $R^{114}$ and $R^{115}$ are taken together to form =O or $=R^{118}$; or

--- $R^{115}$ is hydrogen and $R^{114}$ is $OR^{124}$; $SR^{124}$; $S(O)_nR^{125}$, where n is an integer selected from 0, 1, and 2; $S(=O)_2NR^{127}R^{124}$; $NR^{127}R^{124}$; $NR^{124}C(=O)R^{119}$; $NR^{127}C(Y)R^{124}$; $NR^{127}C(=O)OR^{125}$; $NR^{127}C(Y)NR^{127}R^{124}$; $NR^{127}S(=O)_2NR^{127}R^{124}$; $NR^{127}C(NCN)NR^{127}R^{124}$; $NR^{127}S(=O)_2R^{125}$; $NR^{127}C(CR^{119}NO_2)NR^{127}R^{124}$; $NR^{127}C(NCN)S(C_1-C_4)$ alkyl; $NR^{127}C(CR^{119}NO_2)S(C_1-C_4)$alkyl; $NR^{127}C(NR^{127})NR^{127}R^{124}$; $NR^{127}C(=O)C(=O)NR^{127}R^{124}$; or $NR^{127}C(=O)C(=O)OR^{124}$;

--- $R^{116}$ is a member independently selected from the group consisting essentially of methyl and ethyl substituted by 0 to 5 bromo, chloro, or fluoro, wherein m may be 2 with respect to a single ring carbon atom to which $R^{116}$ is attached;

--- $R^{117}$ is a member independently selected from the group consisting essentially of $OR^{124}$; $SR^{124}$; $SO_2NR^{127}R^{124}$; $NR^{127}R^{124}$; $NR^{124}C(=O)R^{119}$; $NR^{127}C(Y)R^{124}$; $NR^{127}C(=O)OR^{125}$; $S(O)_nR_{12}$ where n is an integer selected from 0, 1, and 2; $OS(=O)_2R^{122}$; $OR^{122}$; $OC(=O)NR^{123}R^{122}$; $OC(=O)R^{123}$; $OC(=O)OR^{123}$; $O(CR^{122}R^{123})_mOR^{122}$ where m is an integer selected from 0, 1, and 2; $CR^{119}R^{120}R^{124}$; $CR^{119}R^{120}NR^{127}R^{124}$; $C(Y)R^{124}$; $C(=O)OR^{124}$; $C(Y)NR^{127}R^{124}$; CN; $C(NR^{127})NR^{127}R^{124}$; $C(NOR^{119})R^{124}$; $C(=O)NR^{119}NR^{119}C(=O)R^{119}$; $C(=O)NR^{119}NR^{127}R^{124}$; $C(NOR^{124})R^{119}$; $C(NR^{119})NR^{127}R^{124}$; $C(NR^{124})NR^{119}R^{120}$; $C(NCN)NR^{127}R^{124}$; $C(NCN)S(C_1\text{-}C_4)$alkyl; tetrazolyl; thiazolyl; imidazolyl; imidazolidinyl; pyrazolyl; thiazolidinyl; oxazolyl; oxazolidinyl; triazolyl; isoxazolyl; oxadiazolyl; and thiadiazolyl; where the recited heterocyclic groups are substituted by 0 to 3 substituents where said substituent is $R^{124}$;

---- $R^{118}$ is a member independently selected from the group consisting essentially of $-NR^{125}$; $-NCR^{119}R^{120}(C_2\text{-}C_6)$alkenyl; $-NOR^{124}$; $-NOR^{129}$; $-NOCR^{119}R^{120}(C_2\text{-}C_6)$alkenyl; $-NNR^{119}R^{124}$; $-NNR^{119}R^{129}$; -NCN; $-NNR^{119}C(Y)NR^{119}R^{124}$; $-C(CN)_2$; $-CR^{124}CN$; $-CR^{124}C(=O)OR^{119}$; $-CR^{124}C(=O)NR^{119}R^{124}$; $-C(CN)NO_2$; $-C(CN)C(=O)O(C_1\text{-}C_4)$ alkyl; $-C(CN)OC(=O)O(C_1\text{-}C_4)$ alkyl; $-C(CN)(C_1\text{-}C_4)$ alkyl; $-C(CN)C(=O)NR^{119}R^{124}$; 2-(1,3-dithiane), 2-(1,3-dithiolane), dimethylthio ketal, diethylthio ketal, 2-(1,3-dioxolane), 2-(1,3-dioxane), 2-(1,3-oxathiolane); dimethyl ketal and diethyl ketal;

---- $R^{119}$ and $R^{120}$ are each a member independently selected from the group consisting essentially of hydrogen and $(C_1\text{-}C_4)$ alkyl substituted by 0 to 3 fluorine atoms;

---- $R^{121}$ is a member independently selected from the group consisting essentially of fluoro and $R^{120}$;

---- $R^{122}$ is a member independently selected from the group consisting essentially of $(C_1\text{-}C_6)$ alkyl; $(C_2\text{-}C_3)$ alkenyl; $(C_3\text{-}C_7)$ cycloalkyl; $(C_3\text{-}C_7)$ cycloalkyl$(C_1\text{-}C_2)$ alkyl; $(C_6\text{-}C_{10})$ aryl; and $(C_3\text{-}C_9)$ heterocyclyl; where said aryl and heterocyclyl are as defined under R above; and where said $R^{122}$ groups are substituted with 0 to 3 substituents independently selected from the group consisting essentially of methyl; ethyl; mono-, di-, and trifluoromethyl; and bromo, chloro, or fluoro;

---- $R^{123}$ is a member independently selected from the group consisting essentially of hydrogen and $R^{122}$;

---- $R^{124}$ is a member independently selected from the group consisting essentially of hydrogen and $R^{125}$; or when $R^{124}$ and $R^{127}$ appear together as $NR^{127}R^{124}$ then $R^{127}$ and $R^{124}$ may be taken together with the nitrogen to which they are attached to form a 5- to 7-membered ring optionally containing one additional heteroatom selected from O, N and S;

---- $R^{125}$ is a member independently selected from the group consisting essentially of $(C_1\text{-}C_6)$ alkyl and $-(CR^{119}R^{120})_nR^{126}$, where n is an integer selected from 0, 1, and 2 and $R^{126}$ and said $(C_1\text{-}C_6)$ alkyl are substituted by 0 to 3 substituents where each said substituent is a member independently selected from the group consisting essentially of bromo, chloro, or fluoro; nitro; cyano; $NR^{120}R^{127}$; $C(=O)R^{119}$; $OR^{119}$; $C(=O)NR^{120}R^{127}$; $OC(=O)NR^{120}R^{127}$; $NR^{127}C(=O)NR^{127}R^{120}$; $NR^{127}C(=O)R^{120}$; $NR_{17}C(=O)O(C_1\text{-}C_4)$ alkyl; $C(NR^{127})NR^{127}R^{120}$; $C(NCN)NR^{127}R^{120}$; $C(NCN)S(C_1\text{-}C_4)$ alkyl; $NR^{127}C(NCN)S(C_1\text{-}C_4)$ alkyl; $NR^{127}C(NCN)NR^{127}R^{120}$; $NR^{127}S(=O)_2(C_1\text{-}C_4)$ alkyl; $S(O)_n(C_1\text{-}C_4)$ alkyl; where n is an integer selected from 0, 1, and 2; $NR^{127}C(=O)C(=O)NR^{127}R^{120}$, $NR^{127}C(=O)C(=O)R^{127}$; thiazolyl; imidazolyl; oxazolyl; pyrazolyl; triazolyl; tetrazolyl; and $(C_1\text{-}C_2)$ alkyl substituted with 0 to 3 fluorine atoms;

---- $R^{126}$ is a member independently selected from the group consisting essentially of $(C_3\text{-}C_7)$ cycloalkyl; pyridyl; pyrimidyl; pyrazolyl; imidazolyl; triazolyl; pyrrolyl; piperazinyl; piperidinyl; morpholinyl; furanyl; thienyl; thiazolyl; quinolinyl; naphthyl; and phenyl;

---- $R^{127}$ is a member independently selected from the group consisting essentially of $OR^{119}$ and $R^{120}$;

---- $R^{128}$ is a member independently selected from the group consisting essentially of H; $C(Y)R^{124}$; $C(=O)OR^{124}$; $C(Y)NR^{127}R^{124}$; CN; $C(NR^{127})NR^{127}R^{124}$; $C(NOR^{119})R^{124}$; $C(=O)NR^{119}NR^{119}C(=O)R^{119}$; $C(=O)NR^{119}NR^{127}R^{124}$; $C(NOR^{124})R^{119}$; $C(NR^{119})NR^{127}R^{124}$; $C(NR^{124})NR^{119}R^{120}$; $C(NCN)NR^{127}R^{124}$; $C(NCN)S(C_1\text{-}C_4)$ alkyl; $CR^{119}R^{120}OR^{124}$; $CR^{119}R^{120}SR^{124}$; $CR^{119}R^{120}S(O)_nR^{125}$, where n is an integer selected from 0, 1, and 2; $CR^{119}R^{120}NR^{124}R^{127}$; $CR^{119}R^{120}NR^{127}S(=O)_2R^{125}$; $CR^{119}R^{120}NR^{127}C(Y)R^{124}$; $CR^{119}R^{120}NR^{127}C(=O)OR^{125}$; $CR^{119}R^{120}NR^{127}C(Y)NR^{127}R^{124}$; $CR^{119}R^{120}NR^{127}C(NCN)NR^{127}R^{124}$; $CR^{119}R^{120}NR^{127}C(CR_9NO_2)S(C_1\text{-}C_4)$ alkyl; tetrazolyl; thiazolyl; imidazolyl; imidazolidinyl; pyrazolyl; thiazolidinyl; oxazolyl; oxazolidinyl; triazolyl; isoxazolyl; oxadiazolyl; thiadiazolyl; wherein said recited heterocyclic groups are substituted by 0 to 3 substituents where each said substituent is independently selected from the group consisting essentially of $R^{124}$;

---- $R^{129}$ is a member independently selected from the group consisting essentially of -C(=O)$R^{12}$; -C(=O)$NR^{119}R^{124}$; -S(=O)$_2R^{125}$; and -S(=O)$_2NR^{119}R^{124}$;

---- Y is O or S; and,

---- Z is O; $NR^{127}$; NCN; C(-CN)$_2$; $CR^{119}CN$; $CR^{119}NO_2$; $CR^{119}C(=O)OR^{119}$; $CR^{119}C(=O)NR^{119}R^{120}$; C(-CN)C(=O)O($C_1$-$C_4$) alkyl); and C(-CN)C(=O)$NR^{119}R^{120}$;

- or, said substituents defining $R^2{}_a$ and $R^2{}_b$ comprise: -

-- ( - II - )

-- a member selected from the group consisting essentially of $R^{229}$; -C(=O)$NR^{222}(CHR^{222})_mC(=O)NR^{222}O(CH_2)_q$($C_6$-$C_{10}$) aryl); -C(=$NR^{242}$)NH$(CH_2)_p$($C_6$-$C_{10}$) aryl; -C(=O)$NR^{218}(CHR^{222})_mC(=O)NR^{222}(CH_2)_pOR^{222}$; -C(=O)$NR^{222}(CHR^{222})_mS(C_1$-$C_4)$ alkyl; -C[=NOC(=O)$R^{235}$]$R^{236}$; -$CR^{227}R^{228}CHR^{238}NR^{219}SO_2(CH_2)_pA$; -$CR^{227}R^{228}CHR^{238}NR^{219}P(=O)(OR^{222})C(=O)(C_1$-$C_4)$alkyl; -$CR^{227}R^{238}CHR^{238}NR^{219}P(=O)[(C_1$-$C_4)alkoxy]_2$, -$Z^3$-$R^{217}$; and -$(CR^{227}R^{228})_mNR^{219}(C(O))_qR^{220}$ wherein p is an integer selected from 0, 1, and 2; m is an integer selected from 1, 2, 3, 4, 5, and 6; and q is an integer selected from 1 and 2;

- or, said substituents defining $R^2{}_a$ and $R^2{}_b$ comprise a moiety of partial Formulas (2.0.0) through (2.0.8), inclusive: -

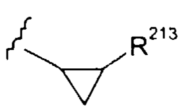
(2.0.0)

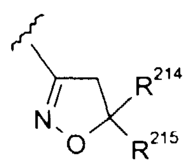
(2.0.1)

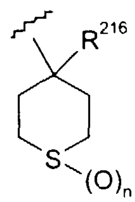
(2.0.2)

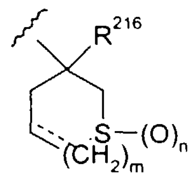
(2.0.3)

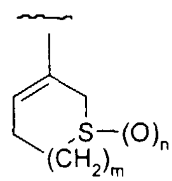
(2.0.4)

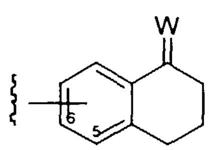
(2.0.5)

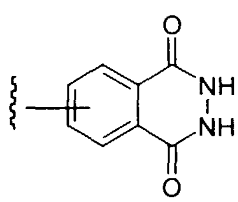
(2.0.6)

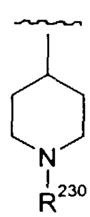
(2.0.7)

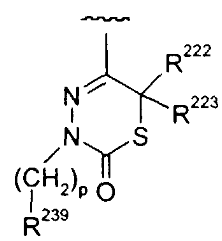
(2.0.8)

--- wherein in said partial Formulas (2.0.0)-(2.0.8), the structures of partial Formulas (2.0.5) and (2.0.6) are attached to the nucleus of Formula (IA) or (IB) at carbons 5, 6, or 7 of said partial Formulas (2.0.5) and (2.0.6); the dashed line in partial Formulas (2.0.2) and (2.0.3) indicates a single bond or double bond, except that $R^{216}$ is absent in partial Formulas (2.0.2) and (2.0.3) where said dashed line indicates a double bond; n is an integer selected from 0, 1, and 2; p is an integer selected from 0, 1, 2, 3, 4, 5, and 6; and m is an integer selected from 0, and 1;

--- $R^{213}$ is a member independently selected from the group consisting essentially of -C(=O)N($CH_3$)($OCH_3$) and -$(CH_2)_n$OH, where n is an integer selected from 0, 1, 2, 3, and 4;

--- $R^{214}$ and $R^{215}$ are independently selected from the group consisting essentially of H; ethyl; -$CO_2$H; and -C(=O)

NHOH;

--- $R^{216}$ is a member independently selected from the group consisting essentially of H; hydroxy; $(C_1-C_6)$ alkyl; $(C_1-C_6)$ alkoxy; $-OC(=O)(C_1-C_6)$ alkyl and $-OC(=O)(C_6-C_{10})$ aryl;

---- $R^{217}$ is a member independently selected from the group consisting essentially of $(C_6-C_{10})$ aryl and a 5- to 10-membered heterocyclyl, wherein said $R^{217}$ groups are substituted by 0 to 3 substituents independently selected from the group consisting essentially of bromo, chloro, or fluroro; trifluoromethyl; cyano; nitro; $-CO_2R^{222}$, $(C_1-C_4)$ alkoxy; $-OC(=O)(C_1-C_4)$ alkyl; $-NR^{222}C(=O)(C_1-C_4)$ alkyl; $-C(=O)NH_2$; -C(=O)NHOH; $-C(=O)O(C_1-C_4)$ alkyl; $(C_1-C_4)$ alkyl; $-S(O)_nR^{222}$ where n is an integer selected from 0, 1, and 2; benzoyl; $-NR^{222}R^{223}$, $-OR^{222}$, $(C_1-C_6)$alkanoyl; $-Y^1-(C_6-C_{10})$aryl; $-C(=O)O(C_6-C_{10})$ aryl; $-NH(C_6-C_{10})$ aryl; $-C(=O)NH(C_6-C_{10})$ aryl; $-C(=O)NR^{222}O(CH_2)_n(C_6-C_{10})$ aryl, where n is an integer selected from 1, 2, and 3; and $-SO_2NH(C_6-C_{10})$ aryl;

---- $R^{218}$ is a member independently selected from the group consisting essentially of H; $(C_1-C_6)$ alkyl; and $-(CH_2)_n(C_6-C_{10})$ aryl, where n is an integer selected from 0, 1, 2, 3, and 4;

---- $R^{219}$ is a member independently selected from the group consisting essentially of H; $-OR^{222}$; $-(CH_2)_mA$ ; and $-CH_2O(CH_2)_mA$, where m is an integer selected from 0, 1, and 2;

---- $R^{220}$ is a member independently selected from the group consisting essentially of $(C_1-C_4)$alkyl; $-OR^{222}$, $-CR^{222}R^{223}OR^{222}$; $-CR^{222}R^{223}NR^{222}R^{223}$; $-CR^{222}(OR^{223})CR^{222}R^{223}OR^{222}$; 2,2-dimethyl-1,3-dioxolan-4-yl; $-NR^{222}C(=O)NR^{222}R^{223}$, $-S(CR^{222}R^{223})_nCH_3$ where n is an integer selected from 0, 1, 2, 3, 4, and 5; $-NR^{222}(CH_2)_q$(pyridyl) where q is an integer selected from 0 and 1; $-P(=O)((C_1-C_4)\ alkoxy)]_2$; $-NR^{222}R^{223}$; $-NR^{222}OR^{223}$; $-NR^{222}NR^{223}R^{221}$, $-NR^{222}CH_2R^{224}$; $-OCH_2NR^{222}C(=O)R^{224}$; $-OCH_2C(=O)NR^{225}R^{226}$, $-OCHR^{222}OC(=O)(C_1-C_4)$ alkyl; $-OCHR^{222}C(=O)(C_1-C_3)$ alkoxy; $-O(CH_2)_mR^{221}$; and $-NR^{222}(CH_2)_mR^{221}$ where m is an integer selected from 0, 1, and 2;

---- $R^{221}$ is a member independently selected from the group consisting essentially of H and A;

--- $R^{222}$ and $R^{223}$ are each a member independently selected from the group consisting essentially of H and $(C_1-C_4)$ alkyl;

---- $R^{224}$ is a member independently selected from the group consisting essentially of methyl and phenyl;
---- $R^{225}$ is a member independently selected from the group consisting essentially of H; methyl; ethyl; and $-CH_2CH_2OH$;
---- $R^{226}$ is a member independently selected from the group consisting essentially of H; methyl; ethyl; $-CH_2C(=O)NH_2$; and $-CH_2CH_2OH$;
---- $R^{227}$ is each a member independently selected from the group consisting essentially of H; hydroxy; cyano; halo; $(C_1-C_3)$ alkyl; $(C_1-C_3)$ alkoxy; $-NR^{222}R^{223}$; $-C(=O)OR^{222}$, $-C(=O)R^{222}$; $-CH=CR^{222}R^{223}$; $-C\equiv CR^{222}$; $-CH_2NR^{222}R^{223}$; $-CH_2OR^{222}$; $-C(=O)NR^{222}R^{223}$; $-C(Y^5)H$; and $-CH_2NR_{12}C(=O)C(=O)NR^{222}R^{223}$; provided that when $R^{227}$ is hydroxy then $R^{228}$ is H or $(C_1-C_4)$ alkyl;
---- $R^{228}$ is each a member independently selected from the group consisting essentially of H; fluoro; cyano; and $(C_1-C_4)$ alkyl; where said methyl is substituted by 0 to 3 substituents each comprising a fluorine atom; or
---- $R^{227}$ and $R^{228}$ are taken together to form an oxo (=O) moiety;

--- $R^{229}$ is a member independently selected from the group consisting essentially of phenyl; naphthyl; pyrrolyl; furanyl; thienyl; oxazolyl; pyridinyl; pyrimidinyl; pyridazinyl; quinolinyl; isoquinolinyl; 5,6,7,8-tetrahydroquinolinyl; and 5,6,7,8-tetrahydroisoquinolinyl, where said $R^{229}$ groups, except said phenyl, are substituted by 0 to 3 substituents $R^{233}$, and wherein said phenyl $R^{229}$ group is substituted by 0 to 3 substituents independently selected from $R^{233}$ and $R^{234}$;
--- $R^{230}$ is a member independently selected from the group consisting essentially of $-C(=O)R^{231}$; $-C(=O)C(=O)R^{231}$, $-C(=O)C(Y^2)C(=O)R^{231}$ and a moiety of partial Formula (2.0.9):

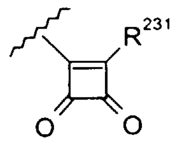

(2.0.9)

wherein:

---- $R^{231}$ is a member independently selected from the group consisting essentially of H; $-OR^{232}$; $-NHR^{232}$; -NHOH; $-NHNH_2$; $-(CH_2)_nY^3$(phenyl) and $-(CH_2)_nY^3$(pyridyl) where n is an integer selected from 0, 1, 2, 3, and 4;

----- $R^{232}$ is a member independently selected from the group consisting essentially of H; $(C_1-C_8)$ alkyl; $-(CH_2)_nY^3$ (phenyl) and $-(CH_2)_nY^3$(pyridyl) where n is an integer selected from 0, 1, 2, 3, and 4;

----- $R^{233}$ is each a member independently selected from the group consisting essentially of bromo, chloro, or fluoro; $(C_1-C_6)$ alkyl; $(C_1-C_7)$ alkoxy; $(C_2-C_6)$ alkylenedioxy; trifluoromethyl; $-NR^{222}R^{223}$; nitro; $-C(NR^{222})NR^{222}R^{223}$; $-C(=O)NR^{222}R^{223}C(=O)R^{222}$; $-C(NOR^{222})R^{223}$; $-C(NCN)NR^{222}R^{223}$; $-C(NCN)SR^{222}$; $-(CH_2)_m$(CN) where m is an integer selected from 0, 1, 2, and 3; hydroxy; $-C(=O)R^{222}$, $-C(=O)NR^{222}OR^{223}$; $-C(=O)NR^{222}NR^{222}R^{223}$, $-OC(=O)N\ R^{222}R^{223}$; $-NR^{222}C(=O)R^{222}$; $-C(=O)C(=O)NR^{222}R^{223}$; $-CO_2R^{222}$, $-SO_2R^{222}$; $-SO_2NR^{222}R^{223}$; $-C(=O)NR^{222}R^{223}$; $-NR^{222}SO_2R^{223}$; and $-NR^{222}C(=O)NR^{222}R^{223}$;

----- $R^{234}$ is each a member independently selected from the group consisting essentially of imidazolyl; pyrazolyl; triazolyl; tetrazolyl; oxazolyl; isoxazolyl; oxadiazolyl; thiadiazolyl; thiazolyl; oxazolidinyl; thiazolidinyl; and imidazolidinyl, where each of said foregoing $R^{234}$ substituents is substituted by 0 to 3 substituents $R^{233}$;

----- $R^{235}$ is a member independently selected from the group consisting essentially of $-NR^{222}R^{223}$; $-NH(C_6-C_{10})$ aryl; $(C_1-C_6)$ alkoxy; and $(C_6-C_{10})$ aryloxy;

----- $R^{236}$ is a member independently selected from the group consisting essentially of H; $(C_1-C_6)$ alkyl and $-(CH_2)_mY^4$(phenyl) where m is an integer selected from 0, 1, 2, 3, and 4 and the phenyl moiety of said $-(CH_2)_mY^4$(phenyl)$R^{236}$ group is substituted by 0 to 3 substituents independently selected from the group consisting essentially of bromo, chloro, or fluoro; $-OR^{222}$; $(C_1-C_6)$ alkanoyloxy; $(C_6-C_{10})$ aryloxy; $-NR^{222}R^{223}$; $-NH(C_6-C_{10})$ aryl; and $-NHC(=O)(C_1-C_4)$ alkyl;

----- $R^{237}$ is each a member independently selected from the group consisting essentially of bromo, chloro, or fluoro; $-(CH_2)_pNR^{222}C(=O)CH_3$ where p is an integer selected from 1, 2, 3, 4, and; $(C_1-C_4)$alkoxy; nitro; cyano; $-NR^{222}R^{223}$; $-CO_2R^{222}$; $-OR^{222}$; $-C(Y^1)NR^{222}R^{223}$; $-NR^{222}C(NCN)S(C_1-C_3)$ alkyl; $-NR^{222}C(NCN)NR^{222}R^{223}$; $-NR^{222}C(=O)NR^{222}R^{223}$; $-NR^{222}C(=O)C(=O)NR^{222}R^{223}$; $-C(=NR^{222})NR^{222}R^{223}$; $-S(O)_mCH_3$ where m is an integer selected from 0, 1, and 2; $-C(=NR^{222})S(C_1-C_3)$ alkyl; $-NR^{222}SO_2(C_1-C_3)$ alkyl; $-OC(=O)R^{222}$; $-OC(=O)NR^{222}R^{223}$; $-NR^{222}SO_2CF_3$; $-NR^{222}C(=O)C(=O)OR^{222}$; $-NR^{222}C(=O)R^{222}$; $-NR^{222}C(=O)OR^{222}$; imidazolyl; thiazolyl; oxazolyl; pyrazolyl; triazolyl; and tetrazolyl;

----- $R^{238}$ is a member independently selected from the group consisting essentially of H; fluoro; cyano; and $(C_1-C_2)$alkyl, where said alkyl is substituted by 0 to 3 substituents independently selected from the group consisting essentially of bromo, chloro, or fluoro; $-C(=O)NR^{222}R^{223}$; and $-C(=O)OR^{222}$;

----- $R^{239}$ is a member independently selected from the group consisting essentially of phenyl substituted by 0 to 2 substituents independently selected from $-NR^{222}R^{223}$, nitro, halo, $-OR^{222}$, $-NHR^{240}$, $-NR^{240}R^{241}$, and $-C(=O)OR^{222}$;

----- $R^{240}$ and $R^{241}$ are each a member independently selected from the group consisting essentially of $(C_1-C_8)$ alkyl and $(C_2-C_8)$ alkenyl;

----- $R^{242}$ is pyridin-4-yl substituted by 0 to 2 substituents independently selected from the group consisting essentially of bromo, chloro, or fluoro; and $(C_1-C_4)$ alkyl;

----- A is each a member independently selected from the group consisting essentially of $(C_1-C_6)$alkyl; pyridyl; morpholinyl; piperidinyl; imidazolyl; thienyl; pyrimidyl; thiazolyl; triazolyl; quinolinyl; phenyl; and naphthyl; wherein the foregoing A groups are substituted with 0 to 3 substituents $R^{237}$; or A is $-(CH_2)_qS(C_1-C_4)$ alkyl wherein q is an integer selected from 1 and 2;

----- W is a member independently selected from the group consisting essentially of O; NOH; $NNH_2$; $NOC(=O)CH_3$; and $NNHC(=O)CH_3$;

----- $Y^1$ is O or S;

----- $Y^2$ is O, NOH or $H_2$;

----- $Y^3$ is a bond or -CH=CH-;

----- $Y^4$ is a bond, O, S, or -NH-;

----- $Y^5$ is a member independently selected from the group consisting essentially of O; $NR^{222}$; $NOR^{222}$; NCN; $C(CN)_2$; $CR^{222}NO_2$; $CR^{222}C(=O)OR^{222}$; $CR^{222}C(=O)NR^{222}R^{223}$; $C(CN)NO_2$; $C(CN)C(=O)OR^{222}$; and $C(CN)C(=O)NR^{222}R^{223}$; and

----- $Z^3$ is a member independently selected from the group consisting essentially of $-NR^{222}-$; $-(CH_2)_m-$; $-CH_2C(=O)NH-$; $-NHCH_2C(=O)-$; $-CH_2C(Y^1)CH_2-$; -CH=CH-; -C≡C-, $-CH(Y^1H)-$; $-C(Y^1)-$; $-CH_2C(Y^1)-$; $-C(Y^1)CH_2-$; $-C(Y_1)C(Y_1)-$; $-CH_2NR^{222}-$; $-CH_2-Y^1-$; $-C(Y^1)NR^{218}(CHR^{222})_n-$; $-NR^{218}C(Y^1)(CHR^{222})_n-$; $-NHCH_2-$; $-Y^1-CH_2-$; $-SOCH_2-$; $-CH_2SO-$; $-SO_2CH_2-$; $-CH_2SO_2-$; $-OC(Y^1)-$; -N=N-; $-NHSO_2-$; $-SO_2NH-$; $-C(Y^1)C(Y^1)NH-$; -NHC(=O)O-; -OC(=O)NH-; and -NHC(=O)NH-; wherein for said $Z_3$ moieties n is an integer selected from 0, 1, 2, 3, and 4; and m is an integer selected from 1, 2, and 3;

- or said substituents defining $R^2_a$ and $R^2_b$ comprise: -

-- ( - III - )

-- a member independently selected from the group consisting essentially of 2-oxo-4-pyrrolyl; pyrazolyl; 2-oxo-3,4-dihydro-5-pyrimidyl; 2-oxo-3,4-dihydro-4-pyrimidyl; 2-oxo-tetrahydro-4-pyrimidyl; 2-oxo-tetrahyro-5-pyrimidyl; 2-oxo-4-pyrimidyl; and 2-oxo-5-pyrimidyl; wherein each of said $R^2_a$ and $R^2_b$ groups is substituted by 0, 1, 2, 3, or 4 $R^{236}$ groups;

- or, said substituents defining $R^2_a$ and $R^2_b$ comprise a moiety of partial Formulas (3.0.0) through (3.0.19), inclusive: -

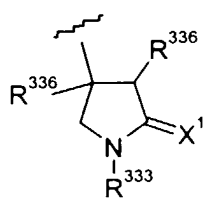
(3.0.0)

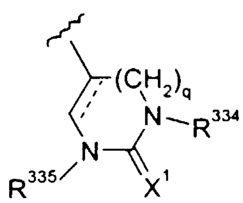
(3.0.1)

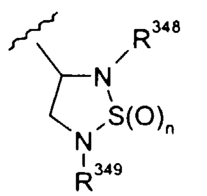
(3.0.2)

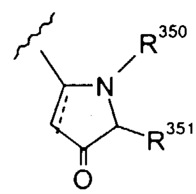
(3.0.3)

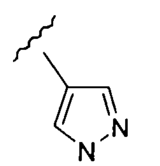
(3.0.4)

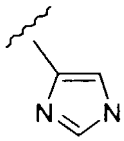
(3.0.5)

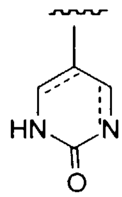
(3.0.6)

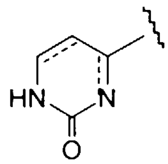
(3.0.7)

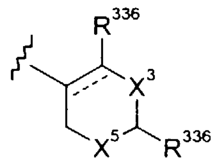
(3.0.8)

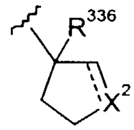
(3.0.9)

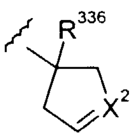
(3.10 )

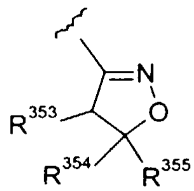
(3.11)

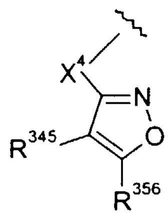
(3.12)

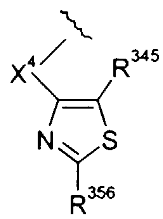
(3.13)

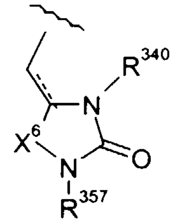
(3.14)

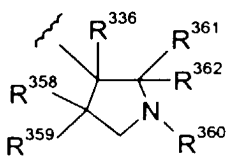
(3.15)

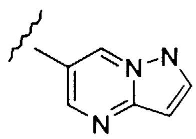
(3.16)

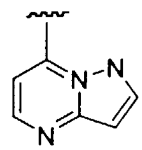
(3.17)

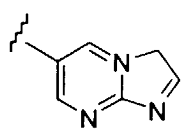
(3.18)

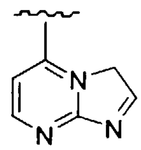
(3.19)

---- wherein in said partial Formulas (3.0.0) through (3.0.19), q is an integer selected from 0 and 1 in partial Formula (3.0.1); n is an integer selected from 0, 1, and 2 in partial Formula (3.0.2); and the dashed lines appearing in formulas (3.0.1), (3.0.3), (3.0.6), (3.0.7), (3.0.8), (3.0.9) and (3.0.14) represent a double bond or a single bond;

----- $X^1$ is O or S;

----- $X^2$ in formula (3.0.10) and where the dashed line in formula (3.0.9) represents a double bond, is a member independently selected from the group consisting essentially of $CR^{335}$; $CR^{336}$; $CR^{346}$; and COC(=O) $NR^{339}R^{342}$; or, where the dashed line in formula (3.0.9) represents a single bond, $X^2$ is a member independently selected from the group consisting essentially of $CR^{335}R^{339}$; $CR^{336}R^{339}$; and $CR^{346}R^{339}$;

----- $X^3$ is a member independently selected from the group consisting essentially of $C(=Z^3)$; C(S); and $CR^{336}R^{340}$;

----- $X^4$ is a member independently selected from the group consisting essentially of $-(CH_2)_{m}-$ where m is an integer selected from 0,1, and 2;

----- $X^5$ is a bond or $-CH_2-$;

----- $X^6$ is a member independently selected from the group consisting essentially of $-CH_2-$and -C(=O)-;

----- $R^{333}$ is a member independently selected from the group consisting essentially of H; hydroxy; $(C_1-C_4)$ alkoxy; $-CHR^{337}(O)_q(CH_2)_mA$ where q is an integer selected from 0 and 1, and m is an integer selected from 0, 1, and 2;

----- $R^{334}$ is a member independently selected from the group consisting essentially of H; hydroxy; $(C_1-C_4)$ alkyl;

$(C_1-C_2)$ alkoxy; $-OC(=O)CH_3$; $(C_2-C_3)$ alkenyl; and phenyl$(C_1-C_2)$ alkyl-;

----- $R^{335}$ is a member independently selected from the group consisting essentially of H; hydroxy; $-(CH_2)_mA$ where m is an integer selected from 0, 1, and 2; $(C_1-C_6)$alkyl; and $(C_2-C_3)$ alkanoyl; where said alkyl group is substituted by 0 to 3 subtituents independently selected from the group consisting essentially of bromo, chloro, or fluoro; nitro; $-NR^{340}R^{341}$; $-CO_2R^{340}$; $-OR^{340}$; $-OC(=O)R^{340}$; $-C(=O)R^{340}$; cyano; $-C(=Y)NR^{340}R^{341}$; $-NR^{340}(=Y)NR^{340}R^{341}$, $-NR^{340}C(=Y)R^{340}$; $-NR^{340}C(=O)OR^{340}$; $-C(NR^{340})NR^{340}R^{341}$; $-C(NCN)NR^{340}R^{341}$; $-C(NCN)SR^{340}$; $-NR^{340}SO_2R^{340}$; $-S(O)_mR^{340}$, where m is an integer selected from 0, 1, and 2; $-NR^{340}SO_2CF_3$; $-NR^{340}C(=O)C(=O)NR^{340}R^{341}$; $-NR^{340}C(=O)C(=O)OR^{340}$; imidazolyl; and 1-$(NHR^{340})$-2-imidazolyl;

----- $R^{336}$ is each a member independently selected from the group consisting essentially of H; bromo, chloro, or fluoro; cyano; $R^{343}$; cyclopropyl substituted by 0 or 1 substituent independently selected from the group consisting essentially of $R^{339}$; $-OR^{340}$; $-CH_2OR^{340}$; $-NR^{340}R^{342}$; $-CH_2NR^{340}R^{342}$; $-C(=O)OR^{340}$; $-C(=O)NR^{340}R^{342}$; $-CH=CR^{339}R^{339}$; $-C=CR^{339}$; and $-C(=Z^3)H$;

----- $R^{337}$ is a member independently selected from the group consisting essentially of H; $-C(=O)R^{338}$; imidazolyl; pyrazolyl; triazolyl; tetrazolyl; oxazolyl; isoxazolyl; oxadiazolyl; thiadiazolyl; thiazolyl; oxazolidinyl; thiazolidinyl; and imidazolidinyl;

------$R^{338}$ is each a member independently selected from the group consisting essentially of $--OR^{340}$; $-NR^{340}R^{342}$; and $-R^{343}$;

------$R^{339}$ is each a member independently selected from the group consisting essentially of H; bromo, chloro, or fluoro; and $(C_1-C_4)$ alkyl substituted by 0 to 3 fluorine atoms;

------$R^{340}$ and $R^{341}$ are each a member independently selected from the group consisting essentially of hydrogen and $(C_1-C_4)$ alkyl;

------$R^{342}$ is each a member independently selected from the group consisting essentially of $-OR^{340}$ and $-R^{340}$;

------$R^{343}$ is $(C_1-C_4)$ alkyl;

------$R^{344}$ is each a member independently selected from the group consisting essentially of bromo, chloro, or fluoro; nitro; cyano; $-NR^{340}R^{346}$; $-NR^{346}R^{342}$; $-C(=Z^3)R^{338}$; $-S(O)_mR^{343}$ where m is an integer selected from 0, 1, and 2; $-OR^{342}$; $-OC(=O)NR^{340}R^{342}$; $-C(NR^{342})NR^{340}R^{342}$; $-C(NR^{340})SR^{343}$; $-OC(=O)CH_3$; $-C(NCN)NR^{340}R^{342}$; $-C(S)NR^{340}R^{342}$; $-NR^{342}C(=O)R^{347}$; $-C(=O)R^{347}$; oxazolyl; imidazolyl; thiazolyl; pyrazolyl; triazolyl; and tetrazolyl;

------$R^{345}$ is each a member independently selected from the group consisting essentially of hydrogen and $(C_1-C_4)$ alkyl substituted by01 to 3 fluorine atoms;

------$R^{346}$ is each a member independently selected from the group consisting essentially of H; $-R^{343}$; $-C(=O)R^{343}$; $-C(=O)C(=O)R^{338}$; $-C(=O)NR^{340}R^{342}$; $-S(O)_{rn}R^{343}$ where m is an integer selected from 0, 1, and 2; $-C(NCN)SR^{343}$; $-C(NCN)R^{343}$; $-C(NR^{342})R^{343}$; $-C(NR^{342})SR^{343}$; and $-C(NCN)NR^{340}R^{342}$;

------$R^{347}$ is each a member independently selected from the group consisting essentially of $-R^{343}$; $-C(=O)R^{343}$; oxazolidinyl; oxazolyl; thiazolyl; pyrazolyl; triazolyl; tetrazolyl; imidazolyl; imidazolidinyl; thiazolidinyl; isoxazolyl; oxadiazolyl; thiadiazolyl; morpholinyl; piperidinyl; piperazinyl; and pyrrolyl; where each of said recited R347 heterocyclic groups is substituted by 0 to 2 $(C_1-C_2)$ alkyl groups;

------$R^{348}$ is each a member independently selected from the group consisting essentially of H; $(C_1-C_5)$ alkyl; $(C_2-C_5)$ alkenyl; benzyl; and phenethyl;

------$R^{349}$ is a member independently selected from the group consisting essentially of H; $(C_1-C_5)$ alkyl; $(C_1-C_5)$ alkanoyl; and benzoyl;

------$R^{350}$ is a member independently selected from the group consisting essentially of H; $(C_1-C_4)$alkyl; carboxy; aminocarbonyl; $(C_1-C_6)$alkyl substituted by 0 or 1 carboxy, $-(CH_2)_mC(=O)(C_1-C_6)$ alkoxy; or $-(CH_2)_m(C_6-C_{10})$

aryl; where m is an integer selected from 0, 1, and 2;

------$R^{351}$ is a member independently selected from the group consisting essentially of H; ($C_1$-$C_6$) alkyl; -C(=Y) $R^{352}$; -C(=Y)NH35; -C(=O)O$R^{352}$; and -$(CH_2)_n$$X^7$(pyridyl) where n is an integer selected from 0, 1, 2, 3, 4, and to 5; and $X^7$ is a bond or -CH=CH-; and where said pyridyl moiety is substituted by 0 or 1 bromo, chloro, or fluoro;

------$R^{352}$ is a member independently selected from the group consisting essentially of ($C_1$-$C_6$) alkyl ($C_3$-$C_8$) cycloalkyl; -$(CH_2)_m$($C_6$-$C_{10}$) aryl; and -$(CH_2)_n$$X^7$(pyridyl) where n is an integer selected from 0, 1, 2, 3, 4, and 5; and $X^7$ is a bond or -CH=CH-; and where said pyridyl moiety is substituted by 0 or 1 bromo, chloro, or fluoro;

------$R^{353}$ is a member independently selected from the group consisting essentially of H; - $R^{345}$; ($C_1$-$C_3$) alkyl substituted by 0 or 1 substituent hydroxy, or ($C_1$-$C_3$) alkyoxy($C_1$-$C_3$) alkyl;

------$R^{354}$ is a member independently selected from the group consisting essentially of H; -$R^{345}$; carboxy; ($C_1$-$C_3$) alkyoxy($C_1$-$C_3$) alkyl-; ($C_3$-$C_7$) cycloalkyl; and ($C_1$-$C_5$) alkyl substituted by 0 or 1 -N$R^{340}$$R^{341}$ ; or

------$R^{353}$ and $R^{354}$ are taken together to form -$CH_2OCH_2OCH_2$-;

------$R^{355}$ is a member independently selected from the group consisting essentially of H; hydroxy; ($C_1$-$C_4$)alkyl substituted by 0 or 1 substituent comprising a member independently selected from the group consisting essentially of hydroxy; -C(=O)$R^{340}$; -N$R^{340}$$R^{341}$; -$(CH_2)_m$NHC(=O)$R^{340}$; -$(CH_2)_m$NHC(=O)$R^{343}$; -$(CH_2)_m$$CO_2$$R^{340}$; -$(CH_2)_m$C(=O)N$R^{340}$$R^{341}$; -$(CH_2)_m$C(=O)N(OH)$R^{340}$, -$(CH_2)_m$$SO_2$N$R^{340}$$R^{341}$; -$(CH_2)_m$$PO_3H_2$; -$(CH_2)_m$$SO_2$NHC(=O)$R^{343}$; and -$(CH_2)_m$$SO_2$NHC(=O)(phenyl), where m is an integer selected from 0, 1, 2, 3, and 4;

------$R^{356}$ is a member independently selected from the group consisting essentially of H; ($C_1$-$C_4$) alkyl; phenyl; -N$R^{340}$$R^{341}$; and -N$R^{340}$($C_1$-$C_4$) alkanoyl;

------$R^{357}$ is a member independently selected from the group consisting essentially of -$R^{340}$; -$CH_2CO_2$$R^{343}$; and -$CH_2$C(=O)N$R^{340}$$R^{341}$;

------$R^{358}$ is a member independently selected from the group consisting essentially of -C(=O)$R^{340}$; -C(=O)($C_6$-$C_{10}$) aryl; -C(=O)($C_3$-$C_9$) heteroaryl; -$CO_2$$R^{340}$; -C(=O)N$R^{340}$$R^{341}$; cyano; nitro; -$CH_2$OH; -N$R^{340}$$SO_2$$R^{340}$; -NH$SO_2$($C_6$-$C_{10}$) aryl; -NH$CO_2$($C_1$-$C_4$) alkyl; -N$R^{340}$C(=O)$R^{340}$; and -NH$CO_2$($C_6$-$C_{10}$) aryl;

------$R^{359}$ is a member independently selected from the group consisting essentially of -$R^{345}$; cyano; carboxy; formyl; -C(=O)$R^{340}$; and ($C_1$-$C_4$) alkanoyl;

------$R^{360}$ is a member independently selected from the group consisting essentially of cyano; -N$R^{340}$$R^{341}$; -$SO_2$ ($C_1$-$C_4$) alkyl; -$SO_2$($C_6$-$C_{10}$) aryl; -C(=O)$R^{340}$; -C(=O)($C_6$-$C_{10}$) aryl; -C(=O)($C_3$-$C_9$) heteroaryl; -C(=O) N$R^{340}$$R^{341}$; and -$CO_2$$R^{340}$;

------$R^{361}$ and $R^{362}$ are each a member independently selected from the group consisting essentially of H; cyano; nitro; -$CO_2$$R^{340}$; -C(=O)N$R^{340}$$R^{341}$; -$CH_2$OH; -C(=O)$R^{340}$; -NH$CO_2$$R^{340}$; and -NH$SO_2$$R^{340}$;

------A is a member independently selected from the group consisting essentially of pyridyl; morpholinyl; piperidinyl; imidazolyl; thienyl; pyrimidyl; thiazolyl; phenyl; and naphthyl; where each of said A groups is substituted by 0 to 2 substituents $R^{344}$ or by 1 substituent $R^{345}$;

------$Z^3$ is a member independently selected from the group consisting essentially of O; -N$R^{342}$; NO$R^{340}$; N(CN); $C(CN)_2$; C$R^{340}$$NO_2$; C$R^{340}$C(=O)O$R^{343}$; C$R^{340}$C(=O)N$R^{340}$$R^{341}$; C(CN)$NO_2$; C(CN)C(=O)O$R^{343}$; and C(CN) C(=O)N$R^{340}$$R^{341}$; and,

------Y is O or S;

- or said substituents defining $R^2_a$ and $R^2_b$ comprise a moiety of partial Formula (4.0.0): -

-- ( - IV - )

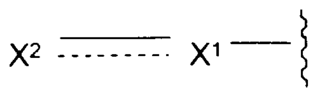

(4.0.0)

--- wherein the broken line indicates a single or double bond;

--- $X^1$ is $-CR^{472}R^{473}-$ where said broken line indicates a single bond; or $-CR^{473}-$ where said broken line indicates a double bond;

--- $X^2$ is $-CR^{475}R^{477}R^{478}-$ or $-C(=NOR^{481})R^{482}-$ where said broken line indicates a single bond; or $-CR^{477}R^{478}$ where said broken line indicates a double bond;

---- $R^{472}$ is a member independently selected from the group consisting essentially of H; hydroxy; bromo, chloro, or fluoro; and $-OR^{479}$;

---- $R^{473}$ is each a member independently selected from the group consisting essentially of cyano; cyanomethyl; benzyloxy; $-R^{475}$; $-CO_2R^{475}$; $-CO_2(CH_2)_n(C_6-C_{10})$ aryl; $-C(Y)NR^{475}R^{476}$; $-C(Y)NR^{475}(CH_2)_n(C_6-C_{10})$ aryl; $-(CH_2)_n(C_6-C_{10})$ aryl; and $(CH_2)_n$(5 to 10-membered heteroaryl); where n is an integer selected from 0, 1, 2, and 3; each $R^{473}$ group is substituted by 0 to 3 substituents $R^{474}$ ; and each $R^{473}$ group is substituted by 0 or 1 substituent $R^{480}$;

---- $-R^{474}$ is each a member independently selected from the group consisting essentially of bromo, chloro, or fluoro; cyano; nitro; $(C_1-C_6)$alkyl; $(C_2-C_6)$alkenyl; $-OR^{475}$; $(C_3-C_7)$ cycloalkoxy; $-NR^{475}R^{476}$; $-NR^{475}OR^{476}$; $-S(O)_mR^{475}$ where m is an integer selected from 0, 1, and 2; $-CO_2R^{475}$, $-C(=O)R^{475}$; $-SO_2NR^{475}R^{476}$; $-C(=O)NR^{475}R^{476}$; $-CR^{475}R^{476}SO_2NR^{475}R^{476}$; $-CR^{475}R^{476}C(=O)NR^{475}R^{476}$; $-NHSO_2R^{475}$; $-NHSO_2NR^{475}R^{476}$; $-NHC(=O)NR^{475}R^{476}$; $-NHC(=O)(C_1-C_6)$ alkyl; and $-NHC(=O)O(C_1-C_6)$ alkyl);

---- $R^{475}$ and $R^{476}$ are each a member independently selected from the group consisting essentially of H; and $(C_1-C_6)$ alkyl;

---- $R^{477}$ is a member independently selected from the group consisting essentially of $-R^{473}$; 2-oxo-pyridyl; 3-oxo-pyridyl; 4-oxo-pyridyl; 2-oxo-pyrrolyl; 4-oxo-thiazolyl; 4-oxo-piperidyl; 2-oxo-quinolyl; 4-oxo-quinolyl; 1-oxo-isoquinolyl; 4-oxo-oxazolyl; 5-oxo-pyrazolyl; 5-oxo-isoxazolyl; and 4-oxo-isoxazolyl; where each of said $R^{477}$ groups is substituted by 0 to 3 substituents $R^{474}$ ;

---- $R^{478}$ is a member independently selected from the group consisting essentially of $-R^{475}$; cyano; $-(CH_2)_p(C_6-C_{10})$ aryl; and $-(CH_2)_p$(5- to 10-membered heteroaryl); where p is an integer selected from 1, 2, and 3; and where each said $R^{478}$ group is substituted by 0 to 3 substituents $R^{474}$;

----- $R^{479}$ is a member independently selected from the group consisting essentially of formyl; carbamoyl; thiocarbamyl; $(C_1-C_6)$ alkyl; $(C_2-C_6)$ alkenyl; $(C_1-C_4)$ alkoxy$(C_1-C_4)$ alkyl-; and $(C_1-C_6)$ alkanoyl; where said alkyl moieties of each of said $R^{479}$ groups is substituted by 0 to 3 substituents independently selected from the group consisting essentially of bromo, chloro, or fluoro; hydroxy; and $(C_1-C_4)$ alkoxy;

----- $R^{480}$ is a member independently selected from the group consisting essentially of cyclobutyl; cyclopentyl; cyclohexyl; 2-cyclobuten-1-yl; 2-cyclopenten-1-yl; 3-cyclopenten-1-yl; 2,4-cyclopentadien-1-yl; 3,5-cyclohexadien-1-yl; pyrrolyl; pyrrolidinyl; dioxolanyl; imidazolyl; oxazolyl; imidazolidinyl; pyrazolyl; pyrazolidinyl; pyranyl; piperidinyl; 1,4-dioxanyl; morpholinyl; 1,4-dithianyl; thiomorpholinyl; piperazinyl; 1,3,5-trithianyl; oxazinyl; isoxazinyl; oxathiazinyl; and oxadiazinyl; where each of said $R^{480}$ groups is substituted by 0 to 2 $(C_1-C_2)$ alkyl;

---- $R^{481}$ is a member independently selected from the group consisting essentially of H; $(C_1-C_6)$ alkyl; $(C_2-C_6)$ alkenyl; $(C_2-C_6)$ alkynyl; $-C(Y)NR^{475}R^{476}$; $-C(Y)NH(C_6-C_{10})$ aryl; $-C(Y)(C_1-C_6)$ alkoxy; $-C(Y)(C_6-C_{10})$ aryloxy; and $-C(Y)(C_1-C_6)$ alkyl);

---- $R^{482}$ is a member independently selected from the group consisting essentially of phenyl and pyridinyl; where each

of said $R^{482}$ groups is substituted by 0 to 3 substituents independently selected from the group consisting essentially of bromo, chloro, or fluoro; $(C_1\text{-}C_4)$alkyl; hydroxy; $(C_1\text{-}C_4)$alkoxy; $-NR^{475}R^{476}$; and $-S(O)_mR^{475}$, where m is an integer selected from 0, 1, and 2; and,

**-----** Y is O or S;

- or, said substituents defining $R^2_a$ and $R^2_b$ comprise a moiety of partial Formulas (5.0.0) through (5.0.13), inclusive: -

**--** ( - V - )

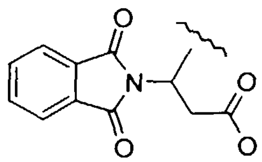

(5.0.0)

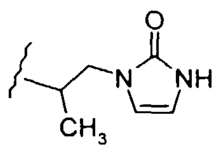

(5.0.1)

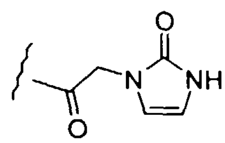

(5.0.2)

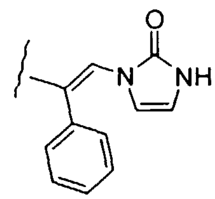

(5.0.3)

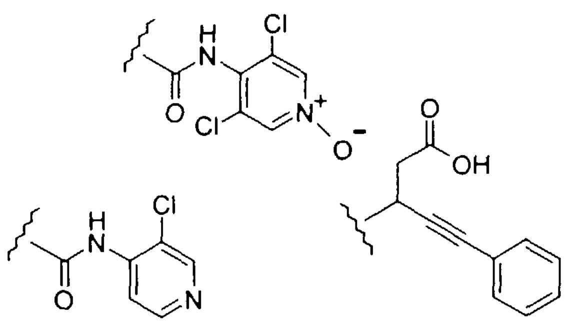

(5.0.4) (5.0.5) (5.0.6)

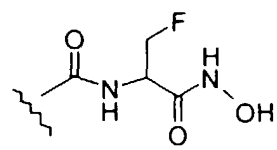

(5.0.7)

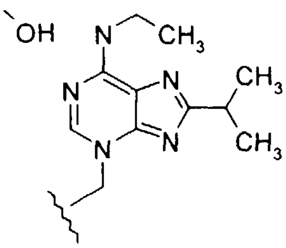

(5.0.8)

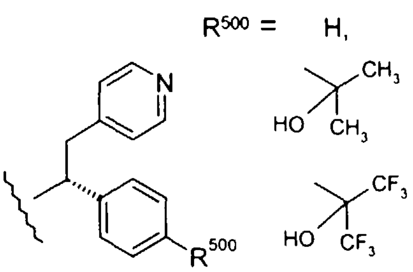

(5.0.9)

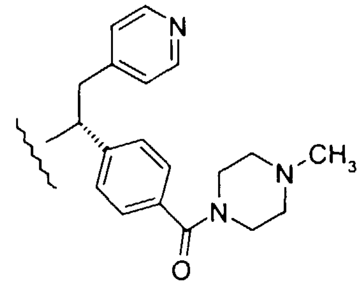

(5.0.10)

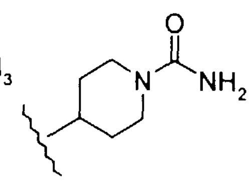

(5.0.11)

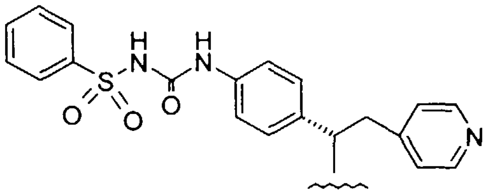

(5.0.12)

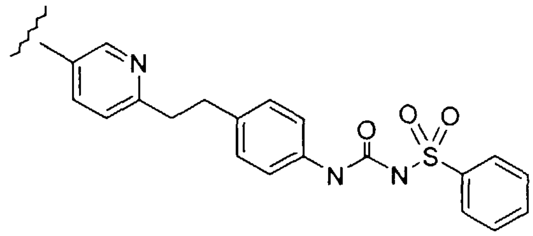

(5.0.13)

Preferred compounds of Formula (IA) or (IB) useful as therapeutic agents in the methods of treatment and pharmaceutical compositions of the present invention include those where $R^2_a$ and $R^2_b$ are as defined under ( - IV - ) above, and further include those wherein $R^1$ is ethyl and R is cyclopentyl, cyclohexyl, or ($C_6$-$C_{10}$) aryl.

Other preferred compounds of Formula (IA) or (IB) useful as therapeutic agents in the methods of treatment and pharmaceutical compositions of the present invention include those wherein $R^{473}$ is $-(CH_2)_n(C_6\text{-}C_{10})$ aryl or $-(CH_2)_n$ (5- to 10-membered heteroaryl), where n is an integer selected from 0, 1, 2, and 3; and, more preferably, wherein $R^{473}$ is phenyl or pyridin-4-yl.

Specific embodiments of the compounds of Formula (IA) or (IB) useful as therapeutic agents in the methods of treatment and pharmaceutical compositions of the present invention include those where $R^2_a$ and $R^2_b$ are as defined under ( - I - ), and further include those wherein R is cyclopentyl or cyclohexyl, $R^1$ is ($C_1$-$C_2$) alkyl, preferably ethyl, one of $R^2_a$ and $R^2_b$ is hydrogen and the other is a substituent of partial Formula (1.0.0) where the dashed line represents a single bond, m is 0, $R^{113}$ and $R^{114}$ are in a *cis* relationship to each other, $R^{113}$ is cyano, $R^{115}$ is hydrogen, and $R^{114}$ is carboxy, $-CH_2OH$, or $-CH_2C(=O)NH_2$.

Other specific embodiments of the compounds of Formula (IA) or (IB) useful as therapeutic agents in the methods of treatment and pharmaceutical compositions of the present invention include those wherein R is phenyl substituted by fluoro, $R^1$ is ($C_1$-$C_2$) alkyl, preferably ethyl, one of $R^2_a$ and $R^2_b$ is hydrogen and the other is a substituent of partial Formula (1.0.0) where the dashed line represents a single bond, $R^{113}$ is cyano, and $R^{115}$ and $R^{114}$ are both hydrogen.

The term "halo", as used herein, unless otherwise indicated, means fluoro, chloro, bromo or iodo. Preferred halo groups are fluoro, chloro and bromo.

The term "alkyl", as used herein, unless otherwise indicated, means saturated monovalent hydrocarbon radicals which are straight or branched moieties containing from one to six, preferably one to four, carbon atoms.

The term "alkoxy", as used herein, unless otherwise indicated, means O-alkyl groups wherein "alkyl" is defined above.

The term "alkenyl", as used herein, unless otherwise indicated, means unsaturated alkyl groups having one or more double bonds wherein "alkyl" is defined above.

The term "cycloalkyl", as used herein, unless otherwise indicated, means saturated monovalent cyclo hydrocarbon radicals containing from three to seven carbon atoms, preferably five or six carbon atoms, including such specific radicals as cyclobutyl, cyclopentyl and cycloheptyl.

The term "aryl", as used herein, unless otherwise indicated, means an organic radical derived from an aromatic hydrocarbon by removal of one hydrogen, comprising a carbocyclic moiety which is a member independently selected from the group consisting essentially of benzyl; *cis*- and *trans*-decahydronaphthalenyl; 2,3-1H-dihydroindenyl (indanyl); indenyl; 1-naphthalenyl; 2-naphthalenyl; phenyl; and 1,2,3,4-tetrahydronaphthalenyl; and preferably means phenyl.

The term "heterocyclyl" or "heterocyclic", as used herein, unless otherwise indicated, means aromatic and non-aromatic heterocyclic groups containing one or more heteroatoms each selected from O, S and N. Included within this meaning are heterocyclic groups which are benzo-fused ring systems and ring systems substituted with an oxo moiety. Included within the scope of this definition are the following specific groups: acridinyl; benzimidazolyl; benzodioxolane; 1,3-benzodioxol-5-yl; benzo[b]furanyl; benzo[b]thiophenyl; benzoxazolyl; benzthiazolyl; carbazolyl; cinnolinyl; 2,3-dihydrobenzofuranyl; 1,3-dioxane; 1,3-dioxolane; 1,3-dithiane; 1,3-dithiolane; furanyl; imidazolidinyl; imidazolinyl; imidazolyl; 1H-indazolyl; indolinyl; indolyl; 3H-indolyl; isoindolyl; isoquinolinyl; isothiazolyl; isoxazolyl; morpholinyl; 1,8-naphthyridinyl; oxadiazolyl; 1,3-oxathiolane; oxazolidinyl; oxazolyl; oxiranyl; parathiazinyl; phenazinyl; phenothiazinyl; phenoxazinyl; phthalazinyl; piperazinyl; piperidinyl; pteridinyl; pyranyl; pyrazinyl; pyrazolidinyl; pyrazolinyl; pyrazolo[1,5-c]triazinyl; pyrazolyl; pyridazinyl; pyridyl; pyrimidinyl; pyrimidyl; pyrrolyl; pyrrolidinyl; purinyl; quinazolinyl; quinolinyl; 4H-quinolizinyl; quinoxalinyl; tetrazolidinyl; tetrazolyl; thiadiazolyl; thiazolidinyl; thiazolyl; thienyl; thiomorpholinyl; triazinyl; and triazolyl.

With reference to the $R^{114}$ substituent of partial Formula (1.0.0) of Formula (IA) or (IB), the ($C_3$-$C_9$)heterocydic

group can be attached to the ($C_1$-$C_6$)alkyl group by a nitrogen or, preferably, a carbon atom. An example of a $C_3$ heterocyclic group is thiazolyl, and an example of a $C_9$ heterocyclic group is quinolinyl. Examples of non-aromatic heterocyclic groups are pyrrolidinyl, piperidino, morpholino, thiomorpholino and piperazinyl. Examples of aromatic heterocyclic groups which are preferred are pyridinyl, imidazolyl, pyrimidinyl, pyrazolyl, triazolyl, pyrazinyl, tetrazolyl, furyl, thienyl, isoxazolyl and thiazolyl. A preferred heterocyclic group having a fused benzene ring is benzimidazolyl.

Where heterocyclic groups are specifically recited or covered as substituents for the compound of Formula (IA) or (IB) under ( - I - ), it is understood that all suitable isomers of such heterocyclic groups are intended. Thus, for example, in the definition of the substituent $R^{114}$, the term "thiazolyl" includes 2-, 4- or 5-thiazolyl; the term "imidazolyl" includes 2-, 4- or 5-imidazolyl; the term "pyrazolyl" includes 3-, 4- or 5-pyrazolyl; the term "oxazolyl" includes 2-, 4- or 5-oxazolyl; the term "isoxazolyl" includes 3-, 4- or 5-isoxazolyl, and so on. Likewise, in the definition of substituent $R^{116}$, the term "pyridyl" includes 2-, 3- or 4-pyridyl.

Certain "aminal" or "acetal"-like chemical structures within the scope of Formula (IA) or (IB) may be unstable. Such structures may occur where two heteroatoms are attached to the same carbon atom. For example, where R is ($C_1$-$C_6$) alkyl substituted by hydroxy, it is possible that the hydroxy may be attached to the same carbon that is attached to the nitrogen atom from which R extends. It is to be understood that such unstable compounds are not within the scope of the present invention.

Preferred compounds of Formula (IA) or (IB) under ( - I - ) useful as therapeutic agents in the methods of treatment and pharmaceutical compositions of the present invention include those wherein $R^2_a$ or $R^2_b$ is a group of the following partial Formula (1.1.0) or (1.2.0):

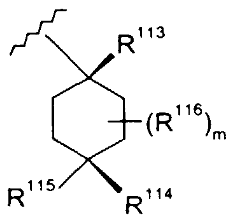

(1.1.0)

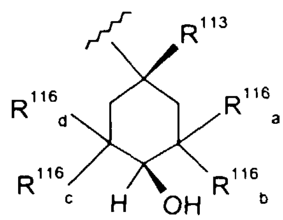

(1.2.0)

where for partial Formula (1.1.0) $R^{113}$ and $R^{114}$, especially where $R^{114}$ is -OH, are *cis* with respect to each other; and for partial Formula (1.2.0) $R^{116}_a$, $R^{116}_b$, $R^{116}_c$, and $R^{116}_d$ are independently selected from the group consisting essentially of -H; $-CH_3$; $-CF_3$; $-CHF_2$; $-CH_2F$; ethyl, i-propyl; and *t*-butyl;

Other preferred compounds of Formula (IA) or (IB) under ( - I - ) ) useful as therapeutic agents in the methods of treatment and pharmaceutical compositions of the present invention include those wherein R is a member independently selected from the group consisting essentially of cyclohexyl, cyclopentyl, cyclobutyl, methylenecyclopropyl, isopropyl, phenyl, and 4-fluoro-phenyl.

Other preferred compounds of Formula (IA) or (IB) under ( - I - ) useful as therapeutic agents in the methods of treatment and pharmaceutical compositions of the present invention include those wherein $R^1$ is ($C_1$-$C_2$)alkyl substituted by 0 to 3 fluorine atoms, and, more preferably, those wherein $R^1$ is ethyl.

Other preferred compounds of Formula (IA) or (IB) under ( - I - ) include those wherein one of $R^2_a$ and $R^2_b$ is hydrogen and the other is a group of partial Formula (1.0.0) wherein the dashed line attached to the ring carbon atom to which $R^{113}$ is attached represents a single bond.

Other preferred compounds of Formula (IA) or (IB) under ( - I - ) include those wherein one of $R^2_a$ and $R^2_b$ is hydrogen and the other is a group of partial Formula (1.0.0) wherein the dashed line attached to the ring carbon atom to which $R^{113}$ is attached represents a single bond and $R^{113}$ is cyano.

Other preferred compounds of Formula (IA) or (IB) under ( - I - ) useful as therapeutic agents in the methods of treatment and pharmaceutical compositions of the present invention include those wherein one of $R^2_a$ and $R^2_b$ is hydrogen and the other is a group of partial Formula (1.0.0) wherein the dashed line attached to the ring carbon atom to which $R^{113}$ is attached represents a single bond, m is 0 and $R^{115}$ is hydrogen.

Other preferred compounds of Formula (IA) or (IB) under ( - I - ) include those wherein one of $R^2_a$ and $R^2_b$ is hydrogen and the other is a group of partial Formula (1.0.0) wherein the dashed line attached to the ring carbon atom to which $R^{113}$ is attached represents a single bond; m is 0; $R^{115}$ is hydrogen; and $R^{114}$ is a member independently selected from the group consisting essentially of -OH; $-CH_2OH$; $-C(CH_3)_2OH$; -C(=O)OH; $-C(=O)OCH_3$; -C(=O) $OCH_2CH_3$; and $-CH_2C(=O)NH_2$.

Other more preferred compounds of Formula (IA) or (IB) under ( - I - ) useful as therapeutic agents in the

methods of treatment and pharmaceutical compositions of the present invention include those wherein R is a member independently selected from the group consisting essentially of cyclobutyl, cyclopentyl, cyclohexyl, and 4-fluoro-phenyl; $R^1$ is ethyl; one of $R^2_a$ and $R^2_b$ is hydrogen and the other is a group of partial Formula (1.2.0); $R^{113}$ is cyano; $R^{115}$ is hydrogen; $R^{114}$ is -OH, and $R^{113}$ and $R^{114}$ are *cis* with respect to each other; and $R^{116}_a$, $R^{116}_b$, $R^{116}_c$, and $R^{116}_d$ are each a member independently selected from the group consisting essentially of -H; and $-CH_3$;

Preferred compounds of Formula (IA) or (IB) include those wherein $R^1$ is ethyl.

Other preferred compounds of Formula (IA) or (IB) include those wherein R is a member independently selected from the group consisting essentially of cyclohexyl; cyclopentyl; methylenecyclopropyl; isopropyl; phenyl; and 4-fluoro-phenyl.

Specific preferred compounds of Formula (IA) or (IB) under ( - I - ) useful as therapeutic agents in the methods of treatment and pharmaceutical compositions of the present invention include:

1 -(1 -Cyclopentyl-3-ethyl-1H-indazol-6-yl)-4-oxocydohexanecarbonitrile;

*Trans*-4-cyano-4-(1-cyclopentyl-3-ethyl-1H-indazol-6-yl)-cyclohexanecarboxylic acid methyl ester;

*Cis*-4-cyano-4-(1-cydopentyl-3-ethyl-1H-indazol-6-yl)cyclohexanecarboxylic acid methyl ester;

*Trans*-4-cyano-4-(1-cyclopentyl-3-ethyl-1H-indazol-6-yl)cyclohexanecarboxylic acid;

*Cis*-4-cyano-4-(1-cyclopentyl-3-ethyl-1H-indazol-6-yl)cyclohexanecarboxylic acid;

1-(1-Cyclohexyl-3-ethyl-1H-indazol-6-yl)-4-oxocyclohexanecarbonitrile;

*Cis*-4-cyano-4-(1-cyclohexyl-3-ethyl-1 H-indazol-6-yl)cyclohexanecarboxylic acid methyl ester;

*Trans*-4-cyano-4-(1-cyclohexyl-3-ethyl-1H-indazol-6-yl)cyclohexanecarboxylic acid methyl ester;

*Cis*-4-cyano-4-(1-cyclohexyl-3-ethyl-1H-indazol-6-yl)cyclohexanecarboxylic acid;

*Trans*-4-cyano-4-(1-cyclohexyl-3-ethyl-1H-indazol-6-yl)cyclohexanecarboxylic acid;

*Cis*-1-(1-cyclohexyl-3-ethyl-1H-indazole-6-yl)-4-hydroxymethylcyclohexanecarbonitrile;

*Cis*-4-cyano-4-(1-cyclohexyl-3-ethyl-1H-indazol-6-yl)-cyclohexanecarboxylic acid amide;

*Trans*-4-cyano-4-(1-cyclohexyl-3-ethyl-1H-indazol-6-yl)cyclohexanecarboxylic acid amide;

*Cis*-1-(1-cyclohexyl-3-ethyl-1H-indazol-6-yl)4-(1-hydroxy-1-methylethyl)cyclohexanecarbonitrile;

*Cis*-1-(1-cyclohexyl-3-ethyl-1H-indazol-6-yl)-4-hydroxycyclohexanecarbonitrile;

*Cis*-1-[3-ethyl-1-(4-fluorophenyl)-1H-indazol-6-yl]-4-hydroxycyclohexanecarbonitrile;

*Cis*-1-(1-cyclopentyl-3-ethyl-1H-indazol-6-yl)-4-hydroxycyclohexanecarbonitrile;

*Cis*-1-(1-cyclobutyl-3-ethyl-1H-indazol-6-yl)-4-hydroxycydohexanecarbonitrile;

*Cis*-1-(-cyclopentyl-3-ethyl-1H-indazol-6-yl)-4-hydroxy-4-methylcyclohexanecarbonitrile;

*Trans*-1-(1-cyclopentyl-3-ethyl-H-indazol-6-yl)-4-hydroxy-4-methylcyclohexanecarbonitrile;

*Cis*-4-cyano-4-(1-cyclobutyl-3-ethyl-1H-indazol-6-yl)-cyclohexanecarboxylic acid;

*Trans*-4-cyano-4-(1-cyclobutyl-3-ethyl-1H-indazol-6-yl)cyclohexanecarboxylic acid;

6-Bromo-3-ethyl-1-(4-fluorophenyl)-1 H-indazole;

4-[3-Ethyl-1-(4-fluorophenyl)-1 H-indazol-6-yl]-4-hydroxycyclohexanecarboxylic acid ethyl ester;

4-Cyano-4-[3-ethyl-1-(4-fluorophenyl)-1H-indazol-6-yl]cyclohexanecarboxylic acid ethyl ester;

4-[3-Ethyl-1-(4-fluorophenyl)-1H-indazol-6-yl]cyclohex-3-enecarboxylic acid ethyl ester;

4-Cyano-4-(1-cyclohexyl-3-ethyl-1H-indazol-6-yl)-cyclohexanecarboxylic acid ethyl ester;

*Cis*-4-Cyano-4-[3-ethyl-1-(4-fluorophenyl)-1H-indazol-6-yl]cyclohexanecarboxylic acid;

4-[3-Ethyl-1-(4-fluorophenyl)-1H-indazol-6-yl]cyclohex-3-enecarboxylic acid; and

4-(1-Cyclohexyl-3-ethyl-1H-indazol-6-yl)-4-hydroxycyclohexanecarboxylic acid.

Certain species of above-desecribed compounds may have asymmetric centers and therefore exist in different enantiomeric forms. All optical isomers and stereoisomers of the compounds of Formula (IA) or (IB), and mixtures thereof, are considered to be within the scope of the invention. With respect to the compounds of Formula (IA) or (IB), the invention includes the use of a racemate, a single enantiomeric form, a single diastereomeric form, or mixtures thereof. The compounds of Formula (IA) or (IB) may also exist as tautomers. This invention relates to the use of all such tautomers and mixtures thereof.

The above-described compounds useful as therapeutic agents in the methods of treatment and pharmaceutical compositions of the present invention may be utilized in the form of acids, esters, or other chemical classes of compounds to which the compounds described belong. It is also within the scope of the present invention to utilize those compounds in the form of pharmaceutically acceptable salts derived from various organic and inorganic acids and bases in accordance with procedures well known in the art. The expression "pharmaceutically acceptable salt" as used herein is intended to mean an active ingredient comprising a compound of Formula (I) utilized in the form of a salt thereof, especially where said salt form confers on said active ingredient improved pharmacokinetic properties as compared to the free form of said active ingredient or some other salt form of said active ingredient utilized previously. The pharmaceutically acceptable salt form of said active ingredient may also initially confer a desirable pharmacokinetic property on said active ingredient which it did not previously possess, and may even positively affect the pharmacodynamics of said active ingredient with respect to its therapeutic activity in the body.

The pharmacokinetic properties of said active ingredient which may be favorably affected include, *e.g.,* the manner in which said active ingredient is transported across cell membranes, which in turn may directly and positively affect the absorption, distribution, biotransformation and excretion of said active ingredient. While the route of administration of the pharmaceutical composition is important, and various anatomical, physiological and pathological factors can critically affect bioavailability, the solubility of said active ingredient is usually dependent upon the character of the particular salt form thereof which it utilized. Further, as the artisan understands, an aqueous solution of said active ingredient will provide the most rapid absorption of said active ingredient into the body of a patient being treated, while lipid solutions and suspensions, as well as solid dosage forms, will result in less rapid absorption of said active ingredient. Oral ingestion of said active ingredient is the most preferred route of administration for reasons of safety, convenience, and economy, but absorption of such an oral dosage form can be adversely affected by physical characteristics such as polarity, emesis caused by irritation of the gastrointestinal mucosa, destruction by digestive enzymes and low pH, irregular absorption or propulsion in the presence of food or other drugs, and metabolism by enzymes of the mucosa, the intestinal flora, or the liver. Formulation of said active ingredient into different pharmaceutically acceptable salt forms may be effective in overcoming or alleviating one or more of the above-recited problems encountered with absorption of oral dosage forms.

Well-known pharmaceutically acceptable salts include, but are not limited to acetate, adipate, alginate, aspartate, benzoate, benzenesulfonate, besylate, bisulfate, butyrate, citrate, camphorate, camphorsulfonate, cyclopentanepropionate, digluconate, dodecysulfate, ethanesulfonate, fumarate, glucoheptanoate, gluconate, glycerophosphate, hemisuccinate, hemisulfate, heptanoate, hexanoate, hippurate, hydrochloride, hydrobromide, hydroiodide, 2-hydroxyethanesulfonate, isethionate, lactate, lactobionate, maleate, mandelate, methanesulfonate, 2-naphthalenesulfonate, nicotinate, nitrate, oxalate, oleate, pamoate, pectinate, persulfate, 3-phenylpropionate, phosphonate, picrate, pivalate, propionate, salicylate, sodium phosphate, stearate, succinate, sulfate, sulfosalicylate, tartrate, thiocyanate, thiomalate, tosylate, and undecanoate.

Base salts of the compounds of the present invention include, but are not limited to ammonium salts; alkali metal salts such as sodium and potassium; alkaline earth metal salts such as calcium and magnesium; salts with organic bases such as dicyclohexylamine, meglumine, N-methyl-D-glucamine, tris-(hydroxymethyl)-methylamine (tromethamine), and salts with amino acids such as arginine, lysine, etc. Compounds of the present invention which

comprise basic nitrogen-containing groups may be quaternized with such agents as ($C_1$-$C_4$)alkyl halides, *e.g.*, methyl, ethyl, *iso*-propyl and *tert*-butyl chlorides, bromides and iodides; di($C_1$-$C_4$)alkyl sulfate, *e.g.*, dimethyl, diethyl and diamyl sulfates; ($C_{10}$-$C_{18}$)alkyl halides, *e.g.,* decyl, dodecyl, lauryl, myristyl and stearyl chlorides, bromides and iodides; and aryl-($C_1$-$C_4$) alkyl halides, *e.g.*, benzyl chloride and phenethyl bromide. Such salts permit the preparation of both water-soluble and oil-soluble compounds of the present invention.

Among the above-recited pharmaceutical salts those which are preferred include, but are not limited to acetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate, and tromethamine.

Multiple salts forms are included within the scope of the present invention where a compound of the present invention contains more than one group capable of forming such pharmaceutically acceptable salts. Examples of typical multiple salt forms include, but are not limited to bitartrate, diacetate, difumarate, dimeglumine, diphosphate, disodium, and trihydrochloride.

The pharmaceutical compositions of the present invention comprise any one or more of the above-described antagonist compounds of the present invention, or a pharmaceutically acceptable salt thereof as also above-described, together with a pharmaceutically acceptable carrier in accordance with the properties and expected performance of such carriers which are well-known in the pertinent art.

The term "carrier" as used herein includes acceptable diluents, excipients, adjuvants, vehicles, solubilization aids, viscosity modifiers, preservatives and other agents well known to the artisan for providing favorable properties in the final pharmaceutical composition. In order to illustrate such carriers, there follows a brief survey of pharmaceutically acceptable carriers that may be used in the pharmaceutical compositions of the present invention, and thereafter a more detailed description of the various types of ingredients. Typical carriers include but are by no means limited to, ion exchange compositions; alumina; aluminum stearate; lecithin; serum proteins, *e.g.*, human serum albumin; phosphates; glycine; sorbic acid; potassium sorbate; partial glyceride mixtures of saturated vegetable fatty acids; hydrogenated palm oils; water; salts or electrolytes, *e.g.*, prolamine sulfate, disodium hydrogen phosphate, potassium hydrogen phosphate, sodium chloride, and zinc salts; colloidal silica; magnesium trisilicate; polyvinyl pyrrolidone; cellulose-based substances; *e.g.*, sodium carboxymethylcellulose; polyethylene glycol; polyacrylates; waxes; polyethylene-polyoxypropylene-block polymers; and wool fat.

More particularly, the carriers used in the pharmaceutical compositions of the present invention comprise various classes and species of additives which are members independently selected from the groups consisting essentially of those recited in the following paragraphs.

Acidifying and alkalizing agents are added to obtain a desired or predetermined pH and comprise acidifying agents, *e.g.,* acetic acid, glacial acetic acid, malic acid, and propionic acid. Stronger acids such as hydrochloric acid, nitric acid and sulfuric acid may be used but are less preferred. Alkalizing agents include, *e.g.*, edetol, potassium carbonate, potassium hydroxide, sodium borate, sodium carbonate, and sodium hydroxide. Alkalizing agents which contain active amine groups, such as diethanolamine and trolamine, may also be used.

Aerosol propellants are required where the pharmaceutical composition is to be delivered as an aerosol under significant pressure. Such propellants include, *e.g.*, acceptable fluorochlorohydrocarbons such as dichlorodifluoromethane, dichlorotetrafluoroethane, and trichloromonofluoromethane; nitrogen; or a volatile hydrocarbon such as butane, propane, isobutane or mixtures thereof.

Antimicrobial agents including antibacterial, antifungal and antiprotozoal agents are added where the pharmaceutical composition is topically applied to areas of the skin which are likely to have suffered adverse conditions or sustained abrasions or cuts which expose the skin to infection by bacteria, fungi or protozoa. Antimicrobial agents include such compounds as benzyl alcohol, chlorobutanol, phenylethyl alcohol, phenylmercuric acetate, potassium sorbate, and sorbic acid. Antifungal agents include such compounds as benzoic acid, butylparaben, ethylparaben, methylparaben, propylparaben, and sodium benzoate.

Antimicrobial preservatives are added to the pharmaceutical compositions of the present invention in order to protect them against the growth of potentially harmful microorganisms, which usually invade the aqueous phase, but in some cases can also grow in the oil phase of a composition. Thus, preservatives with both aqueous and lipid solubility are desirable. Suitable antimicrobial preservatives include, *e.g.*, alkyl esters of p-hydroxybenzoic acid, propionate salts, phenoxyethanol, methylparaben sodium, propylparaben sodium, sodium dehydroacetate, benzalkonium chloride, benzethonium chloride, benzyl alcohol, hydantoin derivatives, quaternary ammonium compounds and cationic polymers, imidazolidinyl urea, diazolidinyl urea, and trisodium ethylenediamine tetracetate (EDTA). Preservatives are preferably employed in amounts ranging from about 0.01% to about 2.0% by weight of the total composition.

Antioxidants are added to protect all of the ingredients of the pharmaceutical composition from damage or degradation by oxidizing agents present in the composition itself or the use environment, *e.g.*, anoxomer, ascorbyl palmitate, butylated hydroxyanisole, butylated hydroxytoluene, hypophosphorous acid, potassium metabisulfite, propyl octyl and dodecyl gallate, sodium metabisulfite, sulfur dioxide, and tocopherols.

Buffering agents are used to maintain a desired pH of a composition once established, from the effects of outside agents and shifting equilibria of components of the composition. The buffering may be selected from among those familiar to the artisan skilled in the preparation of pharmaceutical compositions, *e.g.*, calcium acetate, potassium metaphosphate, potassium phosphate monobasic, and tartaric acid.

Chelating agents are used to help maintain the ionic strength of the pharmaceutical composition and bind to and effectively remove destructive compounds and metals, and include, *e.g.,* edetate dipotassium, edetate disodium, and edetic acid.

Dermatologically active agents are added to the pharmaceutical compositions of the present invention where they are to be applied topically, and include, *e.g.,* wound healing agents such as peptide derivatives, yeast, panthenol, hexylresorcinol, phenol, tetracycline hydrochloride, lamin and kinetin; retinoids for treating skin cancer, *e.g.,* retinol, tretinoin, isotretinoin, etretinate, acitretin, and arotinoid; mild antibacterial agents for treating skin infections, *e.g.*, resorcinol, salicylic acid, benzoyl peroxide, erythromycin-benzoyl peroxide, erythromycin, and clindamycin; antifungal agents for treating tinea corporis, tinea pedis, candidiasis and tinea versicolor, *e.g.*, griseofulvin, azoles such as miconazole, econazole, itraconazole, fluconazole, and ketoconazole, and allylamines such as naftifine and terfinafine; antiviral agents for treating cutaneous herpes simplex, herpes zoster, and chickenpox, *e.g.,* acyclovir, famciclovir, and valacyclovir; antihistamines for treating pruritis, atopic and contact dermatitis, *e.g.*, diphenhydramine, terfenadine, astemizole, loratadine, cetirizine, acrivastine, and temelastine; topical anesthetics for relieving pain, irritation and itching, *e.g.*, benzocaine, lidocaine, dibucaine, and pramoxine hydrochloride; topical analgesics for relieving pain and inflammation, *e.g.*, methyl salicylate, camphor, menthol, and resorcinol; topical antiseptics for preventing infection, *e.g.*, benzalkonium chloride and povidone-iodine; and vitamins and derivatives thereof such as tocopherol, tocopherol acetate, retinoic acid and retinol.

Dispersing and suspending agents are used as aids for the preparation of stable formulations and include, *e.g.*, poligeenan, povidone, and silicon dioxide.

Emollients are agents, preferably non-oily and water-soluble, which soften and soothe the skin, especially skin that has become dry because of excessive loss of water. Such agents are used with pharmaceutical compositions of the present invention which are intended for topical applications, and include,, *e.g.*, hydrocarbon oils and waxes, triglyceride esters, acetylated monoglycerides, methyl and other alkyl esters of $C_{10}$ -$C_{20}$ fatty acids, $C_{10}$ - $C_{20}$ fatty acids, $C_{10}$ -$C_{20}$ fatty alcohols, lanolin and derivatives, polyhydric alcohol esters such as polyethylene glycol (200-600), polyoxyethylene sorbitan fatty acid esters, wax esters, phospholipids, and sterols; emulsifying agents used for preparing oil-in-water emulsions; excipients, *e.g.*, laurocapram and polyethylene glycol monomethyl ether; humectants, *e.g.*, sorbitol, glycerin and hyaluronic acid; ointment bases, *e.g.*, petrolatum, polyethylene glycol, lanolin, and poloxamer; penetration enhancers, *e.g.*, dimethyl isosorbide, diethyl-glycol-monoethylether, 1-dodecylazacycloheptan-2-one, and dimethylsulfoxide (DMSO); preservatives, *e.g.*, benzalkonium chloride, benzethonium chloride, alkyl esters of p-hydroxybenzoic acid, hydantoin derivatives, cetylpyridinium chloride, propylparaben, quaternary ammonium compounds such as potassium benzoate, and thimerosal; sequestering agents comprising cyclodextrins; solvents, *e.g.*, acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water; stabilizers, *e.g.*, calcium saccharate and thymol; surfactants, *e.g.*, lapyrium chloride; laureth 4, *i.e.*, $\alpha$-dodecyl-$\omega$-hydroxy-poly(oxy-1,2-ethanediyl) or polyethylene glycol monododecyl ether.

Emulsifying agents, including emulsifying and stiffening agents and emulsion adjuncts, are used for preparing oil-in-water emulsions when these form the basis of the pharmaceutical compositions of the present invention. Such emulsifying agents include, *e.g.*, non-ionic emulsifiers such as $C_{10}$ -$C_{20}$ fatty alcohols and said fatty alcohols condensed with from 2 to 20 moles of ethylene oxide or propylene oxide, ($C_6$ -$C_{12}$)alkyl phenols condensed with from 2 to 20 moles of ethylene oxide, mono- and di- $C_{10}$ -$C_{20}$ fatty acid esters of ethylene glycol, $C_{10}$ -$C_{20}$ fatty acid monoglyceride, diethylene glycol, polyethylene glycols of MW 200-6000, polypropylene glycols of MW 200-3000, and particularly sorbitol, sorbitan, polyoxyethylene sorbitol, polyoxyethylene sorbitan, hydrophilic wax esters, cetostearyl alcohol, oleyl alcohol, lanolin alcohols, cholesterol, mono- and di-glycerides, glyceryl monostearate, polyethylene glycol monostearate, mixed mono- and distearic esters of ethylene glycol and polyoxyethylene glycol, propylene glycol monostearate, and hydroxypropyl cellulose. Emulsifying agents which contain active amine groups may also be used and typically include anionic emulsifiers such as fatty acid soaps, *e.g.*, sodium, potassium and triethanolamine soaps of $C_{10}$ -$C_{20}$ fatty acids; alkali metal, ammonium or substituted ammonium ($C_{10}$ -$C_{30}$)alkyl sulfates, ($C_{10}$ -$C_{30}$)alkyl sulfonates, and ($C_{10}$ -$C_{50}$)alkyl ethoxy ether sulfonates. Other suitable emulsifying agents include castor oil and hydrogenated castor oil; lecithin; and polymers of 2-propenoic acid together with polymers of acrylic acid, both cross-linked with allyl ethers of sucrose and/or pentaerythritol, having varying viscosities and identified by product names carbomer 910, 934, 934P, 940, 941, and 1342. Cationic emulsifiers having active amine groups may also be used, including those based on quaternary ammonium, morpholinium and pyridinium compounds. Similarly, amphoteric emulsifiers having active amine groups, such as cocobetaines, lauryl dimethylamine oxide and cocoylimidazoline, may be used. Useful emulsifying and stiffening

agents also include cetyl alcohol and sodium stearate; and emulsion adjuncts such as oleic acid, stearic acid, and stearyl alcohol.

Excipients include, *e.g.*, laurocapram and polyethylene glycol monomethyl ether.

Where the pharmaceutical composition of the present invention is to be applied topically, penetration enhancers may be used, which include, *e.g.*, dimethyl isosorbide, diethyl-glycol-monoethylether, 1-dodecylazacycloheptan-2-one, and dimethylsulfoxide (DMSO). Such compositions will also typically include ointment bases, *e.g.*, petrolatum, polyethylene glycol, lanolin, and poloxamer, which is a block copolymer of polyoxyethylene and polyoxypropylene, which may also serve as a surfactant or emulsifying agent.

Preservatives are used to protect pharmaceutical compositions of the present invention from degradative attack by ambient microorganisms, and include, *e.g.*, benzalkonium chloride, benzethonium chloride, alkyl esters of p-hydroxybenzoic acid, hydantoin derivatives, cetylpyridinium chloride, monothioglycerol, phenol, phenoxyethanol, methylparagen, imidazolidinyl urea, sodium dehydroacetate, propylparaben, quaternary ammonium compounds, especially polymers such as polixetonium chloride, potassium benzoate, sodium formaldehyde sulfoxylate, sodium propionate, and thimerosal.

Sequestering agents are used to improve the stability of the pharmaceutical compositions of the present invention and include, *e.g.*, the cyclodextrins which are a family of natural cyclic oligosaccharides capable of forming inclusion complexes with a variety of materials, and are of varying ring sizes, those having 6-, 7- and 8-glucose residues in a ring being commonly referred to as α-cyclodextrins, β-cyclodextrins, and γ-cyclodextrins, respectively. Suitable cyclodextrins include, *e.g.*, α-cyclodextrin, β-cyclodextrin, γ-cyclodextrin, δ-cyclodextrin and cationized cyclodextrins.

Solvents which may be used in preparing the pharmaceutical compositions of the present invention include, *e.g.,* acetone, alcohol, amylene hydrate, butyl alcohol, corn oil, cottonseed oil, ethyl acetate, glycerin, hexylene glycol, isopropyl alcohol, isostearyl alcohol, methyl alcohol, methylene chloride, mineral oil, peanut oil, phosphoric acid, polyethylene glycol, polyoxypropylene 15 stearyl ether, propylene glycol, propylene glycol diacetate, sesame oil, and purified water.

Stabilizers which are suitable for use include, *e.g.*, calcium saccharate and thymol.

Stiffening agents are typically used in formulations for topical applications in order to provide desired viscosity and handling characteristics and include, *e.g.*, cetyl esters wax, myristyl alcohol, parafin, synthetic parafin, emulsifying wax, microcrystalline wax, white wax and yellow wax.

Sugars are often used to impart a variety of desired characteristics to the pharmaceutical compositions of the present invention and in order to improve the results obtained, and include, *e.g.*, monosaccharides, disaccharides and polysaccharides such as glucose, xylose, fructose, reose, ribose, pentose, arabinose, allose, tallose, altrose, mannose, galactose, lactose, sucrose, erythrose, glyceraldehyde, or any combination thereof.

Surfactants are employed to provide stability for multi-component pharmaceutical compositions of the present invention, enhance existing properties of those compositions, and bestow desirable new characteristics on said compositions. Surfactants are used as wetting agents, antifoam agents, for reducing the surface tension of water, and as emulsifiers, dispersing agents and penetrants, and include, *e.g.,* lapyrium chloride; laureth 4, *i.e.*, α-dodecyl-ω-hydroxy-poly(oxy-1,2-ethanediyl) or polyethylene glycol monododecyl ether; laureth 9, *i.e.,* a mixture of polyethylene glycol monododecyl ethers averaging about 9 ethylene oxide groups per molecule; monoethanolamine; nonoxynol 4, 9 and 10, *i.e.,* polyethylene glycol mono(p-nonylphenyl) ether; nonoxynol 15, *i.e.,* α-(*p*-nonylphenyl)-ω-hydroxypentadeca (oxyethylene); nonoxynol 30, *i.e.,* α-(*p*-nonylphenyl)-ω-hydroxytriaconta(oxyethylene); poloxalene, i.e., nonionic polymer of the polyethylene-polypropylene glycol type, MW = approx. 3000; poloxamer, referred to in the discussion of ointment bases further above; polyoxyl 8, 40 and 50 stearate, *i.e.*, poly(oxy-1,2-ethanediyl), α-hydro-ω-hydroxy-; octadecanoate; polyoxyl 10 oleyl ether, *i.e.*, poly(oxy-1,2-ethanediyl), α-[(Z)-9-octadecenyl-ω-hydroxy-; polysorbate 20, *i.e.,* sorbitan, monododecanoate, poly(oxy-1,2-ethanediyl); polysorbate 40, *i.e.,* sorbitan, monohexadecanoate, poly (oxy-1,2-ethanediyl); polysorbate 60, *i.e.*, sorbitan, monooctadecanoate, poly(oxy-1,2-ethanediyl); polysorbate 65, *i.e.,* sorbitan, trioctadecanoate, poly(oxy-1,2-ethanediyl); polysorbate 80, *i.e.,* sorbitan, mono-9-monodecenoate, poly (oxy-1,2-ethanediyl); polysorbate 85, *i.e.,* sorbitan, tri-9-octadecenoate, poly(oxy-1,2-ethanediyl); sodium lauryl sulfate; sorbitan monolaurate; sorbitan monooleate; sorbitan monopalmitate; sorbitan monostearate; sorbitan sesquioleate; sorbitan trioleate; and sorbitan tristearate.

The pharmaceutical compositions of the present invention may be prepared using very straightforward methodology which is well understood by the artisan of ordinary skill. Where the pharmaceutical compositions of the present invention are simple aqueous and/or other solvent solutions, the various components of the overall composition are brought together in any practical order, which will be dictated largely by considerations of convenience. Those components having reduced water solubility, but sufficient solubility in the same co-solvent with water, may all be dissolved in said co-solvent, after which the co-solvent solution will be added to the water portion of the carrier whereupon the solutes therein will become dissolved in the water. To aid in this dispersion/solution process, a surfactant may be employed.

Where the pharmaceutical compositions of the present invention are to be in the form of emulsions, the components of the pharmaceutical composition will be brought together in accordance with the following general procedures. The continuous water phase is first heated to a temperature in the range of from about 60° to about 95°C, preferably from about 70° to about 85°C, the choice of which temperature to use being dependent upon the physical and chemical properties of the components which make up the oil-in-water emulsion. Once the continuous water phase has reached its selected temperature, the components of the final composition to be added at this stage are admixed with the water and dispersed therein under high-speed agitation. Next, the temperature of the water is restored to approximately its original level, after which the components of the composition which comprise the next stage are added to the composition mixture under moderate agitation and mixing continues for from about 5 to about 60 minutes, preferably about 10 to about 30 minutes, depending on the components of the first two stages. Thereafter, the composition mixture is passively or actively cooled to from about 20° to about 55°C for addition of any components in the remaining stages, after which water is added in sufficient quantity to reach its original predetermined concentration in the overall composition.

According to the present invention, the pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically-acceptable oils, such as olive oil or castor oil, especially in their polyoxyethylated versions. These oil solutions or suspensions may also contain a long-chain alcohol diluent or dispersant, such as *Rh,* HCIX or similar alcohol.

The pharmaceutical compositions of the present invention may be orally administered in any orally acceptable dosage form including, but not limited to, capsules, tablets, aqueous suspensions or solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added. Alternatively, the pharmaceutical compositions of this invention may be administered in the form of suppositories for rectal administration. These can be prepared by mixing the agent with a suitable non-irritating excipient which is solid at room temperature but liquid at the rectal temperature and therefore will melt in the rectum to release the drug. Such materials include cocoa butter, beeswax and polyethylene glycols.

The pharmaceutical compositions of the present invention may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation, as described above, or in a suitable enema formulation. Topically active transdermal patches may also be used.

For topical applications, the pharmaceutical compositions may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion or cream containing the active components suspended or dissolved in one or more pharmaceutically acceptable carriers. Suitable carriers include, but are not limited to, mineral oil, sorbitan monostearate, polysorbate , cetyl esters wax, cetearyl alcohol, 2-octyldodecanol, benzyl alcohol and water.

Pharmaceutical compositions within the scope of the present invention include those wherein the therapeutically effective amount of an active ingredient comprising a compound of Formula (IA) or (IB) required for treating or preventing stomach stasis, especially gastric hypomotility and related disorders which are mediated by or associated with inhibition of PDE4 isozyme activity as described herein, is provided in a dosage form suitable for systemic administration. Such a pharmaceutical composition will contain said active ingredient in suitable liquid form for delivery by: (1) injection or infusion which is intraarterial, intra- or transdermal, subcutaneous, intramuscular, intraspinal, intrathecal, or intravenous, wherein said active ingredient: (a) is contained in solution as a solute; (b) is contained in the discontinuous phase of an emulsion, or the discontinuous phase of an inverse emulsion which inverts upon injection or infusion, said emulsions containing suitable emulsifying agents; or (c) is contained in a suspension as a suspended solid in colloidal or microparticulate form, said suspension containing suitable suspending agents; (2) injection or infusion into suitable body tissues or cavities as a depot, wherein said composition provides storage of said active ingredient and thereafter delayed-, sustained-, and/or controlled-release of said active ingredient for systemic distribution; (3)

instillation, inhalation or insufflation into suitable body tissues or cavities of said pharmaceutical composition in suitable solid form, where said active ingredient: (a) is contained in a solid implant composition providing delayed-, sustained-, and/or controlled-release of said active ingredient; (b) is contained in a particulate composition to be inhaled into the lungs; or (c) is contained in a particulate composition to be blown into suitable body tissues or cavities, where said composition optionally provides delayed-, sustained-, and/or controlled-release of said active ingredient; or (4) ingestion of said pharmaceutical composition in suitable solid or liquid form for peroral delivery of said active ingredient, where said active ingredient is contained in a solid dosage form; or (b) is contained in a liquid dosage form.

Particular dosage forms of the above-described pharmaceutical compositions include (1) suppositories as a special type of implant, comprising bases which are solid at room temperature but melt at body temperature, slowly releasing the active ingredient with which they are impregnated into the surrounding tissue of the body, where the active ingredient becomes absorbed and transported to effect systemic administration; (2) solid peroral dosage forms selected from the group consisting of (a) delayed-release oral tablets, capsules, caplets, lozenges, troches, and multiparticulates; (b) enteric-coated tablets and capsules which prevent release and absorption in the stomach to facilitate delivery distal to the stomach of the patient being treated; (c) sustained-release oral tablets, capsules and microparticulates which provide systemic delivery of the active ingredient in a controlled manner up to a 24-hour period; (d) fast-dissolving tablets; (e) encapsulated solutions; (f) an oral paste; (g) a granular form incorporated in or to be incorporated in the food of a patient being treated; and (h) liquid peroral dosage forms selected from the group consisting of solutions, suspensions, emulsions, inverse emulsions, elixirs, extracts, tinctures, and concentrates.

Pharmaceutical compositions within the scope of the present invention include those wherein the therapeutically effective amount of an active ingredient comprising a compound of Formula (IA) or (IB) as described herein required for treating or preventing gastric stasis, especially gastric hypomotility and disorders related thereto which are mediated by or associated with inhibition of PDE4 isozyme activity as described herein, is provided in a dosage form suitable for local administration to a patient being treated, wherein said pharmaceutical composition contains said active ingredient in suitable liquid form for delivering said active ingredient by: (1) injection or infusion into a local site which is intraarterial, intraarticular, intrachondrial, intracostal, intracystic, intra- or transdermal, intrafasicular, intraligamentous, intramedulary, intramuscular, intranasal, intraneural, intraocular, i.e., opthalmic administration, intraosteal, intrapelvic, intrapericardial, intraspinal, intrasternal, intrasynovial, intratarsal, or intrathecal; including components which provide delayed-release, controlled-release, and/or sustained-release of said active ingredient into said local site; where said active ingredient is contained: (a) in solution as a solute; (b) in the discontinuous phase of an emulsion, or the discontinuous phase of an inverse emulsion which inverts upon injection or infusion, said emulsions containing suitable emulsifying agents; or (c) in a suspension as a suspended solid in colloidal or microparticulate form, said suspension containing suitable suspending agents; or (2) injection or infusion as a depot for delivering said active ingredient to said local site; wherein said composition provides storage of said active ingredient and thereafter delayed-, sustained-, and/or controlled-release of said active ingredient into said local site, and wherein said composition also includes components which ensure that said active ingredient has predominantly local activity, with little systemic carryover activity; or wherein said pharmaceutical composition contains said active ingredient in suitable solid form for delivering said inhibitor by: (3) instillation, inhalation or insufflation to said local site, where said active ingredient is contained: (a) in a solid implant composition which is installed in said local site, said composition optionally providing delayed-, sustained-, and/or controlled-release of said active ingredient to said local site; (b) in a particulate composition which is inhaled into a local site comprising the lungs; or (c) in a particulate composition which is blown into a local site, where said composition includes components which will ensure that said active ingredient has predominantly local activity, with insignificant systemic carryover activity, and optionally provides delayed-, sustained-, and/or controlled-release of said active ingredient to said local site. For ophthalmic use, the pharmaceutical compositions may be formulated as micronized suspension in isotonic, pH adjusted sterile saline, or, preferably, as solutions in isotonic, pH adjusted sterile saline, either with our without a preservative such as benzylalkonium chloride. Alternatively, for ophthalmic uses, the pharmaceutical compositions may be formulated in an ointment such as petrolatum.

The pharmaceutical compositions of the present invention may also be administered by nasal aerosol or inhalation through the use of a nebulizer, a dry powder inhaler or a metered dose inhaler. Such compositions are prepared according to techniques well-known in the art of pharmaceutical formulation and may be prepared as solutions in saline, employing benzyl alcohol or other suitable preservatives, absorption promoters to enhance bioavailability, hydrofluorocarbons, and/or other conventional solubilizing or dispersing agents.

As already mentioned, the active ingredients of Formula (IA) or (IB) of the present invention may be administered systemically to a patient to be treated as a pharmaceutical composition in suitable liquid form by injection or infusion. There are a number of sites and organ systems in the body of the patient which will allow the properly formulated pharmaceutical composition, once injected or infused, to permeate the entire body and all of the organ system of the patient being treated. An injection is a single dose of the pharmaceutical composition forced, usually by a syringe, into the tissue involved. The most common types of injections are intramuscular, intravenous, and subcutaneous. By contrast, an infusion is the gradual introduction of the pharmaceutical composition into the tissue involved. The most

common type of infusion is intravenous. Other types of injection or infusion comprise intraarterial, intra- or transdermal (including subcutaneous), or intraspinal especially intrathecal. In these liquid pharmaceutical compositions, the active ingredient may be contained in solution as the solute. This is the most common and most preferred type of such composition, but requires an active ingredient in a salt form that has reasonably good aqueous solubility. Water (or saline) is by far the most preferred solvent for such compositions. Occasionally supersaturated solutions may be utilized, but these present stability problems that make them impractical for use on an everyday basis.

If it is not possible to obtain a form of some compound of Formula (IA) or (IB) that has the requisite degree of aqueous solubility, as may sometimes occur, it is within the skill of the artisan to prepare an emulsion, which is a dispersion of small globules of one liquid, the discontinuous or internal phase, throughout a second liquid, the continuous or external phase, with which it is immiscible. The two liquids are maintained in an emulsified state by the use of emulsifiers which are pharmaceutically acceptable. Thus, if the active ingredient is a water-insoluble oil, it can be administered in an emulsion of which it is the discontinuous phase. Also where the active ingredient is water-insoluble but can be dissolved in a solvent which is immiscible with water, an emulsion can be used. While the active ingredient would most commonly be used as the discontinuous or internal phase of what is referred to as an oil-in-water emulsion, it could also be used as the discontinuous or internal phase of an inverse emulsion, which is commonly referred to as a water-in-oil emulsion. Here the active ingredient is soluble in water and could be administered as a simple aqueous solution. However, inverse emulsions invert upon injection or infusion into an aqueous medium such as the blood, and offer the advantage of providing a more rapid and efficient dispersion of the active ingredient into that aqueous medium than can be obtained using an aqueous solution. Inverse emulsions are prepared by using suitable, pharmaceutically acceptable emulsifying agents well known in the art. Where the active ingredient has limited water solubility, it may also be administered as a suspended solid in colloidal or microparticulate form in a suspension prepared using suitable, pharmaceutically acceptable suspending agents. The suspended solids containing the active ingredient may also be formulated as delayed-, sustained-, and/or controlled-release compositions.

While systemic administration will most frequently be carried out by injection or infusion of a liquid, there are many situations in which it will be advantageous or even necessary to deliver the active ingredient as a solid. Systemic administration of solids is carried out by instillation, inhalation or insufflation of a pharmaceutical composition in suitable solid form containing the active ingredient. Instillation of the active ingredient may entail installing a solid implant composition into suitable body tissues or cavities. The implant may comprise a matrix of bio-compatible and bio-erodible materials in which particles of a solid active ingredient are dispersed, or in which, possibly, globules or isolated cells of a liquid active ingredient are entrapped. Desirably, the matrix will be broken down and completely absorbed by the body. The composition of the matrix is also preferably selected to provide controlled-, sustained-, and/or delayed release of the active ingredient over extended periods of time, even as much as several months.

The term "implant" most often denotes a solid pharmaceutical composition containing the active ingredient, while the term "depot" usually implies a liquid pharmaceutical composition containing the active ingredient, which is deposited in any suitable body tissues or cavities to form a reservoir or pool which slowly migrates to surrounding tissues and organs and eventually becomes systemically distributed. However, these distinctions are not always rigidly adhered to in the art, and consequently, it is contemplated that there is included within the scope of the present invention liquid implants and solid depots, and even mixed solid and liquid forms for each. Suppositories may be regarded as a type of implant, since they comprise bases which are solid at room temperature but melt at a patient's body temperature, slowly releasing the active ingredient with which they are impregnated into the surrounding tissue of the patient's body, where the active ingredient becomes absorbed and transported to effect systemic administration.

Systemic administration can also be accomplished by inhalation or insufflation of a powder, *i.e.,* particulate composition containing the active ingredient. For example, the active ingredient in powder form may be inhaled into the lungs using conventional devices for aerosolizing particulate formulations. The active ingredient as a particulate formulation may also be administered by insufflation, *i.e*., blown or otherwise dispersed into suitable body tissues or cavities by simple dusting or using conventional devices for aerosolizing particulate formulations. These particulate compositions may also be formulated to provide delayed-, sustained-, and/or controlled-release of the active ingredient in accordance with well understood principles and known materials.

Other means of systemic administration which may utilize the active ingredients of the present invention in either liquid or solid form include transdermal, intranasal, and opthalmic routes. In particular, transdermal patches prepared in accordance with well known drug delivery technology may be prepared and applied to the skin of a patient to be treated, whereafter the active agent by reason of its formulated solubility characteristics migrates across the epidermis and into the dermal layers of the patient's skin where it is taken up as part of the general circulation of the patient, ultimately providing systemic distribution of the active ingredient over a desired, extended period of time. Also included are implants which are placed beneath the epidermal layer of the skin, *i.e.* between the epidermis and the dermis of the skin of the patient being treated. Such an implant will be formulated in accordance with well known principles and materials commonly used in this delivery technology, and may be prepared in such a way as to provide controlled-, sustained-, and/or delayed-release of the active ingredient into the systemic circulation of the patient. Such

subepidermal (subcuticular) implants provide the same facility of installation and delivery efficiency as transdermal patches, but without the limitation of being subject to degradation, damage or accidental removal as a consequence of being exposed on the top layer of the patient's skin.

In the above description of pharmaceutical compositions containing an active ingredient of Formula (IA) or (IB), the equivalent expressions: "administration", "administration or, "administering", and "administering a" have been used with respect to said pharmaceutical compositions. As thus employed, these expressions are intended to mean providing to a patient in need of treatment a pharmaceutical composition of the present invention by any of the routes of administration herein described, wherein the active ingredient is a compound of Formula (IA) or (IB) or a prodrug, derivative, or metabolite thereof which is useful in treating gastric stasis, especially gastric hypomotility and related disorders which are mediated by or associated with inhibition of PDE4 isozyme activity in said patient. Accordingly, there is included within the scope of the present invention any other compound which, upon administration to a patient, is capable of directly or indirectly providing a compound of Formula (IA) or (IB). Such compounds are recognized as prodrugs, and a number of established procedures are available for preparing such prodrug forms of the compounds of Formula (IA) or (IB).

The compounds of Formula (IA) or (IB) are described herein as possessing biological activity such that they are able to inhibit PDE4 isozyme activity and consequent or associated pathogenic processes subsequently mediated by the PDE4 isozyme. The expression "inhibit PDE4 isozyme activity" as used herein is intended to refer to manipulation of the basic physiological processes and agencies which involve the PDE4 isozyme. Included within the scope of this intended meaning are all types and subtypes of PDE4 isozymes, in whatever tissues of a particular patient they are found, and in or on whatever components of the cells comprising those tissues they may be located.

The basic functioning of the PDE4 isozyme may be modulated, *i.e.,* inhibited in a number of ways, and the scope of the present invention is not limited in that regard to any particular existing or hypothesized pathway or process. Thus, included within the intended meaning of inhibition of PDE4 isozyme activity, is the use of synthetically derived inhibitors introduced into a patient being treated, such as the compounds of Formula (IA) or (IB) described herein. These exogenous agents may inhibit PDE4 isozyme activity by such well known mechanisms as competitive transition state binding in which the natural substrate of the PDE4 isozyme is displaced and its inherent function thereby disrupted. However, the present invention is not limited to any such specific mechanism or mode of action. Correspondingly, the term "therapeutically effective amount" means the amount of the subject compound that will elicit the biological or medical response of a tissue, system, animal or human that is being sought.

The term "patient" as used above and throughout the instant specification, refers particularly to mammals, including especially humans. However, it is not intended that the present invention be thereby limited. The therapeutic agents, methods of treatment and pharmaceutical compositions of the present invention may be used in the treatment of animals. The terms: "animal" and "animals" as used herein refer to all members of the animal kingdom and the primary divisions thereof which may be beneficially treated with the compounds of Formula (I). Included among the major phyla and subdivions thereof are, *e.g.,* vertebrates of the subphylum *Vertebrata* which includes classes of mammals (*Mammalia*), birds reptiles (*Reptilia*), amphibians (*Amphibia*) and fishes. In preferred embodiments of the present invention, the patient being treated or at risk for becoming such a patient, is a human, monkey, dog, cat, cow, pig, sheep, goat, horse, rat or mouse, or other primate, canine, feline, bovine, porcine, ovine, equine or rodent species.

Further included within the scope of the present invention is the use of metabolites or residues of the compounds of Formula (IA) or (IB) which possess biological activity such that they are able to inhibit PDE4 isozyme activity and consequent or associated pathogenic processes subsequently mediated thereby. Once synthesized, the PDE4 isozyme inhibiting activities and specificities of the compounds of Formula (IA) or (IB) according to the present invention may be determined using *in vitro* and *in vivo* assays which are described in detail further below.

The desirable biological activity of the compounds of Formula (IA) or (IB) may also be improved by appending thereto appropriate functionalities which will function to enhance existing biological properties of the compound, improve the selectivity of the compound for the existing biological activities, or add to the existing biological activities further desirable biological activities. Such modifications are known in the art and include those which increase biological penetration into a given biological system, *e.g.*, blood, the lymphatic system, and central nervous system; increase oral availability; increase solubility to allow administration by injection; alter metabolism; and alter the rate of excretion of the compound of Formula (IA) or (IB).

The amount of active ingredient that may be combined with the carrier materials to produce a single dosage form will vary depending upon the host treated, and the particular mode of administration. It should be understood, however, that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredient may also depend upon the therapeutic or prophylactic agent, if any, with which the active ingredient is co-administered.

The dosage and dose rate of the compounds of Formula (IA) or (IB) effective for treating or preventing diseases

and conditions in a patient, usually a mammal in need of such treatment, which are mediated by or associated with inhibition of PDE4 isozyme activity as described herein, as well as for favorably affecting the outcome thereof in said patient, in accordance with the methods of treatment of the present invention comprising administering to said patient a therapeutically effective amount of a compound of Formula (IA) or (IB), will depend on a variety of factors such as the nature of the active ingredient, the size of the patient, the goal of the treatment, the nature of the pathology being treated, the specific pharmaceutical composition used, the concurrent treatments that the patient may be subject to, and the observations and conclusions of the treating physician.

Generally, however, the effective therapeutic dose of a compound of Formula (IA) or (IB) which will be administered to a patient will be between about 10 μg (0.01 mg)/kg and about 60.0 mg/kg of body weight per day, preferably between about 100 μg (0.1 mg)/kg and about 10 mg/kg of body weight per day, more preferably between about 1.0 mg/kg and about 6.0 mg/kg of body weight per day, and most preferably between about 2.0 mg/kg and about 4.0 mg/kg of body weight per day of the active ingredient of Formula (I).

Where the dosage form is oral, *e.g.*, a tablet or capsule, suitable dosage levels of the compounds of Formula (I) will be between about 100 μg (0.1 mg)/kg and about 50 mg/kg body weight per day, preferably between about 1.0 mg/kg and about 10 mg/kg body weight per day, more preferably between about 1.5 mg/kg and about 5.0 mg/kg of body weight per day, and most preferably between about 2.0 mg/kg and about 3.5 mg/kg of body weight per day of the active ingredient of Formula (I).

Using representative body weights of 10 kg and 100 kg in order to illustrate the range of daily oral dosages which might be used as described above, suitable dosage levels of the compounds of Formula (I) will be between about 1.0 - 500 mg and 0.5 - 5.0 g per day, preferably between about 10.0 - 100 mg and 0.1 - 1 g per day, more preferably between about 15.0 - 40.0 mg and 150 - 400 mg per day, and most preferably between about 20.0 - 35.0 mg and about 200 - 350 mg per day of the active ingredient comprising a compound of Formula (I). These ranges of dosage amounts represent total dosage amounts of the active ingredient per day for a given patient. The number of times per day that a dose is administered will depend upon such pharmacological and pharmacokinetic factors as the half-life of the active ingredient, which reflects its rate of catabolism and clearance, as well as the minimal and optimal blood plasma or other body fluid levels of said active ingredient attained in the patient which are required for therapeutic efficacy

Included within the scope of the present invention are embodiments comprising coadministration of, and compositions which contain, in addition to a compound of the present invention as active ingredient, additional therapeutic agents and active ingredients. Such multiple drug regimens, often referred to as combination thereapy, may be used in the treatment and prevention of gastric stasis, especially gastric hypomotility and related disorders mediated by or associated with PDE4 isozyme inhbition, as described further below in the portion of the instant specification dealing with the methods of treatment of the present invention.

In addition to the requirement of therapeutic efficacy which may necessitate the use of active agents in addition to the PDE4 isozyme inhibiting compounds of Formula (IA) or (IB), there may be additional rationales which compel or highly recommend the use of combinations of drugs involving active ingredients which represent adjunct therapy, *i.e.*, which complement and supplement the function performed by the PDE4 isozyme inhibiting compounds of the present invention. Such supplementary therapeutic agents used for the purpose of auxiliary treatement comprise drugs which, instead of directly treating or preventing a disease or condition mediated by or associated with PDE4 isozyme inhibition, treat diseases or conditions which directly result from or indirectly accompany the basic or underlying gastric stasis, especially gastric hypomotility. For example, other active agents may be used with the compounds of Formula (IA) or (IB), *e.g.*, in order to treat pain and inflammation which accompany the initial and fundamental gastric stasis, especially gastric hypomotility.

Thus, the methods of treatment and pharmaceutical compositions of the present invention may employ the compounds of Formula (IA) or (IB) in the form of monotherapy, but said methods and compositions may also be used in the form of multiple therapy in which one or more compounds of Formula (IA) or (IB) are coadministered in combination with one or more known therapeutic agents such as those described in detail further herein. The terms "coadministered" or "coadministration" as used herein are intended to mean therapeutic utilization of one or more compounds of Formula (IA) or (IB) in combination with one or more additional therapeutic agents, including but not limited to, administration of the combination of therapeutic active agents in a single dosage form or in multiple dosage forms representing the same or different routes of administration, said multiple dosage forms being administered simultaneously, *i.e.,* at substantially the same time, or sequentially, *i.e.,* at different times.

In choosing therapeutic active agents which may be coadministered with the active ingredients comprising the compounds of Formula (IA) or (IB), the skilled artisan will take into account a number of factors. For example, the basic underlying disease or condition being treated must be considered, *e.g.*, whether the gastric stasis affects primarily the stomach or is more widespread, and whether there are any concomitant diseases, conditions or symptoms which might be present and treatable. Where there is a concomitant inflammatory condition present, the artisan would consider coadministration of other therapeutic agents, *e.g.*, anti-inflammatory agents. These do not represent inflexible

and mutually exclusive categories of usage, however, and circumstances may dictate a differnt usage or the utilization of altogether different types of therapeutic agents. It is within the skill of the artisan to make such choices.

Where conditions of inflammation are present in the patient being treated, an auxiliary therapeutic agent is optionally coadministered which comprises one or more members independently selected from the group consisting essentially of (1) anti-inflammatory corticosteroids for oral, injectable, topical, opthalmic, inhalation, or nasal administration useful in treating inflammatory conditions, preferably independently selected from alclometasone dipropionate; amcinonide; beclomethasone dipropionate; betamethasone; betamethasone benzoate; betamethasone dipropionate; betamethasone sodium phosphate; betamethasone sodium phosphate and acetate; betamethasone valerate; clobetasol propionate; clocortolone pivalate; cortisol; cortisol acetate; cortisol butyrate; cortisol cypionate; cortisol sodium phosphate; cortisol sodium succinate; cortisol valerate; cortisone acetate; desonide; desoximetasone; dexamethasone; dexamethasone acetate; dexamethasone sodium phosphate; diflorasone diacetate; fludrocortisone acetate; flunisolide; fluocinolone acetonide; fluocinonide; fluorometholone; flurandrenolide; halcinonide; medrysone; methylprednisolone; methylprednisolone acetate; methylprednisolone sodium succinate; mometasone furoate; paramethasone acetate; prednisolone; prednisolone acetate; prednisolone sodium phosphate; prednisolone tebutate; prednisone; triamcinolone; triamcinolone acetonide; triamcinolone diacetate; and triamcinolone hexacetonide; (2) non-steroidal analgesic, antipyretic and anti-inflammatory active agents preferably independently selected from (*i*) salicylic acid derivatives preferably consisting essentially of aspirin; sodium salicylate; methyl salicylate; choline magnesium trisalicylate; salsalate; diflunisal; salicylsalicylic acid; sulfasalazine; olsalazine; (*ii*) para-aminophenol derivatives preferably consisting essentially of acetaminophen; (*iii*) indole and indene acetic acids preferably consisting essentially of indomethacin; sulindac; and etodolac; (*iv*) heteroaryl acetic acids preferably consisting essentially of tolmetin; diclofenac; and ketorolac; (*v*) arylpropionic acids preferably consisting essentially of ibuprofen; naproxen; flurbiprofen; ketoprofen; fenoprofen; and oxaprozin; (*vi*) anthranilic acids preferably consisting essentially of mefenamic acid; meclofenamic acid; flufenamic acid; tolfenamic acid; and etofenamic acid; (*vii*) enolic acids preferably consisting essentially of meloxicam; piroxicam; and tenoxicam; (*viii*) pyrazolon derivatives preferably consisting essentially of phenylbutazone; and oxyphenthatrazone; (ix) alkanones preferably consisting essentially of nabumetone; (x) apazone; (xi) tenidap; and (xii) nimesulide; (3) potent opioid agonist analgesics preferably independently selected from alfentanil hydrochloride; anileridine; anileridine hydrochloride; brifentanil hydrochloride; carfentanil citrate; codeine; codeine phosphate; codeine sulfate; fentanyl citrate; hydromorphone hydrochloride; levomethadyl acetate; levomethadyl acetate hydrochloride; levorphanol tartrate; lofentanil oxalate; meperidine hydrochloride; methadone hydrochloride; methadyl acetate; morphine sulfate; ocfentanil hydrochloride; oxycodone; oxycodone hydrochloride; oxycodone terephthalate; oxymorphone hydrochloride; pentamorphone; and sufentanil citrate; (4) proteinaceous endogenous and synthetic opioid analgesics comprising enkephalins, endorphins, and dynorphins, which are selective and nonselective agonists and antagonists of the $\mu$, $\kappa$, and $\delta$ opioid receptor subtypes, preferably independently selected from [$Leu^5$] and [$Met^5$]enkephalin; dynorphin A and B; $\alpha$- and $\beta$-neoendorphin; [D-$Ala^2$,$MePhe^4$,-Gly(ol)$^5$]enkephalin (DAMGO); [D-$Pen^2$,D-$Pen^5$]enkephalin (DPDPE); [D-$Ser^2$,$Leu^5$]enkephalin-$Th^6$ (DSLET); [D-$Ala^2$,D-$Leu^5$]enkephalin (DADL); D-Phe-Cys-Tyr-D-Trp-Orn-Thr-Pen-Thr-$NH_2$ (CTOP); [D-$Ala^2$,N-$MePhe^4$,Met(O)$^5$-ol]enkephalin (FK-33824); Tyr-D-Ala-Phe-Asp-Val-Val-Gly-$NH_2$ ([D-$Ala^2$]deltorphin I; Tyr-D-Ala-Phe-Glu-Val-Val-Gly-$NH_2$ ([D-$Ala^2$,$Glu^4$]deltorphin II; Tyr-Pro-Phe-Pro-$NH_2$ (morphiceptin); Tyr-Pro-MePhe-D-Pro-$NH_2$ (PL-017); and [D-$Ala^2$,$Leu^5$,$Cys^6$]enkephalin; (5) leukotriene antagonists preferably independently selected from ablukast; ablukast sodium; cinalukast; iralukast; montelukast sodium; ontazolast; pobilukast edamine; pranlukast; ritolukast; sulukast; tomelukast; verlukast; and zafirlukast; (6) leukotriene biosynthesis (5-lipoxygenase) inhibitors preferably independently selected from docebenone; enazadrem phosphate; and zileuton; (7) thromboxane receptor antagonists preferably independently selected from seratrodast; (8) anticholinergic agents preferably independently selected from ipratropium bromide; (9) autocoids including bradykinin and kallidin, and their analogous derivatives having agonist and antagonist activity useful for the treatment of pain and chronic inflammatory conditions, preferably independently selected from Arg-Pro-Pro-Gly-Phe-Ser-Pro-Phe-Arg (bradykinin); Lys-Arg-Pro-Pro-Gly-Phe-Ser-Pro-Phe-Arg (kallidin); Arg-Pro-Pro-Gly-Phe-Ser-Pro-Phe (des-$Arg^9$-bradykinin); Lys-Arg-Pro-Pro-Gly-Phe-Ser-Pro-Phe (des-$Arg^{10}$-kallidin); Arg-Pro-Pro-Gly-Phe-Ser-Pro-Leu (des-$Arg^9$-[$Leu^8$]-bradykinin); Arg-Pro-Pro-Gly-Phe-Ser-[D-Phe]-Phe-Arg ([D-$Phe^7$]-bradykinin); and [D-Arg]-Arg-Pro-Hyp-Gly-Thi-Ser-Tic-Oic-Arg (HOE 140), where Hyp is *trans*-4-hydroxy-Pro; Thi is $\beta$-(2-thienyl)-Ala; Tic is [D]-1,2,3,4-tetrahydroquinolin-3-yl-carbonyl; and Oic is (3as, 7as)-octahydroindol-2-yl-carbonyl; and (10) cytokines consisting of colony-stimulating factors and interleukins preferably independently selected from granulocyte colony-stimulating factor (G-CSF); granulocyte macrophage colony-stimulating factor (GM-CSF); and interleukin-1 (IL-1) through interleukin-12 (IL-12).

As also alluded to further above, the compounds of Formula (IA) or (IB) may be formulated into pharmaceutical compositions that may be administered orally, parenterally, by inhalation (metered dose inhaler, dry powder inhaler or nebulizer), topically, rectally, nasally, intraocularly, buccally, vaginally or via an implanted reservoir. The term "parenteral" as used herein is intended to include subcutaneous, intravenous, intramuscular, intra-articular, intra-synovial, intrasternal, intrathecal, intrahepatic, intralesional and intracranial injection or infusion techniques.

The present invention includes not only the above-described pharmaceutical compositions, but methods of

treatment as well in which said pharmaceutical compositions are administered to a patient in need of treatment. Such embodiments of the present invention relate to a method of treating or preventing gastric stasis, especially gastric hypomotility, by inhibiting PDE4 isozyme activity and consequent or associated pathogenic processes subsequently mediated by the PDE4 isozyme.

In the above-described preferred embodiment combinations of the present invention, the compound of Formula (IA) or (IB) and other therapeutic active agents may be administered in terms of dosage forms either separately or in conjunction with each other, and in terms of their time of administration, either serially or simultaneously. Thus, the administration of one component agent may be prior to, concurrent with, or subsequent to the administration of the other component agent(s).

The compounds of Formula (IA) or (IB) may be administered in accordance with a regimen of 1 to 4 times per day, preferably once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The ability of the compounds of Formula (IA) or (IB) or the pharmaceutically acceptable salts thereof to inhibit PDE4 may be determined by the assay detailed in the following paragraphs.

Thirty to forty grams of human lung tissue is placed in 50 ml of pH 7.4 Tris/phenylmethylsulfonyl fluoride (PMSF)/sucrose buffer and homogenized using a Tekmar Tissumizer® (Tekmar Co., 7143 Kemper Road, Cincinnati, Ohio 45249) at full speed for 30 seconds. The homogenate is centrifuged at 48,000 x g for 70 minutes at 4°C. The supernatant is filtered twice through a 0.22 mm filter and applied to a Mono-Q FPLC column (Pharmacia LKB Biotechnology, 800 Centennial Avenue, Piscataway, New Jersey 08854) pre-equilibrated with pH 7.4 Tris/PMSF Buffer. A flow rate of 1 ml/minute is used to apply the sample to the column, followed by a 2 ml/minute flow rate for subsequent washing and elution. Sample is eluted using an increasing, step-wise NaCl gradient in the pH 7.4 Tris/PMSF buffer. Eight ml fractions are collected. Fractions are assayed for specific PDE4 activity determined by [$^3$H]cAMP hydrolysis and the ability of a known PDE4 inhibitor (*e.g.* rolipram) to inhibit that hydrolysis. Appropriate fractions are pooled, diluted with ethylene glycol (2 ml ethylene glycol/5 ml of enzyme prep) and stored at -20°C until use.

Compounds are dissolved in dimethylsulfoxide (DMSO) at a concentration of 10 mM and diluted 1:25 in water (400 mM compound, 4% DMSO). Further serial dilutions are made in 4% DMSO to achieve desired concentrations. The final DMSO concentration in the assay tube is 1%. In duplicate the following are added, in order, to a 12 × 75 mm glass tube (all concentrations are given as the final concentrations in the assay tube).

i) 25 ml compound or DMSO (1%, for control and blank)

ii) 25 ml pH 7.5 Tris buffer

iii) [$^3$H]cAMP (1 mM)

iv) 25 ml PDE4 enzyme (for blank, enzyme is preincubated in boiling water for 5 minutes)

The reaction tubes are shaken and placed in a water bath (37°C) for 20 minutes, at which time the reaction is stopped by placing the tubes in a boiling water bath for 4 minutes. Washing buffer (0.5 ml, 0.1M 4-(2-hydroxyethyl)-1-piperazine-ethanesulfonic acid (HEPES)/0.1M naci, pH 8.5) is added to each tube on an ice bath. The contents of each tube are applied to an AFF-Gel 601 column (Biorad Laboratories, P.O. Box 1229, 85A Marcus Drive, Melvile, New York 11747) (boronate affinity gel, 1 ml bed volume) previously equilibrated with washing buffer. [$^3$H]cAMP is washed with 2 x 6 ml washing buffer, and [$^3$H]5'AMP is then eluted with 4 ml of 0.25M acetic acid. After vortexing, 1 ml of the elution is added to 3 ml scintillation fluid in a suitable vial, vortexed and counted for [$^3$H].

$$\% \text{ inhibition} = 1 - \frac{\text{average cpm (test compound - average cmp (blank)}}{\text{average cpm (control) - average cpm (blank)}}$$

$IC_{50}$ is defined as that concentration of compound which inhibits 50% of specific hydrolysis of [$^3$H]cAMP to [$^3$H] 5'AMP.

The compounds of Formula (IA) or (IB), useful in the therapeutic methods of treatment and pharmaceutical compositions of the present invention, may be prepared in accordance with methods known in the art. For example, reaction Schemes 1-4 below illustrate suitable methods for preparing the compounds of Formula (IA) or (IB):

SCHEME 1

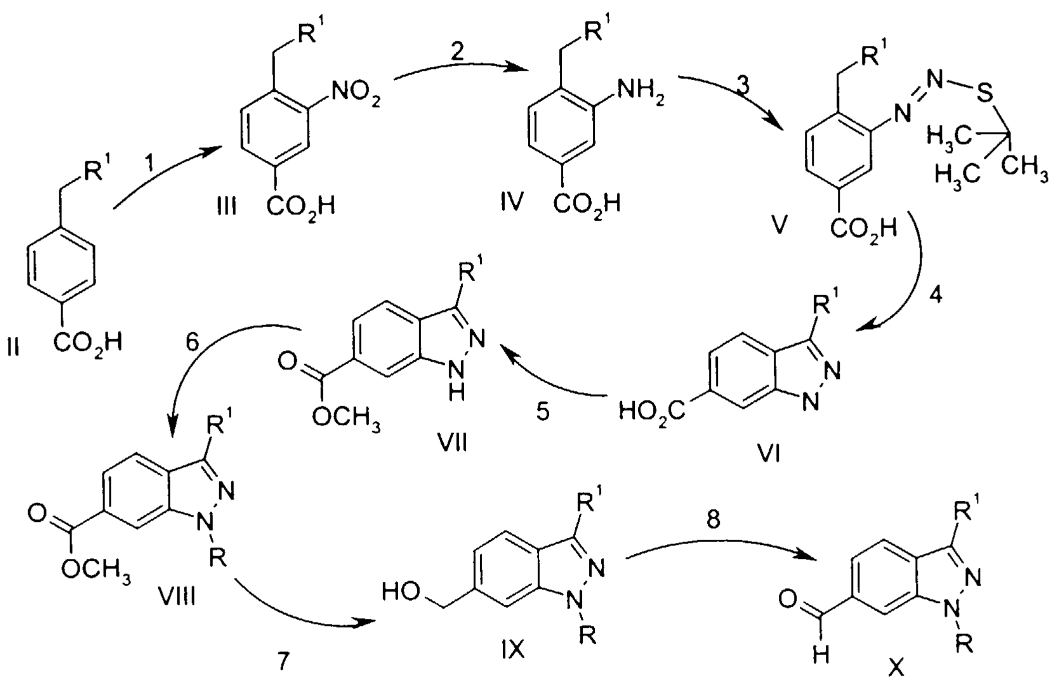


SCHEME 2

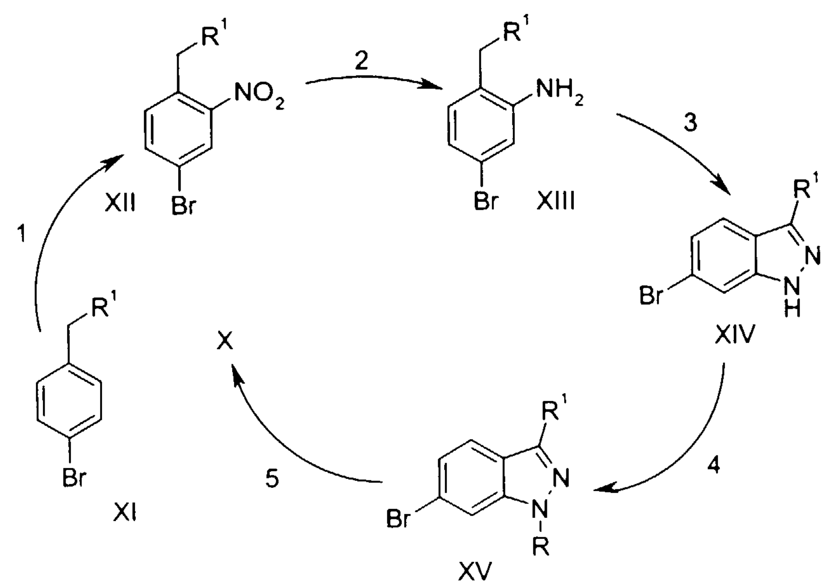

SCHEME 3

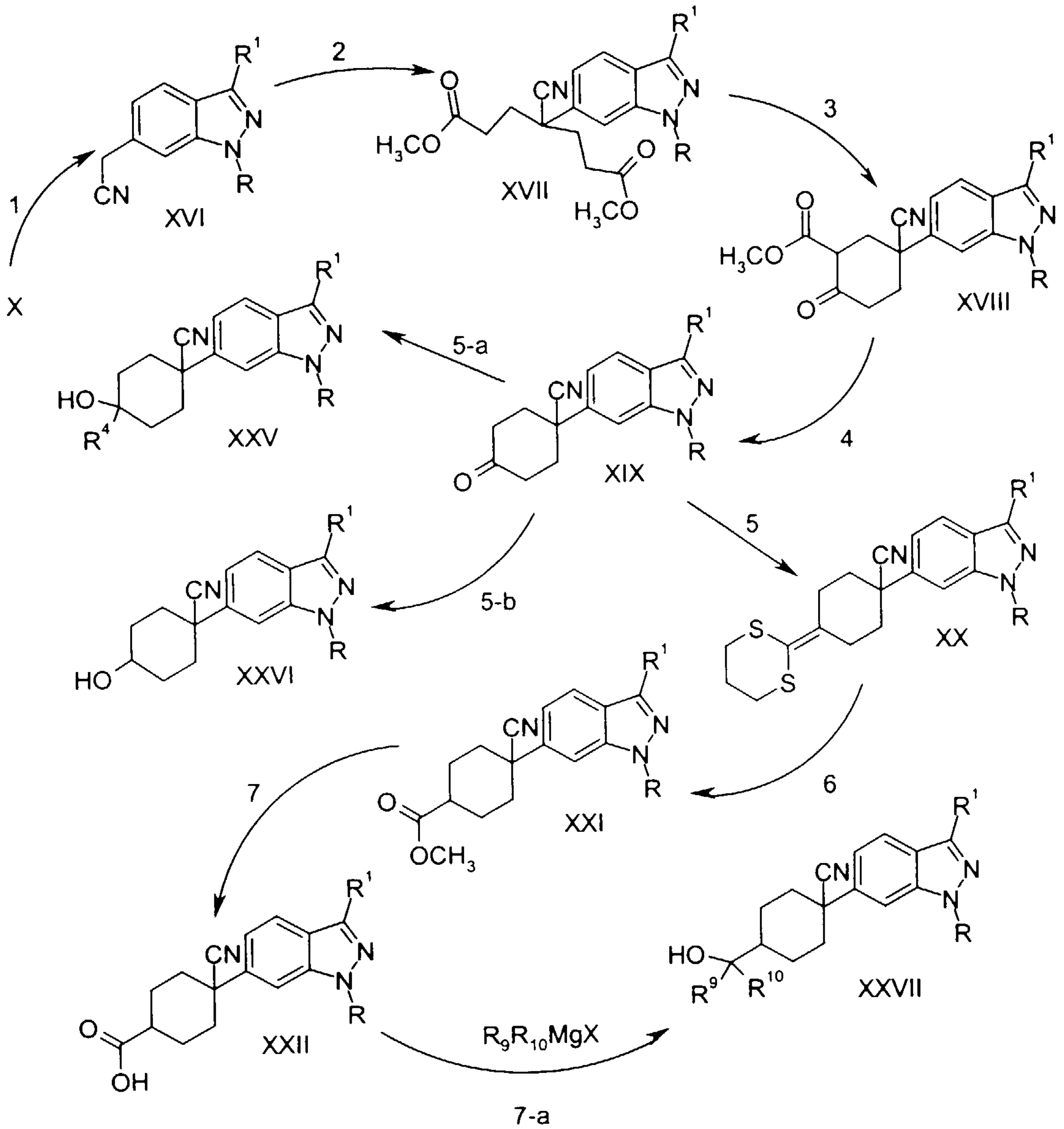


SCHEME 4

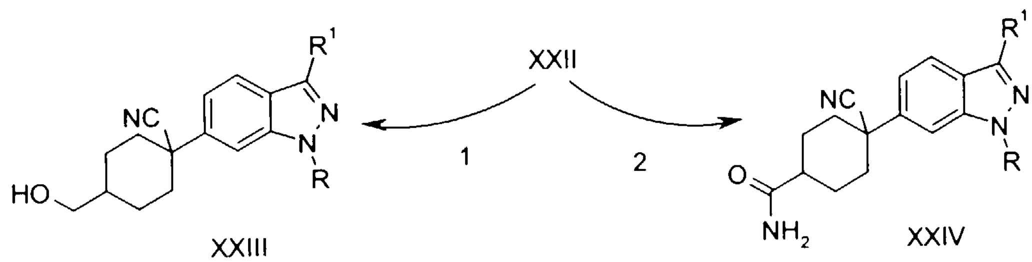


The compounds in the schematic representations above are numbered using Roman numerals in consecutive order, starting with II. These Roman numeral reference numbers are not necessarily related to the Roman numerals

used elsewhere in defining the compounds of the present invention. Unless otherwise indicated, R and $R^1$ in the reaction schemes are defined the same as elsewhere herein.

The preparation of compounds of Formula (IA) or (IB) can be carried out by one skilled in the art according to one or more of the synthetic methods outlined in Schemes 1-4 above. In Step 1 of Scheme 1, the carboxylic acid of Formula II, which is available from known commercial sources or can be prepared according to methods known to those skilled in the art, is nitrated under standard conditions of nitration ($HNO_3/H_2SO_4$, 0°C) and the resulting nitro derivative of Formula III is hydrogenated in Step 2 of Scheme 1 using standard hydrogenation methods ($H_2$-Pd/C under pressure) at ambient temperature (20-25°C) for several hours (2-10 hours) to provide the compound of Formula IV. In Step 3 of Scheme 1, the amino benzoic acid of Formula IV is reacted with a base such as sodium carbonate under aqueous conditions and gently heated until mostly dissolved. The reaction mixture is chilled to a lower temperature (about 0°C) and treated with sodium nitrate in water. After about 15 minutes, the reaction mixture is slowly transferred to an appropriate container holding crushed ice and a strong acid such as hydrochloric acid. The reaction mixture is stirred for 10-20 minutes and then added, at ambient temperature, to a solution of excess *tert*-butyl thiol in an aprotic solvent such as ethanol. The reaction mixture is acidified to a pH of 4-5 through addition of an inorganic base, preferably saturated aqueous $Na_2CO_3$, and the reaction mixture is allowed to stir at ambient temperature for 1-3 hours. Addition of brine to the reaction mixture, followed by filtration, provides the sulfide of Formula V.

In Step 4 of Scheme 1, the sulfide of Formula V is converted to the corresponding indazole carboxylic acid of Formula VI by reacting the sulfide of Formula V with a strong base, preferably potassium tert-butoxide, in dimethyl sulfoxide (DMSO) at ambient temperature. After stirring for several hours (1-4 hours), the reaction mixture is acidified with a strong acid, such as hydrochloric or sulfuric acid, and then extracted using conventional methods. In Step 5 of Scheme 1, the indazole carboxylic acid of Formula VI is converted to the corresponding ester of Formula VII by conventional methods known to those skilled in the art. In Step 6 of Scheme 1, the compound of Formula VIII is provided through alkylation of the ester of Formula VII by subjecting the ester to conventional alkylation conditions (strong base/various alkylating agents and, optionally, a copper catalyst such as $CuBr_2$) in a polar aprotic solvent, such as tetrahydrofuran (THF), N-methylpyrrolidinone or dimethylformamide (DMF), at ambient or higher temperature (25-200°C) for about 6-24 hrs, preferably about 12 hours. In Step 7 of Scheme 1, the compound of Formula VIII is converted to the corresponding alcohol of IX by following conventional methods known to those skilled in the art for reducing esters to alcohols. Preferably, the reduction is effected through use of a metal hydride reducing agent, such as lithium aluminum hydride, in a polar aproptic solvent at a low temperature (about 0°C). In Step 8 of Scheme 1, the alcohol of Formula IX is oxidized to the corresponding aldehyde of Formula X according to conventional methods known to those skilled in the art. For example, the oxidation can be effected through use of a catalytic amount of tetrapropylammonium perrutenate and excess N-methylmorpholine-N-oxide, as described in *J. Chem. Soc., Chem. Commun.,* 1625 (1987), in an anhydrous solvent, preferably methylene chloride.

Scheme 2 provides an alternative method of preparing the aldehyde of Formula X. In Step 1 of Scheme 2, the compound of Formula XI is nitrated using conventional nitration conditions (nitric and sulfuric acid) to provide the compound of Formula XII. In Step 2 of Scheme 2, the nitro derivative of Formula XII is reduced to the corresponding amine of Formula XIII according to conventional methods known to those skilled in the art. Preferably, the compound of Formula XII is reduced to the amine of Formula XIII using anhydrous stannous chloride in an anhydrous aprotic solvent such as ethanol. In Step 3 of Scheme 2, the amine of Formula XIII is converted to the corresponding indazole of Formula XIV by preparing the corresponding diazonium fluoroforates as described in A. Roe, *Organic Reactions,* Vol. 5, Wiley, New York, 1949, pp. 198-206, followed by phase transfer catalyzed cyclization as described in R. A. Bartsch and I. W. Yang, *J. Het. Chem.* 21, 1063 (1984). In Step 4 of Scheme 2, alkylation of the compound of Formula XIV is performed using standard methods known to those skilled in the art, *e.g.*, strong base, polar aprotic solvent and an alkyl halide, to provide the N-alkylated compound of Formula XV. In Step 5 of Scheme 2, the compound of Formula XV is subjected to metal halogen exchange employing an alkyl lithium, such as *n*-butyl lithium, in a polar aprotic solvent, such as THF, at low temperature (-50°C to 100°C, with -78°C being preferred) followed by quenching with DMF at low temperature and warming to ambient temperature to provide the aldehyde compound of Formula X.

Scheme 3 illustrates the preparation of a compound of Formula XXII which is a compound of Formula (IA) or (IB) wherein $R^2{}_a$ or $R^2{}_b$ is a ring moiety of Formula (1.0.0). In Step 1 of Scheme 3, the aldehyde moiety of the compound of Formula X is converted to an appropriate leaving group, such as a halogen, mesylate or another leaving group familiar to those skilled in the art, followed by reacting the resulting compound with sodium cyanate in a polar solvent such as DMF to provide the compound of Formula XVI. In Step 2 of Scheme 3, the compound of Formula XVI is reacted under basic conditions with methyl acrylate or related derivatives depending on the $R^2{}_a$ or $R^2{}_b$ group to be added, in an aprotic solvent such as ethylene glycol dimethyl ether (DME) at high temperature, preferably at reflux, to provide the compound of Formula XVII. In Step 3 of Scheme 3, the compound of Formula XVII is converted to the compound of Formula XVIII using a strong base, such as sodium hydride, and a polar aprotic solvent, such as DMF or THF, at elevated temperature, preferably at reflux.

In Step 4 of Scheme 3, the compound of Formula XVIII is decarboxylated using conventional methods, such

as using sodium chloride in DMSO at a temperature of about 140°C, to provide the compound of Formula XIX. In Step 5 of Scheme 3, derivatization of the compound of Formula XIX to the corresponding dithian-2-ylidine cyclohexane carbonitrile of Formula XX is done by reaction with 2-lithio-1,3-dithiane. In Step 5-a of Scheme 3, further derivatization of the compound of Formula XIX to the corresponding cyclohexane carbonitrile of Formula XXV which is para-substituted on the cyclohexane group wth an hydroxyl moiety and an $R^4$ substituent, *e.g.,* methyl, is carried out by reacting the ketone with a nucleophilic reagent, *e.g.*, an alkyllithium compound or a Grignard reagent in accordance with procedures well known in the art. In Step 5-b of Scheme 3, further derivatization of the compound of Formula XIX to the corresponding cyclohexane carbonitrile of Formula XXVI which is para-substituted on the cyclohexane group with an hydroxyl moiety, is carried out by reducing the ketone with, *e.g.*, lithium aluminum hydride or sodium borohydride in accordance with procedures well known in the art. In Step 6 of Scheme 3, the compound of Formula XX is converted to the corresponding ester of Formula XXI using mercury (II) chloride and perchloric acid in a polar protic solvent such as methanol. In Step 7 of Scheme 3, the compound of Formula XXI is converted through hydrolysis to the corresponding carboxylic acid of Formula XXII using a standard method of hydrolysis, such as using aqueous sodium hydroxide in a polar solvent, or any of numerous existing hydrolysis methods known to those skilled in art as described in T. Green and P.G.M. Wets, *Protecting Groups in Organic Synthesis,* 2nd Edition, John Wiley and Sons, New York (1991). The synthetic steps described for Scheme 3 are analogous to the synthetic methods provided for the preparation of corresponding catechol-containing compounds in PCT published applications WO 93/19751 and WO 93/17949.

Other compounds of Formula (IA) or (IB) wherein one of $R^2_a$ or $R^2_b$ is selected from moieties (1.0.0), (1.0.1), (1.0.2) and (1.0.3), can be prepared from one or more of the intermediate compounds described in Schemes 1-3. In particular, the aldehyde of Formula X or the keto compound of Formula XIX can be used to prepare various compounds of Formula (IA) or (IB). Any of the various $R^2_a$ or $R^2_b$ moieties of formulas (1.0.0), (1.0.1), (1.0.2) or (1.0.3) can be introduced into one or more of the intermediate compounds referred to above using synthetic methods provided for corresponding non-indazole analogs in PCT published applications WO 93/19748, WO 93/19749, WO 93/09751, WO 93/19720, WO 93/19750, WO 95/03794, WO 95/09623, WO 95/09624, WO 95/09627, WO 95/09836, and WO 95/09837. For example, with reference to Step 1 of Scheme 4, the carboxylic acid of Formula XXII can be converted to the alcohol of Formula XXIII by reduction with various metal hydrides in a polar solvent as described in Example 9, referred to below, and in accordance with synthetic methods provided for corresponding non-indazole analogs in PCT published applications publication numbers WO 93/19747, WO 93/19749 and WO 95/09836. Further, with reference to Step 2 of Scheme 4, the carboxylic acid of Formula XXII can be converted to the corresponding carboxamide of Formula XXIV through conversion to an intermediate acid chloride using conventional synthetic methods, and then reacting the acid chloride with ammonia in an aprotic solvent. Other carboxamide analogs of Formula XXIV can be prepared through reaction of the acid chloride intermediate with various primary or secondary amines according to conventional methods known to those skilled in the art and as described in the PCT published applications referred to above.

Other compounds of Formula (IA) or (IB) can be prepared from the intermediate compound of Formula XIX in accord with synthetic methods provided for corresponding non-indazole analogs in the PCT published applications referred to above. Compounds of Formula (IA) or (IB) wherein $R^2_a$ or $R^2_b$ is a moiety of partial Formula (1.0.0), and either $R^{114}$ ($R^4$) or R is H, can be prepared from the keto intermediate of Formula XIX by reaction with a base such as lithium diisopropylamine in a polar aprotic solvent, such as THF, and excess N-phenyltrifluoromethylsulfonamide as described in PCT published application WO 93/19749 for corresponding non-indazole analogs. Compounds of Formula (IA) or (IB) wherein $R^2_a$ or $R^2_b$ is a moiety of partial Formula (1.0.0), $R^{114}$ ($R^4$) is hydrogen, and $R^{115}$ ($R^5$) is $-CO_2CH_3$ or $-CO_2H$, can be prepared from the keto intermediate of Formula XIX through reaction with triflic anhydride in the presence of a tertiary amine base followed by reaction of the resulting triflate with (triphenylphosphine)palladium and carbon monoxide in the presence of an alcohol or amine to provide the methyl ester compounds of Formula (IA) or (IB) wherein $R^{115}$ ($R^5$) is $-CO_2CH_3$. The methyl ester compound can be hydrolyzed to obtain the corresponding carboxylic acid compound by employing standard methods for hydrolysis such as sodium or potassium hydroxide in aqueous methanol/tetrahydrofuran. Such synthetic methods are further described in PCT published application WO 93/19749 for corresponding non-indazole analogs.

Other compounds of Formula (IA) or (IB) can be prepared from the intermediate compound of Formula XIX in accord with synthetic methods described for corresponding non-indazole analogs in the published PCT applications referred to above. Compounds of Formula (IA) or (IB) wherein $R^2_a$ or $R^2_b$ is a moiety of partial Formula (1.0.0), $R^{115}$ ($R^5$) is hydrogen, and $R^{114}$($R^4$) is hydroxy, can be prepared through reaction of the intermediate of Formula XIX with an appropriate reducing agent such as lithium borohydride, diamyl borane, lithium aluminum tris(tert-butoxide), or sodium borohydride in a suitable non-reacting solvent such as 1,2-dimethoxy ethane, THF or alcohol. Compounds of Formula (IA) or (IB) wherein $R^2_a$ or $R^2_b$ is a moiety of Formula (1.0.0), $R^{115}$ ($R^5$) is hydrogen and $R^{114}$ ($R^4$) is $-NH_2$, $-NHCH_3$, or $-N(CH_3)_2$, can be prepared by reacting the intermediate of Formula XIX with an ammonium salt, such as ammonium formate, methylamine hydrochloride or dimethylamine hydrochloride, in the presence of sodium cyanoborohydride in an appropriate solvent such as alcohol.

Alternatively, compounds of Formula (IA) or (IB) wherein $R^2_a$ or $R^2_b$ is a moiety of Formula (1.0.0), $R^{114}$ ($R^4$) is amino, and $R^{115}$ ($R^5$) is hydrogen, can be prepared by reacting the corresponding alcohol of Formula (IA) or (IB) where $R^{114}$ ($R^4$) = OH and $R^{115}$ ($R^5$) = H, with a complex of an azadicarboxylate ester in the presence of an imide or phthalimide followed by reaction in an alcoholic solvent such as ethanol. Compounds of Formula (IA) or (IB) wherein $R^2_a$ or $R^2_b$ is a moiety of Formula (1.0.0), $R^{115}$ ($R^5$) is H, and $R^{114}$ ($R^4$) is $-SR^{124}$ can be prepared by reacting the corresponding compound wherein $R^{114}$ ($R^4$) is a leaving group such as mesylate, tosylate, bromine or chlorine, with a metal salt of mercaptan such as $NaSR^{124}$ in an appropriate aprotic solvent. Corresponding compounds of Formula (IA) or (IB) wherein $R^{114}$ ($R^4$) is -SH can be prepared by reacting the corresponding alcohol $R^{114}$ ($R^4$) = OH, with a complex of a phosphine, such as triphenyl phosphine, and an azidocarboxylate ester in the presence of thiolacetic acid followed by hydrolysis of the resulting thiolacetate. Furthermore, compounds of this structure wherein $R^{114}$ ($R^4$) is hydroxy can be interconverted using a standard alcohol inversion procedure known to those skilled in the art. The foregoing compounds of Formula (IA) or (IB) wherein $R^2_a$ or $R^2_b$ is a moiety of Formula (1.0.0), $R^{115}$ ($R^5$) is hydrogen, and $R^{114}$ ($R^4$) is hydroxy, -SH or $-NH_2$, can be converted to various other compounds of Formula (IA) or (IB) through one or more synthetic methods described in PCT published applications WO 93/19751 and WO 93/19749 for corresponding non-indazole analogs.

Compounds of Formula (IA) or (IB) wherein $R^2_a$ or $R^2_b$ is a moiety of Formula (1.0.0) and the dashed line represents a double bond attached to the ring carbon atom to which substituent $R^{113}$ ($R^3$) is attached, can be prepared from the intermediate of Formula XIX by following one or more synthetic methods provided for the preparation of corresponding non-indazole analogs in PCT published application WO 93/19720. Compounds of Formula (IA) or (IB) wherein $R^2_a$ or $R^2_b$ is a moiety of Formula (1.0.0), and $R^{114}$ ($R^4$) and $R^{115}$ ($R^5$) are taken together to form =O or $=R^{118}$, wherein $R^{118}$ is as defined above, can be prepared from the corresponding ketone intermediate of formula XIX following one or more synthetic methods provided for corresponding non-indazole analogs in PCT published application WO 93/19750. Other compounds of Formula (IA) or (IB) wherein $R^2_a$ or $R^2_b$ is a moiety of Formula (1.0.0) and $R^{114}$ ($R^4$) and $R^{115}$ ($R^5$) are taken together as $=R^{118}$ can be prepared from the intermediate of Formula XIX following one or more synthetic methods provided for the preparation of corresponding non-indazole analogs in PCT published application WO 93/19748.

Compounds of Formula (IA) or (IB) wherein $R^2_a$ or $R^2_b$ is a moiety of Formula (1.0.1) can be prepared from one or more of the intermediates referred to above, such as the bromoindazole intermediate of Formula XV, following one or more synthetic methods provided for the preparation of corresponding non-indazole analogs in PCT published applications WO 95/09627, WO 95/09624, WO 95/09623, WO 95/09836 and WO 95/03794. Compounds of Formula (IA) or (IB) wherein $R^2_a$ or $R^2_b$ is a moiety of Formula (1.0.2) can be prepared from the intermediate of Formula XV following one or more of synthetic methods provided for the preparation of corresponding non-indazole analogs in PCT published applications WO 95/09624 and WO 95/09837. Compounds of Formula (IA) or (IB) wherein $R^2_a$ or $R^2_b$ is a moiety of Formula (1.0.3) can be prepared from the bromoindazole intermediate of Formula XV employing one or more synthetic methods provided for the preparation of the corresponding catechol-containing analogs in PCT published applications WO 95/09627, WO 95/09623 and WO 95/09624.

Preferred compounds of Formula (IA) or (IB) are those represented by Formulas (1.3.0) and (1.4.0):

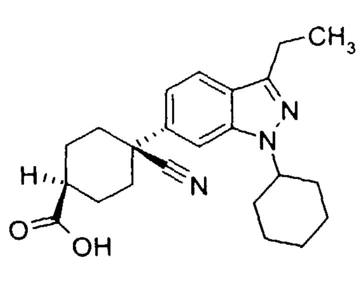

and

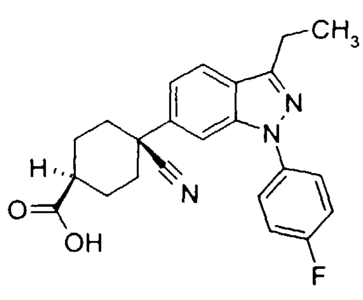

(1.3.0) (1.4.0)

A method for the preparation of the compound of Formula (1.3.0) may be carried out in accordance with the synthesis method described in above-depicted Scheme 2 and Scheme 3, using as the starting material for said method the compound represented by Formula (XV-*i*):

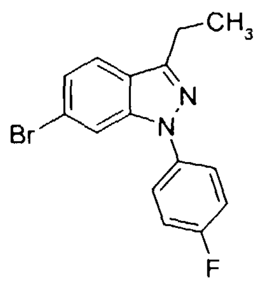

(XV-*i*)

The preferred compound depicted in Formula (1.3.0) above may be prepared in accordance with the synthesis methods described in above-depicted Scheme 1, Scheme 2, and Scheme 3. Another, preferred, method of preparing said compound may also be employed, and is represented in the following synthesis Scheme 5, which is a more generalized representation of the above-mentioned preferred method of preparing said above-described preferred compound.

SCHEME 5

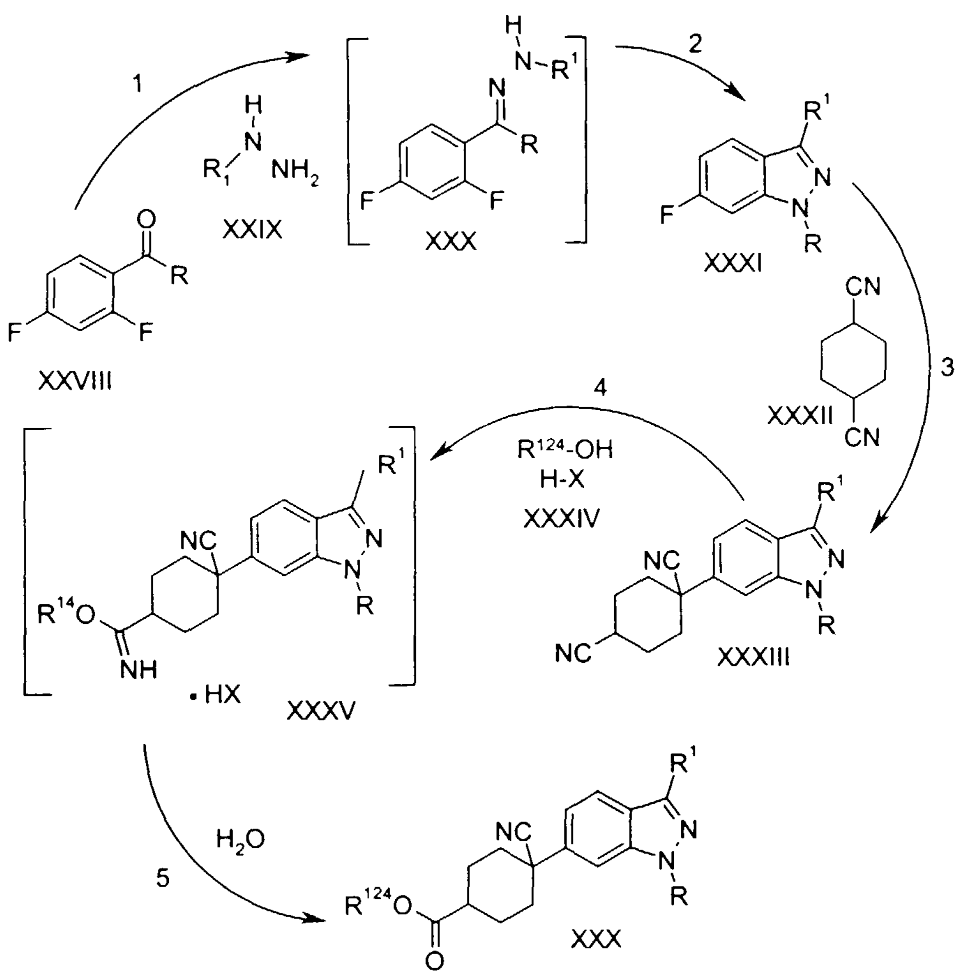


As illustrated, the starting material of Formula XXVIII is reacted with a hydrazine of Formula XXIX and the *in situ* product of Formula XXX is heated without separation to yield an indazole of Formula XXXI, which is in turn reacted with dicyanocyclohexane of Formula XXXII to yield the cyano- analog of said above-described preferred compound of Formula XXXIII.

In Step 1 of Scheme 5, the compound of Formula XXVIII is treated with a hydrazine derivative of Formula XXIX and an acid, preferably ammonium acetate, in a solvent such as heptane, tetrahydrofuran, xylenes, toluene, or mesitylene, or a mixture of two or more of the foregoing solvents, preferably toluene, to provide the compound of Formula XXX. In general, the compound of Formula XXX need not be separated or isolated from the reaction mixture.

In Step 2 of Scheme 5, the reaction mixture containing the compound of Formula XXX is heated at a temperature between about 75 °C and about 200 °C, preferably between about 90 ° and 120 °C, for a period of about 2 hours to 48 hours, preferably 12 hours, to provide the compound of Formula XXXI.

Alternatively, the process of Step 1 of Scheme 5 may be accomplished using a salt of the hydrazine derivative, such as the hydrochloride, hydrobromide, mesylate, tosylate, or oxalate salt of said compound, preferably the mesylate salt, which is reacted with a base, such as sodium or potassium acetate, in a solvent such as heptane, tetrahydrofuran, xylenes, toluene, or mesitylene, or a mixture of two or more of the foregoing solvents, preferably toluene.

In Step 3 of Scheme 5, the compound of Formula XXXI is treated with the compound of Formula XXXII in the presence of a base such as lithium bis(trimethylsilyl)amide, sodium bis(trimethylsilyl)amide, potassium bis(trimethylsilyl)amide, lithium diisopropylamide, or lithium 2,2,6,6-tetramethylpiperidine, preferably potassium bis(trimethylsilyl) amide, in a solvent such as tetrahydrofuran, toluene, or xylenes, preferably toluene, at a temperature between about 25 °C and about 125 °C, preferably about 100 °C, for a period 1 hour to 15 hours, preferably 5 hours, to provide compound of Formula XXXIII.

In Step 4 of Scheme 5, the compound of Formula XXXIII is treated with an acid such as hydrochloric acid, hydrobromic acid, sulfuric acid, *p*-toluenesulfonic acid, methanesulfonic acid, or trifluoromthanesulfonic acid, preferably hydrochloric acid, in a solvent of the Formula XXXIV, *i.e.,* $R^{124}$-OH wherein $R^{124}$ is as defined herein, *e.g.*, ($C_1$-$C_6$) alkyl, such as methanol, ethanol, propanol, isopropanol, preferably ethanol, at a temperature between 0 °C and 50 °C, preferably ambient temperature (20-25 °C) for a period of 1 hour to 48 hours, preferably 14 hours, to provide a compound of Formula XXXV. In general, the compound of Formula XXXV need not to be separated or isolated from the reaction mixture.

In step 5 of Scheme 5, the compound of Formula XXXV is treated with water in a solvent such as toluene, ethyl acetate, diisopropyl ether, methyl tert-butyl ether, or dichloromethane, preferably toluene, at a temperature between about 0 °C and 50 °C, preferably ambient temperature (20-25 °C) for a period of 1 hour to 24 hours, preferably 8 hours, to provide a compound of Formula XXXVI.

A particular version of the synthesis of Scheme 5 above carried out with reactants suitable for obtaining the preferred cyclohexanecarboxylic acid compound of the present invention, is illustrated below in Scheme 6:

SCHEME 6

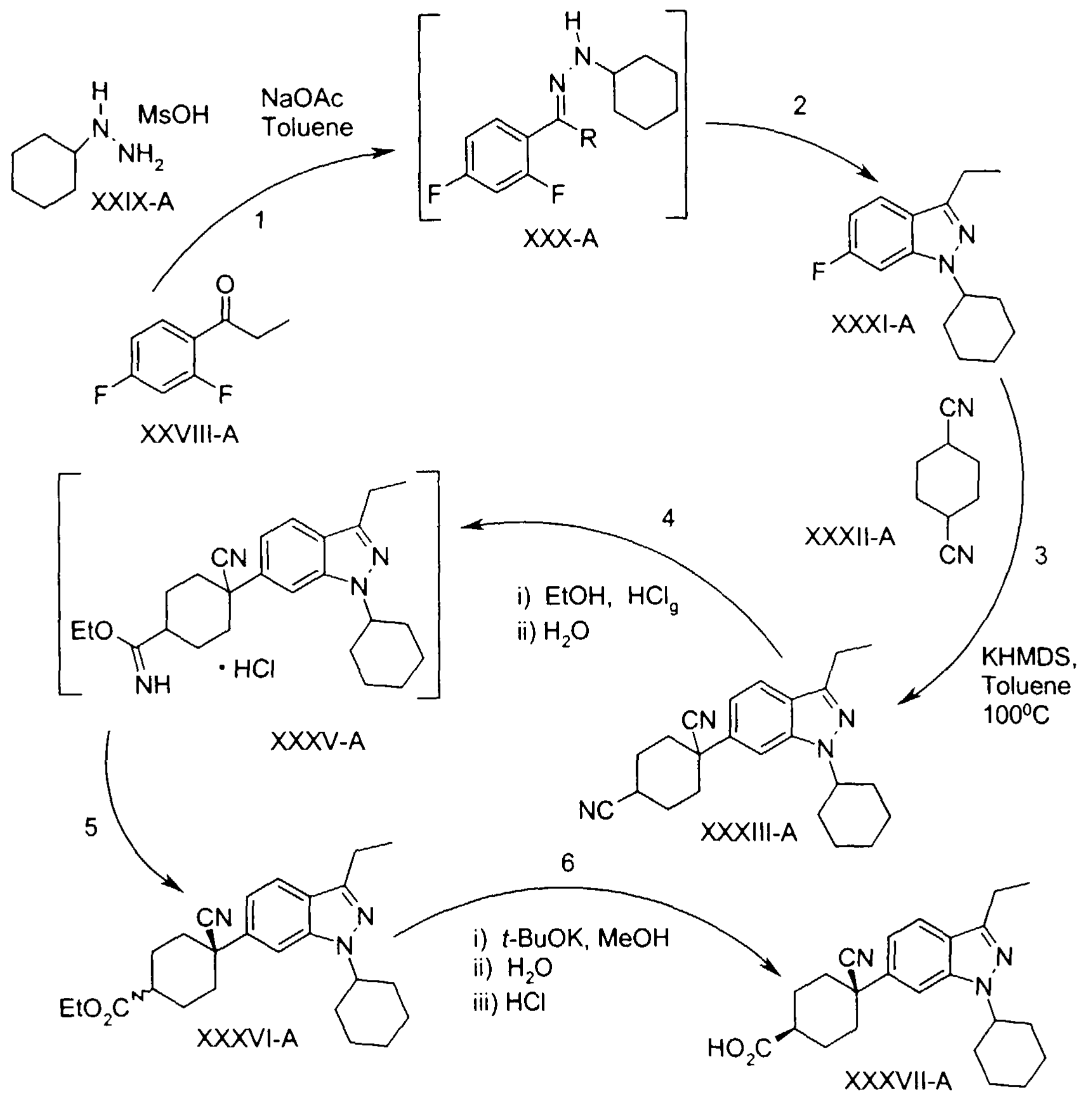


Scheme 7 set out below illustrates a procedure to facilitate the handling and purification of the indazole intermediate of Formula XXXI which is described above in reference to Scheme 5. In Step 1 of Scheme 7, the indazole of Formula XXXI is treated with an acid, such as hydrobromic, hydrochloric, or sulfuric acid, preferably hydrobromic acid, in a solvent such as toluene, xylenes, acetic acid, or ethyl acetate, preferably toluene, at a temperature ranging from 0 °C to ambient temperature (20-25 °C), preferably ambient temperature, to form a salt of the compound of Formula XXXVIII, wherein HX indicates the acid used to prepare the salt and X is the anion of said acid. The salt may be separated and purified according to methods familiar to those skilled in the art. In Step 2 of Scheme 7, the salt is converted back to the free base. In this step, the salt of the compound of Formula XXXVIII is treated with an aqueous base, such as sodium hydroxide, potassium hydroxide, sodium carbonate, sodium bicarbonate, potassium carbonate, or potassium bicarbonate, preferably sodium hydroxide, in a solvent such as hexane, toluene, dichloromethane, diisopropyl ether, methyl *tert*-butyl ether, or ethyl acetate, preferably toluene, at a temperature ranging from 0 °C to ambient temperature (20-25 °C), preferably ambient temperature, for a period of 5 minutes to 1 hour, preferably 20 minutes, to provide the compound of Formula XXXI.

SCHEME 7

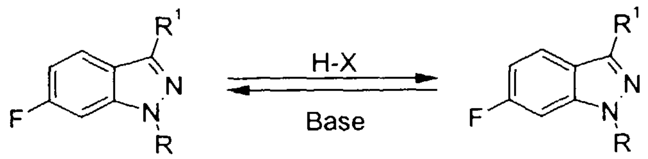


The compounds of the Formulas XXVIII through XXXVIII may have asymmetric carbon atoms and therefore exist in different enantiomeric forms. Diastereomeric mixtures can be separated into their individual diastereomers on the basis of their physical chemical differences by methods known to those skilled in the art, for example, by chromatography or fractional crystallization. Enantiomers may be separated by converting the enantiomeric mixtures into a diastereomeric mixture by reaction with an appropriate optically active compound, *e.g.*, alcohol, separating the diastereomers and converting, *e.g.*, hydrolyzing, the individual diastereomers to the corresponding pure enantiomers. The use of all such isomers, including diastereomer mixtures and pure enantiomers, are considered to be part of the present invention.

Further details concerning the above-identified synthesis methods which are preferred for preparing the above-recited preferred compound of Formula (IA) or (IB), may be found in copending U.S. Serial No. 09/153762 (Attorney Docket No. PC10004A), filed September 15, 1998, which is incorporated herein by reference in its entirety. Still further details concerning synthesis methods for the compounds of Formula (IA) or (IB) may be found in (*i*) copending U.S. Serial No. 08/963904 (Attorney Docket No. PC9281B), filed April 1, 1997, which is incorporated herein by reference in its entirety, and which was published on November 13, 1997 as WO 97/42174; (*ii*) provisional U.S. Serial No. 60/025446 (Attorney Docket No. PC9282), filed September 4, 1996, copending as PCT/IB97/01023 filed August 25, 1997, which is incorporated herein by reference in its entirety, and which was published on March 12, 1998 as WO 98/09961; (*iii*) copending U.S. Serial No. 08/869358 (Attorney Docket No. PC9283A), filed June 5, 1997, which is incorporated herein by reference in its entirety, and which was published on December 31, 1997 as WO 97/49702; and (*iv*) copending U.S. Serial No. 08/882275 (Attorney Docket No. PC9284A), filed June 25, 1997, which is incorporated herein by reference in its entirety, and which was published on January 7, 1998 as EP 816357.

## Claims

**1.** A method of treating or preventing stasis in all or any part or parts of the stomach of a patient in need of such treatment, wherein said stasis results from hypomotility in said stomach or part thereof, comprising administering to said patient a therapeutically effective amount of an inhibitor of phosphodiesterase-4 (PDE4), including isozyme subtypes thereof, sufficient to restore normal motility to said patient, wherein said PDE4 inhibitor comprises a compound of Formula (IA) or (IB):

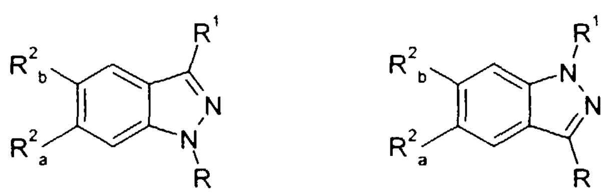

(IA) (IB)

and to pharmaceutically acceptable salts thereof, wherein:

- R is a member independently selected from the group consisting essentially of hydrogen, $(C_1\text{-}C_9)$alkyl; $-(CH_2)_n(C_3\text{-}C_{10})$ cycloalkyl wherein n is an integer selected from 0, 1, and 2; $(C_1\text{-}C_6)$ alkoxy$(C_1\text{-}C_6)$ alkyl; $(C_2\text{-}C_6)$ alkenyl; $-(CH_2)_n(C_3\text{-}C_9)$ heterocyclyl wherein n is an integer selected from 0, 1, and 2; and $-(Z^1)_b(Z^2)_c(C_6\text{-}C_{10})$ aryl wherein b and c are integers independently selected from 0 and 1, $Z^1$ is $(C_1\text{-}C_6)$ alkylene or $(C_2\text{-}C_6)$ alkenylene, and $Z^2$ is O, S, $SO_2$, or $NR^{119}$; and further wherein said heterocyclyl is a member independently selected from the group consisting essentially of acridinyl; benzimidazolyl; benzodioxolane; 1,3-benzodioxol-5-yl; benzo[*b*]furanyl; benzo[*b*]thiophenyl; benzoxazolyl; benzthiazolyl; carbazolyl; cinnolinyl; 2,3-dihydrobenzofuranyl; 1,3-dioxane; 1,3-dioxolane; 1,3-dithiane; 1,3-dithiolane; furanyl; imidazolidinyl; imidazolinyl; imidazolyl; 1H-indazolyl; indolinyl; indolyl; 3H-indolyl; isoindolyl; isoquinolinyl; isothiazolyl; isoxazolyl; morpholinyl; 1,8-naphthyridinyl; oxadiazolyl; 1,3-oxathiolane; oxazolidinyl; oxazolyl; oxiranyl; parathiazinyl; phenazinyl; phenothiazinyl; phenoxazinyl; phthalazinyl; piperazinyl; piperidinyl; pteridinyl; pyranyl; pyrazinyl; pyrazolidinyl; pyrazolinyl; pyrazolo[1,5-c]triazinyl; pyrazolyl; pyridazinyl; pyridyl; pyrimidinyl; pyrimidyl; pyrrolyl; pyrrolidinyl; purinyl; quinazolinyl; quinolinyl; 4H-quinolizinyl; quinoxalinyl; tetrazolidinyl; tetrazolyl; thiadiazolyl; thiazolidinyl; thiazolyl; thienyl; thiomorpholinyl; triazinyl; and triazolyl; wherein said aryl is a carbocyclic moiety which is a member independently selected from the group consisting essentially of benzyl; *cis*- and trans-decahydronaphthalenyl; 2,3-1H-dihydroindenyl (indanyl); indenyl; 1-naphthalenyl; 2-naphthalenyl; phenyl; and 1,2,3,4-tetrahydronaphthalenyl; wherein said alkyl, alkenyl, alkoxyalkyl, heterocyclyl, and aryl moieties defining said R groups are substituted by 0 to 3 substituents where each said substituent comprises a member independently selected from the group consisting essentially of bromo, chloro, or fluoro; hydroxy; ($C_1$-$C_5$) alkyl; ($C_2$-$C_5$) alkenyl; ($C_1$-$C_5$)alkoxy; ($C_3$-$C_6$) cycloalkoxy; mono-, di-, and tri-fluoromethyl; nitro; -C(=O)O$R^{119}$, -C(=O)N$R^{119}R^{120}$, -N$R^{119}R^{120}$ and -S$(=O)_2$N$R^{119}R^{120}$;

- $R^1$ is a member independently selected from the group consisting essentially of hydrogen; ($C_1$-$C_9$)alkyl; ($C_2$-$C_3$) alkenyl; phenyl; ($C_3$-$C_7$) cycloalkyl; and ($C_3$-$C_7$) cycloalkyl($C_1$-$C_2$) alkyl; wherein said alkyl, alkenyl and phenyl moieties defining said $R^1$ groups are substituted by 0 to 3 substituents where each said substituent comprises a member independently selected from the group consisting essentially of methyl; ethyl; mono-, di-, and tri-fluoromethyl; and bromo, chloro, or fluoro; and

- $R^2_a$ and $R^2_b$ are independently selected from the group consisting essentially of hydrogen and hereinafter recited substituents, provided that one, but not both of $R^2_a$ and $R^2_b$ must be independently selected as hydrogen, wherein said substituents comprise moieties of the groups (- I ) through (- V -):

-- ( - I - )

-- a moiety of partial Formulas (1.0.0), (1.0.1), (1.0.2), and (1.0.3):

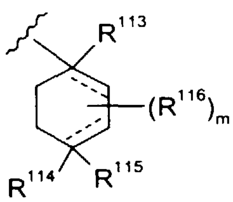
(1.0.0)

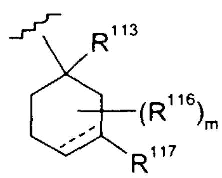
(1.0.1)

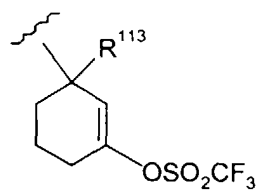
(1.0.2)

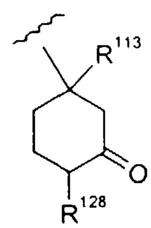
(1.0.3)

--- wherein the dashed lines in partial Formulas (1.0.0) and (1.0.1) independently and optionally represent a single or double bond, provided that in formula (1.0.0) both dashed lines cannot both represent double bonds at the same time;

--- m is an integer selected from 0, 1, 2, 3, and 4, and when 2, may apply to a single carbon atom on the ring;

--- $R^{113}$ is a member selected from the group consisting essentially of H; bromo, chloro, or fluoro; cyano; ($C_2$-$C_4$) alkynyl substituted by 0 or 1 substituent where said substituent is a member selected from the group consisting essentially of phenyl, pyridyl and pyrimidinyl; ($C_1$-$C_4$)alkyl substituted by 0 to 6 bromo, chloro, or fluoro; -$CH_2$NHC(=O)C(=O)$NH_2$; cyclopropyl substituted by 0 or 1 substituent where said substituent is a member selected from the group consisting essentially of $R^{121}$; $R^{127}$; $CH_2OR^{119}$; $NR^{119}R^{120}$; $CH_2NR^{119}R^{120}$; C(=O)O$R^{119}$; C(=O)N$R^{119}R^{120}$; C≡C$R_{11}$; C(Z)H; and -CH=C$R^{121}R^{121}$; provided that $R^{113}$ is H in Formula (1.0.0) when the dashed line for the ring carbon of $R^{113}$ attachment represents a double bond;

--- $R^{114}$ is a member selected from the group consisting essentially of H; $R^{116}$; C(Y)$R^{124}$; C(=O)O$R^{124}$; C(Y)N$R^{127}R^{124}$; CN; C(N$R^{127}$)N$R^{127}R^{124}$; C(NO$R^{119}$)$R^{124}$; C(=O)N$R^{119}$N$R^{119}$C(=O)$R^{119}$; C(=O)

$NR^{119}NR^{127}R^{124}$; $C(NOR^{124})R^{119}$; $C(NR^{119})NR^{127}R^{124}$; $C(NR^{124})NR^{119}R^{120}$; $C(NCN)NR^{127}R^{124}$, C (NCN)S($C_1$-$C_4$) alkyl; $CR^{119}R^{120}OR^{124}$, $CR^{119}R^{120}SR^{124}$, $CR^{119}R^{120}S(O)_nR^{125}$ where n is an integer selected from 0, 1, and 2; $CR^{119}R^{120}NR^{124}R^{127}$; $CR^{119}R^{120}NR^{127}S(=O)_2R_{15}$; $CR^{119}R^{120}NR^{127}C(Y)R^{124}$; $CR^{119}R^{120}NR^{127}C(=O)OR^{125}$; $CR^{119}R^{120}NR^{127}C(Y)NR^{127}R^{124}$; $CR^{119}R^{120}NR^{127}C(NCN)NR^{127}R^{124}$; $CR^{119}R^{120}NR^{127}C(CR^{119}NO_2)S(C_1\text{-}C_4)$ alkyl; $CR^{119}R^{120}C(=O)OR^{125}$; $CR^{119}R^{120}C(Y)NR^{127}R^{124}$; $CR^{119}R^{120}C(NR^{127})NR^{127}R^{124}$; $CR^{119}R^{120}CN$; $CR^{119}R^{120}C(NOR^{120})R^{124}$; $CR^{119}R^{120}C$ (NOR124)$R^{120}$; $CR^{119}R^{120}NR^{127}C(NR^{127})S(C_1\text{-}C_4)$ alkyl; $CR^{119}R^{120}NR^{127}C(NR^{127})NR^{127}R^{124}$; $CR^{119}R^{120}NR^{127}C(=O)C(=O)NR^{127}R^{124}$; $CR^{119}R^{120}NR^{127}C(=O)C(=O)OR^{124}$; tetrazolyl; thiazolyl; imidazolyl; imidazolidinyl; pyrazolyl; thiazolidinyl; oxazolyl; oxazolidinyl; triazolyl; isoxazolyl; oxadiazolyl; thiadiazolyl; $CR^{119}R^{120}$(tetrazolyl); $CR^{119}R^{120}$(thiazolyl); $CR^{119}R^{120}$(imidazolyl); $CR^{119}R^{120}$(imidazolidinyl); $CR^{119}R^{120}$(pyrazolyl); $CR^{119}R^{120}$(thiazolidinyl); $CR^{119}R^{120}$(oxazolyl); ); $CR^{119}R^{120}$(oxazolidinyl); $CR^{119}R^{120}$(triazolyl); $CR^{119}R^{120}$(isoxazolyl); $CR^{119}R^{120}$(oxadiazolyl); $CR^{119}R^{120}$(thiadiazolyl); $CR^{119}R^{120}$(morpholinyl); $CR^{119}R^{120}$(piperidinyl); $CR^{119}R^{120}$(piperazinyl); and $CR^{119}R^{120}$(pyrrolyl); said heterocyclic groups being substituted by 0 to 3 substituents $R^{124}$;

--- $R^{115}$ is a member selected from the group consisting essentially of $R^{119}$; $OR^{119}$; $-CH_2OR^{119}$; cyano; $C(=O)R^{119}$; $C(=O)OR^{119}$; $C(=O)NR^{119}R^{120}$; and $NR^{119}R^{120}$; provided that $R^{115}$ is absent when the dashed line in partial Formula (1.0.0) represents a double bond; or

--- $R^{114}$ and $R^{115}$ are taken together to form =O or $=R^{118}$; or

--- $R^{115}$ is hydrogen and $R^{114}$ is $OR^{124}$; $SR^{124}$; $S(O)_nR^{125}$, where n is an integer selected from 0, 1, and 2; $S(=O)_2NR^{127}R^{124}$; $NR^{127}R^{124}$; $NR^{124}C(=O)R^{119}$; $NR^{127}C(Y)R^{124}$; $NR^{127}C(=O)OR^{125}$; $NR^{127}C(Y)NR^{127}R^{124}$; $NR^{127}S(=O)_2NR^{127}R^{124}$; $NR^{127}C(NCN)NR^{127}R^{124}$; $NR^{127}S(=O)_2R^{125}$; $NR^{127}C(CR^{119}NO_2)NR^{127}R^{124}$; $NR^{127}C(NCN)S(C_1\text{-}C_4)$ alkyl; $NR^{127}C(CR^{119}NO_2)S(C_1\text{-}C_4)$ alkyl; $NR^{127}C(NR^{127})NR^{127}R^{124}$; $NR^{127}C(=O)C(=O)NR^{127}R^{124}$; or $NR^{127}C(=O)C(=O)OR^{124}$;

--- $R^{116}$ is a member independently selected from the group consisting essentially of methyl and ethyl substituted by 0 to 5 bromo, chloro, or fluoro, wherein m may be 2 with respect to a single ring carbon atom to which $R^{116}$ is attached;

--- $R^{117}$ is a member independently selected from the group consisting essentially of $OR^{124}$; $SR^{124}$; $SO_2NR^{127}R^{124}$; $NR^{127}R^{124}$; $NR^{124}C(=O)R^{119}$; $NR^{127}C(Y)R^{124}$; $NR^{127}C(=O)OR^{125}$; $S(O)_nR_{12}$ where n is an integer selected from 0, 1, and 2; $OS(=O)_2R^{122}$; $OR^{122}$; $OC(=O)NR^{123}R^{122}$; $OC(=O)R^{123}$; OC (=O)$OR^{123}$; $O(CR^{122}R^{123})_mOR^{122}$ where m is an integer selected from 0, 1, and 2; $CR^{119}R^{120}OR^{124}$; $CR^{119}R^{120}NR^{127}R^{124}$; $C(Y)R^{124}$; $C(=O)OR^{124}$; $C(Y)NR^{127}R^{124}$; CN; $C(NR^{127})NR^{127}R^{124}$; $C(NOR^{119})R^{124}$; $C(=O)NR^{119}NR^{119}C(=O)R^{119}$; $C(=O)NR^{119}NR^{127}R^{124}$; $C(NOR^{124})R^{119}$; $C(NR^{119})NR^{127}R^{124}$; C $(NR^{124})NR^{119}R^{120}$; $C(NCN)NR^{127}R^{124}$; C(NCN)S($C_1$-$C_4$) alkyl; tetrazolyl; thiazolyl; imidazolyl; imidazolidinyl; pyrazolyl; thiazolidinyl; oxazolyl; oxazolidinyl; triazolyl; isoxazolyl; oxadiazolyl; and thiadiazolyl; where the recited heterocyclic groups are substituted by 0 to 3 substituents where said substituent is $R^{124}$;

---- $R^{118}$ is a member independently selected from the group consisting essentially of $-NR^{125}$; $-NCR^{119}R^{120}(C_2\text{-}C_6)$ alkenyl; $-NOR^{124}$; $-NOR^{129}$; $-NOCR^{119}R^{120}(C_2\text{-}C_6)$ alkenyl; $-NNR^{119}R^{124}$; $-NNR^{119}R^{129}$; -NCN; $-NNR^{119}C(Y)NR^{119}R^{124}$; $-C(CN)_2$; $-CR^{124}CN$; $-CR^{124}C(=O)OR^{119}$; $-CR^{124}C(=O)NR^{119}R^{124}$; $-C(CN)NO_2$; $-C(CN)C(=O)O(C_1\text{-}C_4)$ alkyl; $-C(CN)OC(=O)O(C_1\text{-}C_4)$ alkyl; -C(CN) ($C_1$-$C_4$) alkyl; $-C(CN)C(=O)NR^{119}R^{124}$; 2-(1,3-dithiane), 2-(1,3-dithiolane), dimethylthio ketal, diethylthio ketal, 2-(1,3-dioxolane), 2-(1,3-dioxane), 2-(1,3-oxathiolane); dimethyl ketal and diethyl ketal;

---- $R^{119}$ and $R^{120}$ are each a member independently selected from the group consisting essentially of hydrogen and ($C_1$-$C_4$) alkyl substituted by 0 to 3 fluorine atoms;

---- $R^{121}$ is a member independently selected from the group consisting essentially of fluoro and $R^{120}$;

---- $R^{122}$ is a member independently selected from the group consisting essentially of ($C_1$-$C_6$) alkyl; ($C_2$-$C_3$) alkenyl; ($C_3$-$C_7$) cydoalkyl; ($C_3$-$C_7$) cycloalkyl($C_1$-$C_2$) alkyl; ($C_6$-$C_{10}$) aryl; and ($C_3$-$C_9$) heterocyclyl; where said aryl and heterocyclyl are as defined under R above; and where said $R^{122}$

groups are substituted with 0 to 3 substituents independently selected from the group consisting essentially of methyl; ethyl; mono-, di-, and tri-fluoromethyl; and bromo, chloro, or fluoro;

---- $R^{123}$ is a member independently selected from the group consisting essentially of hydrogen and $R^{122}$;

---- $R^{124}$ is a member independently selected from the group consisting essentially of hydrogen and $R^{125}$; or when $R^{124}$ and $R^{127}$ appear together as $NR^{127}R^{124}$ then $R^{127}$ and $R^{124}$ may be taken together with the nitrogen to which they are attached to form a 5- to 7-membered ring optionally containing one additional heteroatom selected from O, N and S;

---- $R^{125}$ is a member independently selected from the group consisting essentially of $(C_1-C_6)$ alkyl and $-(CR^{119}R^{120})_nR^{126}$, where n is an integer selected from 0, 1, and 2 and $R^{126}$ and said $(C_1-C_6)$ alkyl are substituted by 0 to 3 substituents where each said substituent is a member independently selected from the group consisting essentially of bromo, chloro, or fluoro; nitro; cyano; $NR^{120}R^{127}$; $C(=O)R^{119}$; $OR^{119}$; $C(=O)NR^{120}R^{127}$; $OC(=O)NR^{120}R^{127}$; $NR^{127}C(=O)NR^{127}R^{120}$; $NR^{127}C(=O)R^{120}$; $NR_{17}C(=O)O(C_1-C_4)$ alkyl; $C(NR^{127})NR^{127}R^{120}$; $C(NCN)NR^{127}R^{120}$; $C(NCN)S(C_1-C_4)$ alkyl; $NR^{127}C(NCN)S(C_1-C_4)$ alkyl; $NR^{127}C(NCN)NR^{127}R^{120}$; $NR^{127}S(=O)_2(C_1-C_4)$ alkyl; $S(O)_n(C_1-C_4)$ alkyl; where n is an integer selected from 0, 1, and 2; $NR^{127}C(=O)C(=O)NR^{127}R^{120}$, $NR^{127}C(=O)C(=O)R^{127}$; thiazolyl; imidazolyl; oxazolyl; pyrazolyl; triazolyl; tetrazolyl; and $(C_1-C_2)$ alkyl substituted with 0 to 3 fluorine atoms;

---- $R^{126}$ is a member independently selected from the group consisting essentially of $(C_3-C_7)$ cycloalkyl; pyridyl; pyrimidyl; pyrazolyl; imidazolyl; triazolyl; pyrrolyl; piperazinyl; piperidinyl; morpholinyl; furanyl; thienyl; thiazolyl; quinolinyl; naphthyl; and phenyl;

---- $R^{127}$ is a member independently selected from the group consisting essentially of $OR^{119}$ and $R^{120}$;

---- $R^{128}$ is a member independently selected from the group consisting essentially of H; $C(Y)R^{124}$; $C(=O)OR^{124}$; $C(Y)NR^{127}R^{124}$; CN; $C(NR^{127})NR^{127}R^{124}$; $C(NOR^{119})R^{124}$; $C(=O)NR^{119}NR^{119}C(=O)R^{119}$; $C(=O)NR^{119}NR^{127}R^{124}$; $C(NOR^{124})R^{119}$; $C(NR^{119})NR^{127}R^{124}$; $C(NR^{124})NR^{119}R^{120}$; $C(NCN)NR^{127}R^{124}$; $C(NCN)S(C_1-C_4)$ alkyl; $CR^{119}R^{120}OR^{124}$; $CR^{119}R^{120}SR^{124}$; $CR^{119}R^{120}S(O)_nR^{125}$, where n is an integer selected from 0, 1, and 2; $CR^{119}R^{120}NR^{124}R^{127}$; $CR^{119}R^{120}NR^{127}S(=O)_2R^{125}$; $CR^{119}R^{120}NR^{127}C(Y)R^{124}$; $CR^{119}R^{120}NR^{127}C(=O)OR^{125}$; $CR^{119}R^{120}NR^{127}C(Y)NR^{127}R^{124}$; $CR^{119}R^{120}NR^{127}C(NCN)NR^{127}R^{124}$; $CR^{119}R^{120}NR^{127}C(CR_9NO_2)S(C_1-C_4)$ alkyl; tetrazolyl; thiazolyl; imidazolyl; imidazolidinyl; pyrazolyl; thiazolidinyl; oxazolyl; oxazolidinyl; triazolyl; isoxazolyl; oxadiazolyl; thiadiazolyl; wherein said recited heterocyclic groups are substituted by 0 to 3 substituents where each said substituent is independently selected from the group consisting essentially of $R^{124}$;

---- $R^{129}$ is a member independently selected from the group consisting essentially of $-C(=O)R^{12}$; $-C(=O)NR^{119}R^{124}$; $-S(=O)_2R^{125}$; and $-S(=O)_2NR^{119}R^{124}$;

---- Y is O or S; and,

---- Z is O; $NR^{127}$; NCN; $C(-CN)_2$; $CR^{119}CN$; $CR^{119}NO_2$; $CR^{119}C(=O)OR^{119}$; $CR^{119}C(=O)NR^{119}R^{120}$; $C(-CN)C(=O)O(C_1-C_4)$ alkyl); and $C(-CN)C(=O)NR^{119}R^{120}$;

- or, said substituents defining $R^2{}_a$ and $R^2{}_b$ comprise: -

-- ( - II - )

-- a member selected from the group consisting essentially of $R^{229}$; $-C(=O)NR^{222}(CHR^{222})_mC(=O)NR^{222}O(CH_2)_q(C_6-C_{10})$ aryl); $-C(=NR^{242})NH(CH_2)_p(C_6-C_{10})$ aryl; $-C(=O)NR^{218}(CHR^{222})_mC(=O)NR^{222}(CH_2)_pOR^{222}$; $-C(=O)NR^{222}(CHR^{222})_mS(C_1-C_4)$ alkyl; $-C[=NOC(=O)R^{235}]R^{236}$; $-CR^{227}R^{228}CHR^{238}NR^{219}SO_2(CH_2)_pA$; $-CR^{227}R^{228}CHR^{238}NR^{219}P(=O)(OR^{222})C(=O)(C_1-C_4)$alkyl; $-CR^{227}R^{238}CHR^{238}NR^{219}P(=O)[(C_1-C_4)$alkoxy]2, $-Z^3-R^{217}$; and $-(CR^{227}R^{228})_mNR^{219}(C(O))_qR^{220}$ wherein p is an integer selected from 0, 1, and 2; m is an integer selected from 1, 2, 3, 4, 5, and 6; and q is an integer selected from 1 and 2;

- or, said substituents defining $R^2{}_a$ and $R^2{}_b$ comprise a moiety of partial Formulas (2.0.0) through (2.0.8), inclusive: -

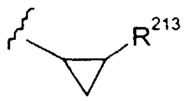
(2.0.0)

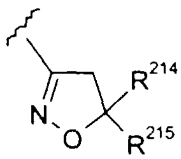
(2.0.1)

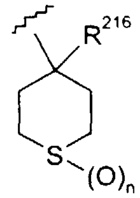
(2.0.2)

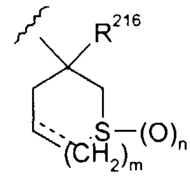
(2.0.3)

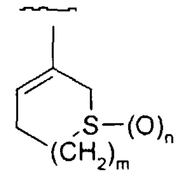
(2.0.4)

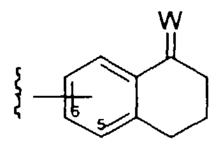
(2.0.5)

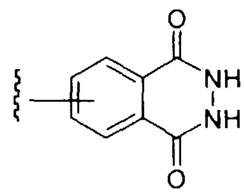
(2.0.6)

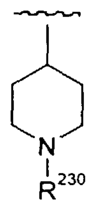
(2.0.7)

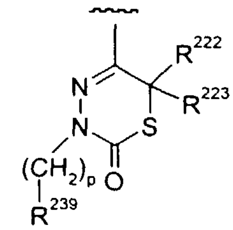
(2.0.8)

--- wherein in said partial Formulas (2.0.0)-(2.0.8), the structures of partial Formulas (2.0.5) and (2.0.6) are attached to the nucleus of Formula (IA) or (IB) at carbons 5, 6, or 7 of said partial Formulas (2.0.5) and (2.0.6); the dashed line in partial Formulas (2.0.2) and (2.0.3) indicates a single bond or double bond, except that $R^{216}$ is absent in partial Formulas (2.0.2) and (2.0.3) where said dashed line indicates a double bond; n is an integer selected from 0, 1, and 2; p is an integer selected from 0, 1, 2, 3, 4, 5, and 6; and m is an integer selected from 0, and 1;

--- $R^{213}$ is a member independently selected from the group consisting essentially of $-C(=O)N(CH_3)(OCH_3)$ and $-(CH_2)_nOH$, where n is an integer selected from 0, 1, 2, 3, and 4;

--- $R^{214}$ and $R^{215}$ are independently selected from the group consisting essentially of H; ethyl; $-CO_2H$; and -C(=O)NHOH;

--- $R^{216}$ is a member independently selected from the group consisting essentially of H; hydroxy; $(C_1-C_6)$ alkyl; $(C_1-C_6)$ alkoxy; $-OC(=O)(C_1-C_6)$ alkyl and $-OC(=O)(C_6-C_{10})$ aryl;

---- $R^{217}$ is a member independently selected from the group consisting essentially of $(C_6-C_{10})$ aryl and a 5- to 10-membered heterocyclyl, wherein said $R^{217}$ groups are substituted by 0 to 3 substituents independently selected from the group consisting essentially of bromo, chloro, or fluroro; trifluoromethyl; cyano; nitro; $-CO_2R^{222}$, $(C_1-C_4)$ alkoxy; $-OC(=O)(C_1-C_4)$ alkyl; $-NR^{222}C(=O)(C_1-C_4)$ alkyl; $-C(=O)NH_2$; -C(=O) NHOH; $-C(=O)O(C_1-C_4)$ alkyl; $(C_1-C_4)$ alkyl; $-S(O)_nR^{222}$ where n is an integer selected from 0, 1, and 2; benzoyl; $-NR^{222}R^{223}$, $-OR^{222}$, $(C_1-C_6)$alkanoyl; $-Y^1-(C_6-C_{10})$ aryl; $-C(=O)O(C_6-C_{10})$ aryl; $-NH(C_6-C_{10})$aryl; $-C(=O)NH(C_6-C_{10})$ aryl; $-C(=O)NR^{222}O(CH_2)_n(C_6-C_{10})$ aryl, where n is an integer selected from 1, 2, and 3; and $-SO_2NH(C_6-C_{10})$ aryl;

---- $R^{218}$ is a member independently selected from the group consisting essentially of H; $(C_1-C_6)$ alkyl; and $-(CH_2)_n(C_6-C_{10})$ aryl, where n is an integer selected from 0, 1, 2, 3, and 4;

---- $R^{219}$ is a member independently selected from the group consisting essentially of H; $-OR^{222}$; $-(CH_2)_mA$ ; and $-CH_2O(CH_2)_mA$, where m is an integer selected from 0, 1, and 2;

---- $R^{220}$ is a member independently selected from the group consisting essentially of $(C_1-C_4)$alkyl; $-OR^{222}$, $-CR^{222}R^{223}OR^{222}$; $-CR^{222}R^{223}NR^{222}R^{223}$; $-CR^{222}(OR^{223})CR^{222}R^{223}OR^{222}$; 2,2-dimethyl-1,3-dioxolan-

4-yl; $-NR^{222}C(=O)NR^{222}R^{223}$, $-S(CR^{222}R^{223})_nCH_3$ where n is an integer selected from 0, 1, 2, 3, 4, and 5; $-NR^{222}(CH_2)_q$(pyridyl) where q is an integer selected from 0 and 1; $-P(=O)[(C_1-C_4)$ alkoxy$)]_2$; $-NR^{222}R^{223}$; $-NR^{222}OR^{223}$; $-NR^{222}NR^{223}R^{221}$, $-NR^{222}CH_2R^{224}$; $-OCH_2NR^{222}C(=O)R^{224}$; $-OCH_2C(=O)NR^{225}R^{226}$, $-OCHR^{222}OC(=O)(C_1-C_4)$ alkyl; $-OCHR^{222}C(=O)(C_1-C_3)$ alkoxy; $-O(CH_2)_mR^{221}$ ; and $-NR^{222}(CH_2)_mR^{221}$ where m is an integer selected from 0, 1, and 2;

---- $R^{221}$ is a member independently selected from the group consisting essentially of H and A;

---- $R^{224}$ is a member independently selected from the group consisting essentially of methyl and phenyl;

---- $R^{225}$ is a member independently selected from the group consisting essentially of H; methyl; ethyl; and $-CH_2CH_2OH$;

---- $R^{226}$ is a member independently selected from the group consisting essentially of H; methyl; ethyl; $-CH_2C(=O)NH_2$; and $-CH_2CH_2OH$;

---- $R^{227}$ is each a member independently selected from the group consisting essentially of H; hydroxy; cyano; halo; $(C_1-C_3)$ alkyl; $(C_1-C_3)$ alkoxy; $-NR^{222}R^{223}$; $-C(=O)OR^{222}$; $-C(=O)R^{222}$; $-CH=CR^{222}R^{223}$; $-C\equiv CR^{222}$; $-CH_2NR^{222}R^{223}$; $-CH_2OR^{222}$; $-C(=O)NR^{222}R^{223}$; $-C(Y^5)H$; and $-CH_2NR_{12}C(=O)C(=O)NR^{222}R^{223}$; provided that when $R^{227}$ is hydroxy then $R^{228}$ is H or $(C_1-C_4)$ alkyl;

---- $R^{228}$ is each a member independently selected from the group consisting essentially of H; fluoro; cyano; and $(C_1-C_4)$ alkyl; where said methyl is substituted by 0 to 3 substituents each comprising a fluorine atom; or

---- $R^{227}$ and $R^{228}$ are taken together to form an oxo (=O) moiety;

--- $R^{222}$ and $R^{223}$ are each a member independently selected from the group consisting essentially of H and $(C_1-C_4)$ alkyl;

--- $R^{229}$ is a member independently selected from the group consisting essentially of phenyl; naphthyl; pyrrolyl; furanyl; thienyl; oxazolyl; pyridinyl; pyrimidinyl; pyridazinyl; quinolinyl; isoquinolinyl; 5,6,7,8-tetrahydroquinolinyl; and 5,6,7,8-tetrahydroisoquinolinyl, where said $R^{229}$ groups, except said phenyl, are substituted by 0 to 3 substituents $R^{233}$, and wherein said phenyl $R^{229}$ group is substituted by 0 to 3 substituents independently selected from $R^{233}$ and $R^{234}$;

--- $R^{230}$ is a member independently selected from the group consisting essentially of $-C(=O)R^{231}$; $-C(=O)C(=O)R^{231}$, $-C(=O)C(Y^2)C(=O)R^{231}$ and a moiety of partial Formula (2.0.9):

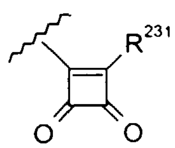

(2.0.9)

wherein:

---- $R^{231}$ is a member independently selected from the group consisting essentially of H; $-OR^{232}$; $-NHR^{232}$; -NHOH; $-NHNH_2$; $-(CH_2)_nY^3$(phenyl) and $-(CH_2)_nY^3$(pyridyl) where n is an integer selected from 0, 1, 2, 3, and 4;

----- $R^{232}$ is a member independently selected from the group consisting essentially of H; $(C_1-C_8)$ alkyl; $-(CH_2)_nY^3$(phenyl) and $-(CH_2)_nY^3$(pyridyl) where n is an integer selected from 0, 1, 2, 3, and 4;

----- $R^{233}$ is each a member independently selected from the group consisting essentially of bromo, chloro, or fluoro; ($C_1$-$C_6$) alkyl; ($C_1$-$C_7$) alkoxy; ($C_2$-$C_6$) alkylenedioxy; trifluoromethyl; -$NR^{222}R^{223}$; nitro; -C($NR^{222}$)$NR^{222}R^{223}$; -C(=O)$NR^{222}R^{223}$C(=O)$R^{222}$; -C($NOR^{222}$)$R^{223}$; -C(NCN)$NR^{222}R^{223}$; -C(NCN)$SR^{222}$; -$(CH_2)_m$(CN) where m is an integer selected from 0, 1, 2, and 3; hydroxy; -C(=O)$R^{222}$, -C(=O)$NR^{222}OR^{223}$; -C(=O)$NR^{222}NR^{222}R^{223}$; -OC(=O)$NR^{222}R^{223}$; -$NR^{222}$C(=O)$R^{222}$; -C(=O)C(=O)$NR^{222}R^{223}$; -$CO_2R^{222}$; -$SO_2R^{222}$; -$SO_2NR^{222}R^{223}$; -C(=O)$NR^{222}R^{223}$; -$NR^{222}SO_2R^{223}$; and -$NR^{222}$C(=O)$NR^{222}R^{223}$;

----- $R^{234}$ is each a member independently selected from the group consisting essentially of imidazolyl; pyrazolyl; triazolyl; tetrazolyl; oxazolyl; isoxazolyl; oxadiazolyl; thiadiazolyl; thiazolyl; oxazolidinyl; thiazolidinyl; and imidazolidinyl, where each of said foregoing $R^{234}$ substituents is substituted by 0 to 3 substituents $R^{233}$;

----- $R^{235}$ is a member independently selected from the group consisting essentially of -$NR^{222}R^{223}$; -NH($C_6$-$C_{10}$) aryl; ($C_1$-$C_6$) alkoxy; and ($C_6$-$C_{10}$) aryloxy;

----- $R^{236}$ is a member independently selected from the group consisting essentially of H; ($C_1$-$C_6$) alkyl and -$(CH_2)_mY^4$(phenyl) where m is an integer selected from 0, 1, 2, 3, and 4 and the phenyl moiety of said -$(CH_2)_mY^4$(phenyl)$R^{236}$ group is substituted by 0 to 3 substituents independently selected from the group consisting essentially of bromo, chloro, or fluoro; -$OR^{222}$; ($C_1$-$C_6$)alkanoyloxy; ($C_6$-$C_{10}$)aryloxy; -$NR^{222}R^{223}$; -NH($C_6$-$C_{10}$)aryl; and -NHC(=O)($C_1$-$C_4$) alkyl;

----- $R^{237}$ is each a member independently selected from the group consisting essentially of bromo, chloro, or fluoro; -$(CH_2)_pNR^{222}$C(=O)$CH_3$ where p is an integer selected from 1, 2, 3, 4, and; ($C_1$-$C_4$)alkoxy; nitro; cyano; -$NR^{222}R^{223}$; -$CO_2R^{222}$; -$OR^{222}$; -C($Y^1$)$NR^{222}R^{223}$; -$NR^{222}$C(NCN)S($C_1$-$C_3$) alkyl; -$NR^{222}$C(NCN)$NR^{222}R^{223}$; -$NR^{222}$C(=O)$NR^{222}R^{223}$; -$NR^{222}$C(=O)C(=O)$NR^{222}R^{223}$; -C(=$NR^{222}$)$NR^{222}R^{223}$; -S(O)$_mCH_3$ where m is an integer selected from 0, 1, and 2; -C(=$NR^{222}$)S($C_1$-$C_3$) alkyl; -$NR^{222}SO_2$($C_1$-$C_3$) alkyl; -OC(=O)$R^{222}$; -OC(=O)$NR^{222}R^{223}$; -$NR^{222}SO_2CF_3$; -$NR^{222}$C(=O)C(=O)$OR^{222}$; -$NR^{222}$C(=O)$R^{222}$; -$NR^{222}$C(=O)$OR^{222}$; imidazolyl; thiazolyl; oxazolyl; pyrazolyl; triazolyl; and tetrazolyl;

----- $R^{238}$ is a member independently selected from the group consisting essentially of H; fluoro; cyano; and ($C_1$-$C_2$)alkyl, where said alkyl is substituted by 0 to 3 substituents independently selected from the group consisting essentially of bromo, chloro, or fluoro; -C(=O)$NR^{222}R^{223}$; and -C(=O)$OR^{222}$;

----- $R^{239}$ is a member independently selected from the group consisting essentially of phenyl substituted by 0 to 2 substituents independently selected from -$NR^{222}R^{223}$, nitro, halo, -$OR^{222}$, -$NHR^{240}$, -$NR^{240}R^{241}$, and -C(=O)$OR^{222}$;

----- $R^{240}$ and $R^{241}$ are each a member independently selected from the group consisting essentially of ($C_1$-$C_8$) alkyl and ($C_2$-$C_8$) alkenyl;

----- $R^{242}$ is pyridin-4-yl substituted by 0 to 2 substituents independently selected from the group consisting essentially of bromo, chloro, or fluoro; and ($C_1$-$C_4$) alkyl;

----- A is each a member independently selected from the group consisting essentially of ($C_1$-$C_6$)alkyl; pyridyl; morpholinyl; piperidinyl; imidazolyl; thienyl; pyrimidyl; thiazolyl; triazolyl; quinolinyl; phenyl; and naphthyl; wherein the foregoing A groups are substituted with 0 to 3 substituents $R^{237}$; or A is -$(CH_2)_q$S($C_1$-$C_4$) alkyl wherein q is an integer selected from 1 and 2;

----- W is a member independently selected from the group consisting essentially of O; NOH; $NNH_2$; NOC(=O)$CH_3$; and NNHC(=O)$CH_3$;

----- $Y^1$ is O or S;

----- $Y^2$ is O, NOH or $H_2$;

----- $Y^3$ is a bond or -CH=CH-;

----- $Y^4$ is a bond, O, S, or -NH-;

----- $Y^5$ is a member independently selected from the group consisting essentially of O; $NR^{222}$; $NOR^{222}$; NCN; $C(CN)_2$; $CR^{222}NO_2$; $CR^{222}C(=O)OR^{222}$; $CR^{222}C(=O)NR^{222}R^{223}$; $C(CN)NO_2$; $C(CN)C(=O)OR^{222}$; and $C(CN)C(=O)NR^{222}R^{223}$; and

----- $Z^3$ is a member independently selected from the group consisting essentially of $-NR^{222}-$; $-(CH_2)_m-$; $-CH_2C(=O)NH-$; $-NHCH_2C(=O)-$; $-CH_2C(Y^1)CH_2-$; -CH=CH-; -C≡C-, $-CH(Y^1H)$; $-C(Y^1)$; $-CH_2C(Y^1)-$; $-C(Y^1)CH_2-$; $-C(Y_1)C(Y_1)-$; $-CH_2NR^{222}-$; $-CH_2-Y^1-$; $-C(Y^1)NR^{218}(CHR^{222})_n-$; $-NR^{218}C(Y^1)(CHR^{222})_n-$; $-NHCH_2-$; $-Y^1-CH_2-$; $-SOCH_2-$; $-CH_2SO-$; $-SO_2CH_2-$; $-CH_2SO_2-$; $-OC(Y^1)-$; -N=N-; $-NHSO_2-$; $-SO_2NH-$; $-C(Y^1)C(Y^1)NH-$; -NHC(=O)O-; -OC(=O)NH-; and -NHC(=O)NH-; wherein for said $Z_3$ moieties n is an integer selected from 0, 1, 2, 3, and 4; and m is an integer selected from 1, 2, and 3;

- or said substituents defining $R^2_a$ and $R^2_b$ comprise: -

-- ( - III - )

-- a member independently selected from the group consisting essentially of 2-oxo-4-pyrrolyl; pyrazolyl; 2-oxo-3,4-dihydro-5-pyrimidyl; 2-oxo-3,4,dihydro-4-pyrimidyl; 2-oxo-tetrahydro-4-pyrimidyl; 2-oxo-tetrahyro-5-pyrimidyl; 2-oxo-4-pyrimidyl; and 2-oxo-5-pyrimidyl; wherein each of said $R^2_a$ and $R^2_b$ groups is substituted by 0, 1, 2, 3, or 4 $R^{236}$ groups;

- or, said substituents defining $R^2_a$ and $R^2_b$ comprise a moiety of partial Formulas (3.0.0) through (3.0.19), inclusive:

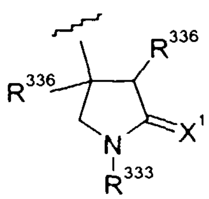
(3.0.0)

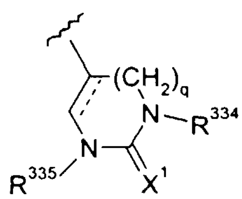
(3.0.1)

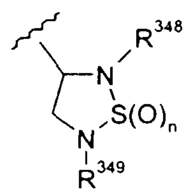
(3.0.2)

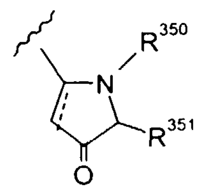
(3.0.3)

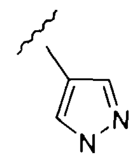
(3.0.4)

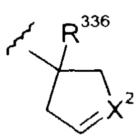
(3.0.5)

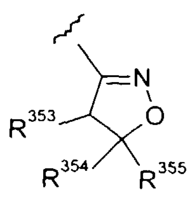
(3.0.6)

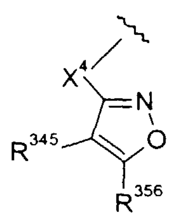
(3.0.7)

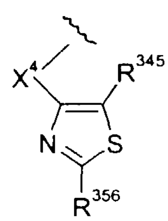
(3.0.8)

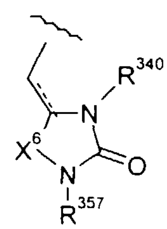
(3.0.9)

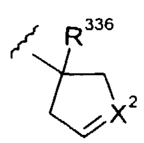
(3.10 )

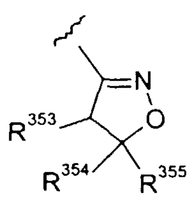
(3.11)

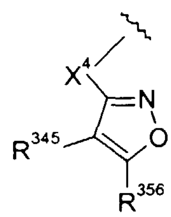
(3.12)

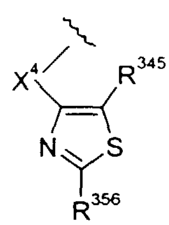
(3.13)

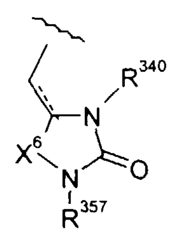
(3.14)

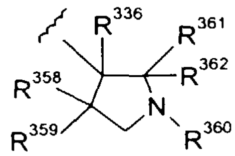 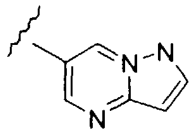 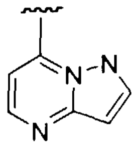 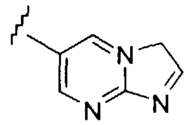 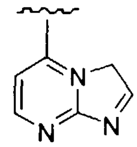

(3.15) (3.16) (3.17) (3.18) (3.19)

---- wherein in said partial Formulas (3.0.0) through (3.0.19), q is an integer selected from 0 and 1 in partial Formula (3.0.1); n is an integer selected from 0, 1, and 2 in partial Formula (3.0.2); and the dashed lines appearing in formulas (3.0.1), (3.0.3), (3.0.6), (3.0.7), (3.0.8), (3.0.9) and (3.0.14) represent a double bond or a single bond;

----- $X^1$ is O or S;

----- $X^2$ in formula (3.0.10) and where the dashed line in formula (3.0.9) represents a double bond, is a member independently selected from the group consisting essentially of $CR^{335}$; $CR^{336}$; $CR^{346}$; and $COC(=O)NR^{339}R^{342}$; or, where the dashed line in formula (3.0.9) represents a single bond, $X^2$ is a member independently selected from the group consisting essentially of $CR^{335}R^{339}$; $CR^{336}R^{339}$; and $CR^{346}R^{339}$;

----- $X^3$ is a member independently selected from the group consisting essentially of $C(=Z^3)$; C(S); and $CR^{336}R^{340}$;

----- $X^4$ is a member independently selected from the group consisting essentially of $-(CH_2)_m-$ where m is an integer selected from 0, 1, and 2;

----- $X^5$ is a bond or $-CH_2-$;

----- $X^6$ is a member independently selected from the group consisting essentially of $-CH_2$-and -C(=O)-;

----- $R^{333}$ is a member independently selected from the group consisting essentially of H; hydroxy; ($C_1$-$C_4$) alkoxy; $-CHR^{337}(O)_q(CH_2)_mA$ where q is an integer selected from 0 and 1, and m is an integer selected from 0, 1, and 2;

----- $R^{334}$ is a member independently selected from the group consisting essentially of H; hydroxy; ($C_1$-$C_4$) alkyl; ($C_1$-$C_2$) alkoxy; $-OC(=O)CH_3$; ($C_2$-$C_3$) alkenyl; and phenyl($C_1$-$C_2$) alkyl-;

----- $R^{335}$ is a member independently selected from the group consisting essentially of H; hydroxy; $-(CH_2)_mA$ where m is an integer selected from 0, 1, and 2; ($C_1$-$C_6$)alkyl; and ($C_2$-$C_3$) alkanoyl; where said alkyl group is substituted by 0 to 3 subtituents independently selected from the group consisting essentially of bromo, chloro, or fluoro; nitro; $-NR^{340}R^{341}$; $-CO_2R^{340}$; $-OR^{340}$; $-OC(=O)R^{340}$; $-C(=O)R^{340}$; cyano; $-C(=Y)NR^{340}R^{341}$; $-NR^{340}C(=Y)NR^{340}R^{341}$, $-NR^{340}C(=Y)R^{340}$; $-NR^{340}C(=O)OR^{340}$; $-C(NR^{340})NR^{340}R^{341}$; $-C(NCN)NR^{340}R^{341}$; $-C(NCN)SR^{340}$; $-NR^{340}SO_2R^{340}$; $-S(O)_mR^{340}$, where m is an integer selected from 0, 1, and 2; $-NR^{340}SO_2CF_3$; $-NR^{340}C(=O)C(=O)NR^{340}R^{341}$; $-NR^{340}C(=O)C(=O)OR^{340}$; imidazolyl; and 1-($NHR^{340}$)-2-imidazolyl;

----- $R^{336}$ is each a member independently selected from the group consisting essentially of H; bromo, chloro, or fluoro; cyano; $R^{343}$; cyclopropyl substituted by 0 or 1 substituent independently selected from the group consisting essentially of $R^{339}$; $-OR^{340}$; $-CH_2OR^{340}$; $-NR^{340}R^{342}$; $-CH_2NR^{340}R^{342}$; $-C(=O)OR^{340}$; $-C(=O)NR^{340}R^{342}$; $-CH=CR^{339}R^{339}$; $-C\equiv CR^{339}$; and $-C(=Z^3)H$;

----- $R^{337}$ is a member independently selected from the group consisting essentially of H; $-C(=O)R^{338}$; imidazolyl; pyrazolyl; triazolyl; tetrazolyl; oxazolyl; isoxazolyl; oxadiazolyl; thiadiazolyl; thiazolyl; oxazolidinyl; thiazolidinyl; and imidazolidinyl;

----- $R^{338}$ is each a member independently selected from the group consisting essentially of $--OR^{340}$;

$-NR^{340}R^{342}$; and $-R^{343}$;

----- $R^{339}$ is each a member independently selected from the group consisting essentially of H; bromo, chloro, or fluoro; and ($C_1$-$C_4$) alkyl substituted by 0 to 3 fluorine atoms;

----- $R^{340}$ and $R^{341}$ are each a member independently selected from the group consisting essentially of hydrogen and ($C_1$-$C_4$) alkyl;

----- $R^{342}$ is each a member independently selected from the group consisting essentially of $-OR^{340}$ and $-R^{340}$;

----- $R^{343}$ is ($C_1$-$C_4$) alkyl;

----- $R^{344}$ is each a member independently selected from the group consisting essentially of bromo, chloro, or fluoro; nitro; cyano; $-NR^{340}R^{346}$; $-NR^{346}R^{342}$; $-C(=Z^3)R^{338}$; $-S(O)_mR^{343}$ where m is an integer selected from 0, 1, and 2; $-OR^{342}$; $-OC(=O)NR^{340}R^{342}$; $-C(NR^{342})NR^{340}R^{342}$; $-C(NR^{340})SR^{343}$; $-OC(=O)CH_3$; $-C(NCN)NR^{340}R^{342}$; $-C(S)NR^{340}R^{342}$; $-NR^{342}C(=O)R^{347}$; $-C(=O)R^{347}$; oxazolyl; imidazolyl; thiazolyl; pyrazolyl; triazolyl; and tetrazolyl;

----- $R^{345}$ is each a member independently selected from the group consisting essentially of hydrogen and ($C_1$-$C_4$) alkyl substituted by01 to 3 fluorine atoms;

----- $R^{346}$ is each a member independently selected from the group consisting essentially of H; $-R^{343}$; $-C(=O)R^{343}$; $-C(=O)C(=O)R^{338}$; $-C(=O)NR^{340}R^{342}$; $-S(O)_mR^{343}$ where m is an integer selected from 0, 1, and 2; $-C(NCN)SR^{343}$; $-C(NCN)R^{343}$; $-C(NR^{342})R^{343}$; $-C(NR^{342})SR^{343}$; and $-C(NCN)NR^{340}R^{342}$;

----- $R^{347}$ is each a member independently selected from the group consisting essentially of $-R^{343}$; $-C(=O)R^{343}$; oxazolidinyl; oxazolyl; thiazolyl; pyrazolyl; triazolyl; tetrazolyl; imidazolyl; imidazolidinyl; thiazolidinyl; isoxazolyl; oxadiazolyl; thiadiazolyl; morpholinyl; piperidinyl; piperazinyl; and pyrrolyl; where each of said recited R347 heterocyclic groups is substituted by 0 to 2 ($C_1$-$C_2$) alkyl groups;

----- $R^{348}$ is each a member independently selected from the group consisting essentially of H; ($C_1$-$C_5$) alkyl; ($C_2$-$C_5$) alkenyl; benzyl; and phenethyl;

----- $R^{349}$ is a member independently selected from the group consisting essentially of H; ($C_1$-$C_5$) alkyl; ($C_1$-$C_5$) alkanoyl; and benzoyl;

----- $R^{350}$ is a member independently selected from the group consisting essentially of H; ($C_1$-$C_4$)alkyl; carboxy; aminocarbonyl; ($C_1$-$C_6$)alkyl substituted by 0 or 1 carboxy, $-(CH_2)_mC(=O)$($C_1$-$C_6$) alkoxy; or $-(CH_2)_m$($C_6$-$C_{10}$) aryl; where m is an integer selected from 0, 1, and 2;

----- $R^{351}$ is a member independently selected from the group consisting essentially of H; ($C_1$-$C_6$) alkyl; $-C(=Y)R^{352}$; -C(=Y)NH35; $-C(=O)OR^{352}$; and $-(CH_2)_nX^7$(pyridyl) where n is an integer selected from 0, 1, 2, 3, 4, and to 5; and $X^7$ is a bond or -CH=CH-; and where said pyridyl moiety is substituted by 0 or 1 bromo, chloro, or fluoro;

----- $R^{352}$ is a member independently selected from the group consisting essentially of ($C_1$-$C_6$) alkyl ($C_3$-$C_8$) cycloalkyl; $-(CH_2)_m$($C_6$-$C_{10}$) aryl; and $-(CH_2)_nX^7$(pyridyl) where n is an integer selected from 0, 1, 2, 3, 4, and 5; and $X^7$ is a bond or -CH=CH-; and where said pyridyl moiety is substituted by 0 or 1 bromo, chloro, or fluoro;

----- $R^{353}$ is a member independently selected from the group consisting essentially of H; $-R^{345}$; ($C_1$-$C_3$) alkyl substituted by 0 or 1 substituent hydroxy, or ($C_1$-$C_3$) alkyoxy($C_1$-$C_3$) alkyl;

----- $R^{354}$ is a member independently selected from the group consisting essentially of H; $-R^{345}$; carboxy; ($C_1$-$C_3$) alkyoxy($C_1$-$C_3$) alkyl-; ($C_3$-$C_7$) cycloalkyl; and ($C_1$-$C_5$) alkyl substituted by 0 or 1 $-NR^{340}R^{341}$; or

----- $R^{353}$ and $R^{354}$ are taken together to form $-CH_2OCH_2OCH_2-$;

----- $R^{355}$ is a member independently selected from the group consisting essentially of H; hydroxy; $(C_1-C_4)$alkyl substituted by 0 or 1 substituent comprising a member independently selected from the group consisting essentially of hydroxy; $-C(=O)R^{340}$; $-NR^{340}R^{341}$; $-(CH_2)_mNHC(=O)R^{340}$; $-(CH_2)_mNHC(=O)R^{343}$; $-(CH_2)_mCO_2R^{340}$; $-(CH_2)_mC(=O)NR^{340}R^{341}$; $-(CH_2)_mC(=O)N(OH)R^{340}$; $-(CH_2)_mSO_2NR^{340}R^{341}$; $-(CH_2)_mPO_3H_2$; $-(CH_2)_mSO_2NHC(=O)R^{343}$; and $-(CH_2)_mSO_2NHC(=O)$(phenyl), where m is an integer selected from 0, 1, 2, 3, and 4;

----- $R^{356}$ is a member independently selected from the group consisting essentially of H; $(C_1-C_4)$ alkyl; phenyl; $-NR^{340}R^{341}$; and $-NR^{340}(C_1-C_4)$ alkanoyl;

----- $R^{357}$ is a member independently selected from the group consisting essentially of $-R^{340}$; $-CH_2CO_2R^{343}$; and $-CH_2C(=O)NR^{340}R^{341}$;

----- $R^{358}$ is a member independently selected from the group consisting essentially of $-C(=O)R^{340}$; $-C(=O)(C_6-C_{10})$ aryl; $-C(=O)(C_3-C_9)$ heteroaryl; $-CO_2R^{340}$; $-C(=O)NR^{340}R^{341}$; cyano; nitro; $-CH_2OH$; $-NR^{340}SO_2R^{340}$; $-NHSO_2(C_6-C_{10})$ aryl; $-NHCO_2(C_1-C_4)$ alkyl; $-NR^{340}C(=O)R^{340}$; and $-NHCO_2(C_6-C_{10})$ aryl;

----- $R^{359}$ is a member independently selected from the group consisting essentially of $-R^{345}$; cyano; carboxy; formyl; $-C(=O)R^{340}$; and $(C_1-C_4)$ alkanoyl;

----- $R^{360}$ is a member independently selected from the group consisting essentially of cyano; $-NR^{340}R^{341}$; $-SO_2(C_1-C_4)$alkyl; $-SO_2(C_6-C_{10})$ aryl; $-C(=O)R^{340}$; $-C(=O)(C_6-C_{10})$ aryl; $-C(=O)(C_3-C_9)$ heteroaryl; $-C(=O)NR^{340}R^{341}$; and $-CO_2R^{340}$;

----- $R^{361}$ and $R^{362}$ are each a member independently selected from the group consisting essentially of H; cyano; nitro; $-CO_2R^{340}$; $-C(=O)NR^{340}R^{341}$; $-CH_2OH$; $-C(=O)R^{340}$; $-NHCO_2R^{340}$; and $-NHSO_2R^{340}$;

----- A is a member independently selected from the group consisting essentially of pyridyl; morpholinyl; piperidinyl; imidazolyl; thienyl; pyrimidyl; thiazolyl; phenyl; and naphthyl; where each of said A groups is substituted by 0 to 2 substituents $R^{344}$ or by 1 substituent $R^{345}$;

----- $Z^3$ is a member independently selected from the group consisting essentially of O; $-NR^{342}$; $NOR^{340}$; N(CN); $C(CN)_2$; $CR^{340}NO_2$; $CR^{340}C(=O)OR^{343}$; $CR^{340}C(=O)NR^{340}R^{341}$; $C(CN)NO_2$; $C(CN)C(=O)OR^{343}$; and $C(CN)C(=O)NR^{340}R^{341}$; and,

----- Y is O or S;

- or said substituents defining $R^2_a$ and $R^2_b$ comprise a moiety of partial Formula (4.0.0): -

-- ( - IV - )

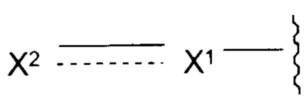

(4.0.0)

--- wherein the broken line indicates a single or double bond;

--- $X^1$ is $-CR^{472}R^{473}-$ where said broken line indicates a single bond; or $-CR^{473}-$ where said broken line indicates a double bond;

--- $X^2$ is $-CR^{475}R^{477}R^{478}-$ or $-C(=NOR^{481})R^{482}-$ where said broken line indicates a single bond; or $-CR^{477}R^{478}$ where said broken line indicates a double bond;

---- $R^{472}$ is a member independently selected from the group consisting essentially of H; hydroxy;

bromo, chloro, or fluoro; and -$OR^{479}$;

---- $R^{473}$ is each a member independently selected from the group consisting essentially of cyano; cyanomethyl; benzyloxy; -$R^{475}$; -$CO_2R^{475}$; -$CO_2(CH_2)_n(C_6$-$C_{10})$ aryl; -C(Y)$NR^{475}R^{476}$; -C(Y)$NR^{475}(CH_2)_n(C_6$-$C_{10})$ aryl; -$(CH_2)_n(C_6$-$C_{10})$ aryl; and -$(CH_2)_n$(5- to 10-membered heteroaryl); where n is an integer selected from 0, 1, 2, and 3; each $R^{473}$ group is substituted by 0 to 3 substituents $R^{474}$ ; and each $R^{473}$ group is substituted by 0 or 1 substituent $R^{480}$;

----- $R^{474}$ is each a member independently selected from the group consisting essentially of bromo, chloro, or fluoro; cyano; nitro; $(C_1$-$C_6)$ alkyl; $(C_2$-$C_6)$ alkenyl; -$OR^{475}$; $(C_3$-$C_7)$ cycloalkoxy; -$NR^{475}R^{476}$; -$NR^{475}OR^{476}$; -$S(O)_mR^{475}$ where m is an integer selected from 0, 1, and 2; -$CO_2R^{475}$, -C(=O)$R^{475}$; -$SO_2NR^{475}R^{476}$; -C(=O)$NR^{475}R^{476}$; -$CR^{475}R^{476}SO_2NR^{475}R^{476}$; -$CR^{475}R^{476}$C(=O)$NR^{475}R^{476}$; -$NHSO_2R^{475}$; -$NHSO_2NR^{475}R^{476}$; -NHC(=O)$NR^{475}R^{476}$; -NHC(=O)$(C_1$-$C_6)$ alkyl; and -NHC(=O)O$(C_1$-$C_6)$ alkyl);

---- $R^{475}$ and $R^{476}$ are each a member independently selected from the group consisting essentially of H; and $(C_1$-$C_6)$ alkyl;

---- $R^{477}$ is a member independently selected from the group consisting essentially of -$R^{473}$; 2-oxo-pyridyl; 3-oxo-pyridyl; 4-oxo-pyridyl; 2-oxo-pyrrolyl; 4-oxo-thiazolyl; 4-oxo-piperidyl; 2-oxo-quinolyl; 4-oxo-quinolyl; 1-oxo-isoquinolyl; 4-oxo-oxazolyl; 5-oxo-pyrazolyl; 5-oxo-isoxazolyl; and 4-oxo-isoxazolyl; where each of said $R^{477}$ groups is substituted by 0 to 3 substituents $R^{474}$ ;

---- $R^{478}$ is a member independently selected from the group consisting essentially of -$R^{475}$; cyano; -$(CH_2)_p(C_6$-$C_{10})$ aryl; and -$(CH_2)_p$(5- to 10-membered heteroaryl); where p is an integer selected from 1, 2, and 3; and where each said $R^{478}$ group is substituted by 0 to 3 substituents $R^{474}$;

----- $R^{479}$ is a member independently selected from the group consisting essentially of formyl; carbamoyl; thiocarbamyl; $(C_1$-$C_6)$alkyl, $(C_2$-$C_6)$ alkenyl; $(C_1$-$C_4)$alkoxy$(C_1$-$C_4)$ alkyl-; and $(C_1$-$C_6)$ alkanoyl; where said alkyl moieties of each of said $R^{479}$ groups is substituted by 0 to 3 substituents independently selected from the group consisting essentially of bromo, chloro, or fluoro; hydroxy; and $(C_1$-$C_4)$ alkoxy;

----- $R^{480}$ is a member independently selected from the group consisting essentially of cyclobutyl; cyclopentyl; cyclohexyl; 2-cyclobuten-1-yl; 2-cyclopenten-1-yl; 3-cyclopenten-1-yl; 2,4-cyclopentadien-1-yl; 3,5-cyclohexadien-1-yl; pyrrolyl; pyrrolidinyl; dioxolanyl; imidazolyl; oxazolyl; imidazolidinyl; pyrazolyl; pyrazolidinyl; pyranyl; piperidinyl; 1,4-dioxanyl; morpholinyl; 1,4-dithianyl; thiomorpholinyl; piperazinyl; 1,3,5-trithianyl; oxazinyl; isoxazinyl; oxathiazinyl; and oxadiazinyl; where each of said $R^{480}$ groups is substituted by 0 to 2 $(C_1$-$C_2)$ alkyl;

----- $R^{481}$ is a member independently selected from the group consisting essentially of H; $(C_1$-$C_6)$ alkyl; $(C_2$-$C_6)$ alkenyl; $(C_2$-$C_6)$ alkynyl; -C(Y)$NR^{475}R^{476}$; -C(Y)NH$(C_6$-$C_{10})$ aryl; -C(Y)$(C_1$-$C_6)$ alkoxy; -C(Y)$(C_6$-$C_{10})$ aryloxy; and -C(Y)$(C_1$-$C_6)$ alkyl);

----- $R^{482}$ is a member independently selected from the group consisting essentially of phenyl and pyridinyl; where each of said $R^{482}$ groups is substituted by 0 to 3 substituents independently selected from the group consisting essentially of bromo, chloro, or fluoro; $(C_1$-$C_4)$ alkyl; hydroxy; $(C_1$-$C_4)$ alkoxy; -$NR^{475}R^{476}$; and -$S(O)_mR^{475}$, where m is an integer selected from 0, 1, and 2; and,

----- -Y is O or S;

- or, said substituents defining $R^2_a$ and $R^2_b$ comprise a moiety of partial Formulas (5.0.0) through (5.0.13), inclusive: -

-- ( - V - )

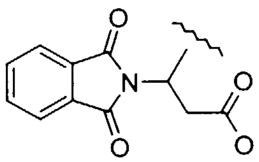

(5.0.0)

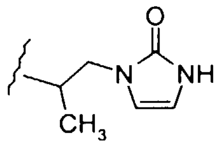

(5.0.1)

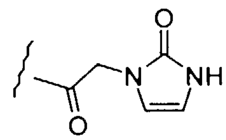

(5.0.2)

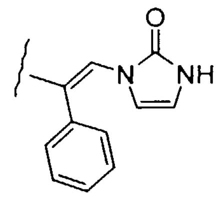

(5.0.3)

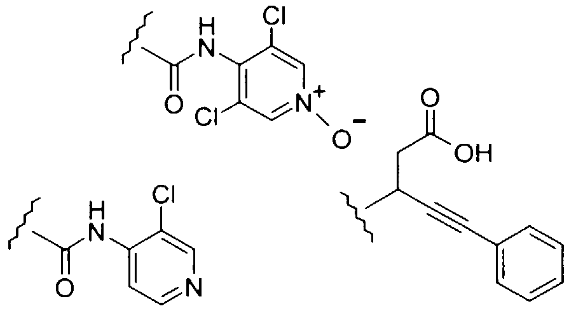

(5.0.4) (5.0.5) (5.0.6)

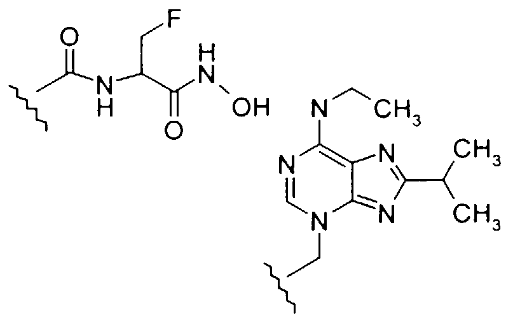

(5.0.7) (5.0.8)

$R^{500}$ = H,

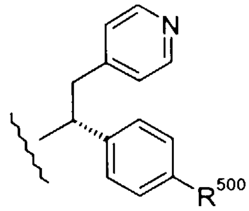

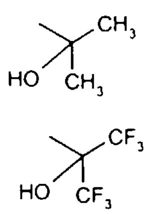

(5.0.9)

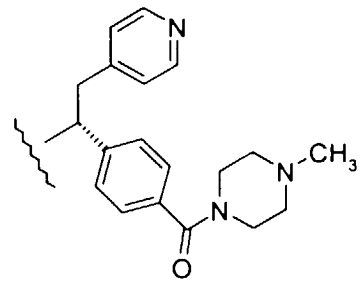

(5.0.10)

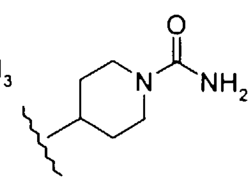

(5.0.11)

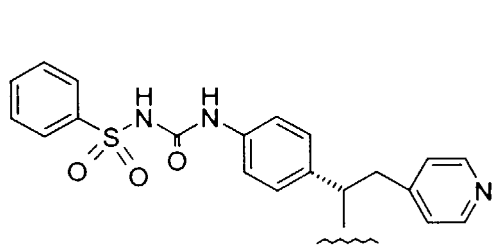

(5.0.12)

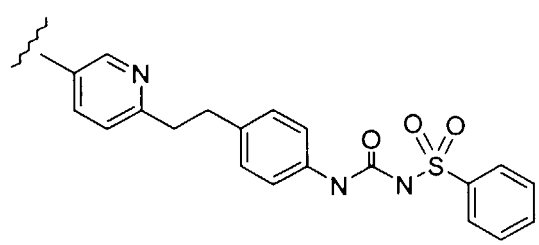

(5.0.13)

**2.** A method according to Claim 1 wherein $R^2_a$ and $R^2_b$ are as defined under ( - IV - ) in Claim 1.

**3.** A method according to Claim 2 wherein $R^1$ is ethyl and R is cyclopentyl, cyclohexyl, or ($C_6$-$C_{10}$) aryl.

**4.** A method according to Claim 2 wherein $R^{473}$ is $-(CH_2)_n(C_5\text{-}C_{10})$aryl or $-(CH_2)_n$(5- to 10-membered heteroaryl), where n is an integer selected from 0, 1, 2, and 3.

**5.** A method according to Claim 4 wherein $R^{473}$ is phenyl or pyridin-4-yl.

**6.** A method according to Claim 1 wherein $R^2{}_a$ and $R^2{}_b$ are as defined under ( - I - ) in Claim 1.

**7.** A method according to Claim 6 wherein R is cyclopentyl or cyclohexyl; $R^1$ is $(C_1\text{-}C_2)$ alkyl; one of $R^2{}_a$ and $R^2{}_b$ is hydrogen and the other is a substituent of partial Formula (1.0.0) where the dashed line represents a single bond, m is 0, $R^{113}$ and $R^{114}$ are in a *cis* relationship to each other, $R^{113}$ is cyano, $R^{115}$ is hydrogen, and $R^{114}$ is carboxy, $-CH_2OH$, or $-CH_2C(=O)NH_2$.

**8.** A method according to Claim 6 wherein R is phenyl substituted by fluoro; $R^1$ is $(C_1\text{-}C_2)$alkyl; one of $R^2{}_a$ and $R^2{}_b$ is hydrogen and the other is a substituent of partial Formula (1.0.0) where the dashed line represents a single bond, $R^{113}$ is cyano, and $R^{115}$ and $R^{114}$ are both hydrogen.

**9.** A method according to Claim 1 wherein said compound of Formula (IA) or (IB) as defined in Claim 1 is a member independently selected from the group consisting of:

1-(1-Cyclopentyl-3-ethyl-1H-indazol-6-yl)4-oxocyclohexanecarbonitrile;

*Trans*-4-cyano-4-(1-cyclopentyl-3-ethyl-1H-indazol-6-yl)-cyclohexanecarboxylic acid methyl ester;

*Cis*-4-cyano-4-(1-cyclopentyl-3-ethyl-1H-indazol-6-yl)cyclohexanecarboxylic acid methyl ester;

*Trans*-4-cyano-4-(1-cyclopentyl-3-ethyl-1H-indazol-6-yl)cyclohexanecarboxylic acid;

*Cis*-4-cyano-4-(1-cyclopentyl-3-ethyl-1H-indazol-6-yl)cyclohexanecarboxylic acid;

1-(1-Cyclohexyl-3-ethyl-1H-indazol-6-yl)-4-oxocyclohexanecarbonitrile;

*Cis*-4-cyano-4-(1-cyclohexyl-3-ethyl-1H-indazol-6-yl)cyclohexanecarboxylic acid methyl ester;

*Trans*-4-cyano-4-(1-cyclohexyl-3-ethyl-1H-indazol-6-yl)-cyclohexanecarboxylic acid methyl ester;

*Cis*-4-cyano-4-(1-cyclohexyl-3-ethyl-1H-indazol-6-yl)cyclohexanecarboxylic acid;

*Trans*-4-cyano-4-(1-cyclohexyl-3-ethyl-1H-indazol-6-yl)cyclohexanecarboxylic acid;

*Cis*-1-(1-cyclohexyl-3-ethyl-1H-indazole-6-yl)-4-hydroxymethylcyclohexanecarbonitrile;

*Cis*-4-cyano-4-(1-cyclohexyl-3-ethyl-1H-indazol-6-yl)cyclohexanecarboxylic acid amide;

*Trans*-4-cyano-4-(-cyclohexyl-3-ethyl-1H-indazol-6-yl)cyclohexanecarboxylic acid amide;

*Cis*-1-(1-cyclohexyl-3-ethyl-1H-indazol-6-yl)-4-(1-hydroxy-1-methylethyl)cyclohexanecarbonitrile;

*Cis*-1-(1-cyclohexyl-3-ethyl-1H-indazol-6-yl)-4-hydroxycyclohexanecarbonitrile;

*Cis*-1-[3-ethyl-1-(4-fluorophenyl)-1H-indazol-6-yl]-4-hydroxycyclohexanecarbonitrile;

*Cis*-1-(-cyclopentyl-3-ethyl-1H-indazol-6-yl)-4-hydroxycyclohexanecarbonitrile;

*Cis*-1-(1-cyclobutyl-3-ethyl-1H-indazol-6-yl)-4-hydroxycyclohexanecarbonitrile;

*Cis*-1-(1-cyclopentyl-3-ethyl-1H-indazol-6-yl)-4-hydroxy-4-methylcyclohexanecarbonitrile;

*Trans*-1-(1-cyclopentyl-3-ethyl-1H-indazol-6-yl)-4-hydroxy-4-methylcyclohexanecarbonitrile;

*Cis*-4-cyano-4-(1-cyclobutyl-3-ethyl-1H-indazol-6-yl)cyclohexanecarboxylic acid;

*Trans*-4-cyano-4-(1-cyclobutyl-3-ethyl-1H-indazol-6-yl)-cyclohexanecarboxylic acid; 6-Bromo-3-ethyl-1-(4-fluorophenyl)-1H-indazole;

4-[3-Ethyl-1-(4-fluorophenyl)-1H-indazol-6-yl]-4-hydroxycyclohexanecarboxylic acid ethyl ester;

4-Cyano-4-[3-ethyl-1-(4-fluorophenyl)-1H-indazol-6-yl]cyclohexanecarboxylic acid ethyl ester;

4-[3-Ethyl-1-(4-fluorophenyl)-1H-indazol-6-yl]cyclohex-3-enecarboxylic acid ethyl ester;

4-Cyano-4-(1-cyclohexyl-3-ethyl-1H-indazol-6-yl)-cyclohexanecarboxylic acid ethyl ester;

Cis-4-Cyano-4-[3-ethyl-1-(4-fluorophenyl)-1H-indazol-6-yl]cyclohexanecarboxylic acid;

4-[3-Ethyl-1-(4-fluorophenyl)-1H-indazol-6-yl]cydohex-3-enecarboxylic acid; and

4-(1-Cyclohexyl-3-ethyl-1H-indazol-6-yl)4-hydroxycyclohexanecarboxylic acid.

**10.** A pharmaceutical composition comprising (*i*) a pharmaceutically acceptable carrier; (*ii*) an amount of an inhibitor of phosphodiesterase-4 (PDE4), including isozyme subtypes thereof, which is therapeutically sufficient to treat or prevent stasis in all or any part or parts of the stomach of a patient in need of such treatment, wherein said stasis results from hypomotility in said stomach or part thereof caused by a therapeutic agent which causes or is known to cause gastric hypomotility or related gastric or gastrointestinal disorders when administered to said patient in therapeutically effective amounts, wherein said inhibitor of phosphodiesterase-4 (PDE4) comprises a compound of Formula (IA) or (IB) as defined in Claim 1; and (*iii*) a therapeutic agent which causes or is known to cause gastric hypomotility or related gastric or gastrointestinal disorders when administered to said patient in therapeutically effective amounts, wherein said therapeutic agent comprises one or more members independently selected from the group consisting essentially of analgesics acting by inhibition of prostaglandin synthesis; antacids which contain calcium carbonate or aluminum hydroxide; anticholinergic agents; antidiarrheal agents; antihistamines which are $H_1$ blockers or have an anticholinergic effect; antiparkinsonian drugs which have an anticholinergic effect; barium sulfate; corticosteroids; clonidine; diuretics which cause hypokalemia; ganglionic blocking agents; heavy metals; laxatives; lithium; monoamine oxidase inhibitors; muscle relaxants; octreotide; opioids; phenothiazines having an anticholinergic effect; polystyrene resins, propranolol; tricyclic antidepressants having an anticholinergic effect; and verapamil.